(19) **Europäisches Patentamt / European Patent Office / Office européen des brevets**

(11) **EP 4 067 906 B1**

(12) # EUROPEAN PATENT SPECIFICATION

(45) Date of publication and mention
of the grant of the patent:
**01.07.2026 Bulletin 2026/27**

(21) Application number: **20893721.9**

(22) Date of filing: **27.11.2020**

(51) International Patent Classification (IPC):
**G01N 33/92** *(2006.01)*       **G01N 33/68** *(2006.01)*

(52) Cooperative Patent Classification (CPC):
**G01N 33/92; G01N 33/6896;** G01N 2333/70596;
G01N 2800/2821

(86) International application number:
**PCT/JP2020/044399**

(87) International publication number:
**WO 2021/107154 (03.06.2021 Gazette 2021/22)**

(54) **METHOD FOR ASSISTING WITH DIAGNOSIS OF ALZHEIMER'S DISEASE OR MILD COGNITIVE IMPAIRMENT, BIOMARKER, REAGENT KIT, AND DEVICE**

VERFAHREN ZUR UNTERSTÜTZUNG DER DIAGNOSE VON MORBUS ALZHEIMER ODER MILDER KOGNITIVER BEEINTRÄCHTIGUNG, BIOMARKER, REAGENZIENKIT UND VORRICHTUNG

PROCÉDÉ D'AIDE AU DIAGNOSTIC DE LA MALADIE D'ALZHEIMER OU D'UN TROUBLE COGNITIF LÉGER, BIOMARQUEUR, KIT DE RÉACTIFS ET DISPOSITIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2019 JP 2019217482**

(43) Date of publication of application:
**05.10.2022 Bulletin 2022/40**

(73) Proprietor: **FUJIFILM Wako Pure Chemical Corporation**
**Osaka 540-8605 (JP)**

(72) Inventors:
• **NISHIBU Takahiro**
**Amagasaki-shi, Hyogo 661-0963 (JP)**
• **IMAWAKA Naoko**
**Amagasaki-shi, Hyogo 661-0963 (JP)**
• **HIRAYASU Kazunari**
**Amagasaki-shi, Hyogo 661-0963 (JP)**
• **UKEKAWA Ryo**
**Amagasaki-shi, Hyogo 661-0963 (JP)**
• **SASAMOTO Kodai**
**Amagasaki-shi, Hyogo 661-0963 (JP)**
• **ONODERA Satoshi**
**Shibukawa-shi, Gunma 377-0007 (JP)**

(74) Representative: **Klunker IP Patentanwälte PartG mbB**
**Lessingstraße 11**
**80336 München (DE)**

(56) References cited:
EP-A1- 3 228 709          WO-A1-2013/054534
WO-A1-2016/088689     WO-A1-2019/009985
WO-A1-2019/058303     WO-A2-2015/061634
JP-A- 2012 508 577      JP-A- 2016 161 480
JP-A- 2016 161 480      JP-A- 2016 535 283
JP-B2- 5 583 600          US-A1- 2019 025 330

• **NAKAI WATARU ET AL: "A novel affinity-based method for the isolation of highly purified extracellular vesicles", vol. 6, no. 33935, 23 September 2016 (2016-09-23), pages 1 - 11, XP055842512, Retrieved from the Internet <URL:https://www.nature.com/articles/ srep33935.pdf> DOI: 10.1038/srep33935**
• **SHIMODA ASAKO, SHIN-ICHI SAWADA, KAZUNARI AKIYOSHI: "Characterization and Functional Modification of Extracellular Vesicles", JAPAN DRUG DELIVERY SYSTEM , vol. 29, no. 2, 25 March 2014 (2014-03-25), Japan , pages 108 - 115, XP055933456, ISSN: 0913-5006, DOI: 10.2745/dds.29.108**

EP 4 067 906 B1

- WATARU NAKAI; TAKESHI YOSHIDA; DIEGO DIEZ; YUJI MIYATAKE; TAKAHIRO NISHIBU; NAOKO IMAWAKA; KEN NARUSE; YOSHIFUSA SADAMURA; RIKINAR: "A navel affinity-based method for the isolation of highly purified extracellular vesicles", SCIENTIFIC REPORTS,, vol. 6, 23 September 2016 (2016-09-23), pages 33935, XP055566011
- AGOSTA, FEDERICA ET AL.: "Myeloid microvesicles in cerebrospinal fluid are associated with myelin damage and neuronal loss in mild cognitive impairment and Alzheimer disease", ANNALS OF NEUROLOGY, vol. 76, no. 6, 2014, pages 813 - 825, XP055278143, DOI: 10.1002/ana.24235
- PULLIAM, LYNN ET AL.: "Plasma neuronal exosomes serve as biomarkers of cognitive impairment in HIV infection and Alzheimer's disease", J NEUROVIROL, vol. 25, no. 5, 4 January 2019 (2019-01-04), pages 702 - 709, XP036925336, DOI: 10.1007/s13365-018-0695-4
- CHARISSE, WINSTON N. ET AL.: "Prediction of conversion from mild cognitive impairment to dementia with neuronally derived blood exosome protein profile", ALZHEIMERS DEMENT, vol. 3, 7 May 2016 (2016-05-07), Amst, pages 63 - 72, XP055569184, DOI: 10.1016/j.dadm.2016.04.001
- IGETA, YUKIFUSA : "Department of integrated diagnostics for elderly Dementia-Related Diseases: Search for predisposing factors and clinical biomarkers by exosome analysis of Alzheimer type dementia", ANNUAL REPORT OF OKINAKA MEMORIAL INSTITUTE FOR MEDICAL RESEARCH, vol. 45, 30 April 2019 (2019-04-30), pages 128 - 129, XP009537076
- GOUWENS LISA K., ISMAIL MUDAR S., ROGERS VICTORIA A., ZELLER NATHAN T., GARRAD EVAN C., AMTASHAR FATIMA S., MAKONI NYASHA J., OSBO: "Abeta42 Protofibrils Interact with and Are Trafficked through Microglial-Derived Microvesicles", ACS CHEMICAL NEUROSCIENCE, vol. 9, no. 6, 15 March 2018 (2018-03-15), pages 1416 - 1425, XP055832622
- CAI, ZHIYOU ET AL.: "Exosomes: a novel therapeutic target for Alzheimer's disease?", NEURAL REGEN RES, vol. 13, no. 5, May 2018 (2018-05-01), pages 930 - 935, XP055825313, DOI: 10.4103/1673-5374.232490
- DELEO, ANNINA M. ET AL.: "Extracellular Vesicle Biology in Alzheimer's Disease and Related Tauopathy", J NEUROIMMUNE PHARMACOL, vol. 13, no. 3, 28 November 2017 (2017-11-28), pages 292 - 308, XP036563929, DOI: 10.1007/s11481-017-9768-z
- ZHOU, JIE ET AL.: "A sensitive detection assay based on signal amplification technology for Alzheimer's disease's early biomarker in exosome", ANAL CHIM ACTA, vol. 1022, 20 March 2018 (2018-03-20), pages 124 - 130, XP085392355, DOI: 10.1016/j.aca.2018.03.016
- LI, TAORAN ET AL.: "Extracellular vesicles as an emerging tool for the early detection of Alzheimer's disease", MECHANISMS OF AGEING AND DEVELOPMENT, vol. 184, 1 November 2019 (2019-11-01), pages 111175, XP085909622, DOI: 10.1016/j.mad.2019.111175
- DINKINS, MICHAEL B. ET AL.: "Exosome reduction in vivo is associated with lower amyloid plaque load in the 5XFAD mouse model of Alzheimer's disease", NEUROBIOLOGY OF AGING, vol. 35, no. 8, 15 February 2014 (2014-02-15), pages 1792 - 1800, XP028654941, DOI: 10.1016/j.neurobiolaging.2014.02.012
- KIMURA, NOBUYUKI: "Exosomes that carry human birth, aging, and diseases: Relationship between exosome involvement and aging in neurodegenerative diseases", EXPERIMENTAL MEDICINE, vol. 29, no. 3, 1 February 2011 (2011-02-01), JP , pages 404 - 409, XP009536878, ISSN: 0288-5514
- AHARON, ANAT ET AL.: "Extracellular Vesicles of Alzheimer's Disease Patients as a Biomarker for Disease Progression", MOLECULAR NEUROBIOLOG, vol. 57, no. 10, 17 July 2020 (2020-07-17), pages 4156 - 4169, XP037235355, DOI: 10.1007/s12035-020-02013-1
- SUN R., WANG H., SHI Y., GAO D., SUN Z., CHEN Z., JIANG H., ZHANG J.: "A Pilot Study of Urinary Exosomes in Alzheimer's Disease", NEURODEGENERATIVE DISEASES, vol. 19, no. 5-6, 6 May 2020 (2020-05-06), CH , pages 184 - 191, XP009536871, ISSN: 1660-2854, DOI: 10.1159/000505851

## Description

### Technical Field

The present invention relates to a method for assisting the diagnosis of Alzheimer's disease or mild cognitive impairment, the *in vitro* use of a biomarker and the *in vitro* use of a reagent kit.

### Background Art

Dementia is a general term for diseases in which the cognitive functions of the brain, such as normally developed memory, learning, judgment, and planning, are continuously reduced due to acquired organic disorders of the brain, which interferes with daily and social life. Known causative diseases of dementia include Alzheimer's disease (AD), dementia with Lewy body (DLB), frontotemporal lobar degeneration (FTLD), and vascular dementia (VaD).

Alzheimer's disease is the most common causative disease of dementia, and the number of patients with Alzheimer's disease is increasing rapidly with the aging of the population. The brain of patients with Alzheimer's disease is characterized by a large number of senile plaques formed by the accumulation of amyloid β (Aβ) protein and neurofibrillary tangles formed by the accumulation of phosphorylated Tau protein, mainly in the cerebral cortex and hippocampus, and it is thought that dementia develops due to neuronal cell death, a decrease in synapses, and a decrease in acetylcholine.

A method of concentrating nerve-derived extracellular vesicles in blood using an anti-NCAM antibody or an anti-L1CAM antibody, and determining Alzheimer's disease using Aβ42, Tau, phosphorylated Tau, or the like contained in the nerve-derived extracellular vesicles as an indicator has been reported as a method for the diagnosis of Alzheimer's disease (Patent Literature 1, Patent Literature 2, Non-Patent Literature 1, and Non-Patent Literature 2).

Patent Literature 3 and Non-Patent Literature 3 disclose an affinity-based method for the isolation of highly purified extracellular vesicles, especially, methods for obtaining, removing and detecting extracellular membrane vesicles using a Tim protein-bonding carrier.

Patent Literature 4 and Patent Literature 5 disclose a method the diagnosing and prognosticating method for Alzheimer's disease by detecting and quantifying exosomes having CD9, CD63 or CD81.

Mild cognitive impairment refers to a state of the boundary between healthy and dementia, in which a part of cognitive function is impaired but there is no problem in daily life. A method for the diagnosis of mild cognitive impairment includes the Mini-Mental State Examination (MMSE). In a case where the MMSE score is 27 points or less out of 30 points, a subject is suspected of having mild cognitive impairment. In addition, in a case where the MMSE score is 23 points or less, a subject is suspected of having dementia.

Citation List

Patent Literature

Patent Literature 1: JP2016-550673
Patent Literature 2: JP2017-520760
Patent Literature 3: EP 3 228 709 A1
Patent Literature 4: JP 2016-161480 A
Patent Literature 5: WO 2015/061634 A2

Non-Patent Literature

Non-Patent Literature 1: C.N. Winston et al./Alzheimer's & Dementia: Diagnosis, Assessment & Disease Monitoring (2016) 3:63-72
Non-Patent Literature 2: Mustapic M et al. Front. Neurosci. (2017) 11:278
Non-Patent Literature 3: W. Nakai et al., SCIENTIFIC REPORTS, vol. 6, no. 33935, pages 1 - 11 (2016.09.23)

### Summary of Invention

Technical Problem

However, the method for the diagnosis of Alzheimer's disease using nerve-derived extracellular vesicles in blood as an indicator requires an operation of affinity concentration of the nerve-derived extracellular vesicles from a test specimen, and the affinity concentration operation is complicated. In addition, an error is likely to occur between the assays since the test specimen cannot be measured directly, and it is difficult to apply such a diagnosis method to the measurement of multiple test specimens.

In addition, there is no useful biomarker for the diagnosis of mild dementia.

The present invention has been conceived in view of the above circumstances, and an object of the present invention is to provide a method for easily assisting the diagnosis of Alzheimer's disease or mild cognitive impairment, *in vitro* uses of a biomarker, and *in vitro* uses of a reagent kit.

Solution to Problem

The present inventors examined whether a specific extracellular vesicle could be a biomarker to assist in the diagnosis of Alzheimer's disease or mild cognitive impairment.

As a result, the present inventors have newly found that a specific extracellular vesicle having phosphatidylserine and tetraspanin, among extracellular vesicles, serves as a biomarker for assisting the diagnosis of Alzheimer's disease.

Furthermore, the present inventors have found that a ratio of an amount of extracellular vesicles having phosphatidylserine and CD9 to an amount of extracellular vesicles having CD9 is an indicator for assisting the diagnosis of Alzheimer's disease or mild cognitive impairment. The present invention has been completed based on these findings.

The present invention relates to a method for assisting the diagnosis of Alzheimer's disease, *in vitro* uses of a biomarker, and *in vitro* uses of a reagent kit, each of which will be described below (first invention in the present invention).

Furthermore, the present invention relates to a method for assisting the diagnosis of Alzheimer's disease or mild cognitive impairment, *in vitro* uses of a biomarker, and *in vitro* uses of a reagent kit, each of which will be described below (second invention in the present invention).

Note that No. [20] to [38] are references.

[1] A method for assisting the diagnosis of Alzheimer's disease, containing measuring an amount of extracellular vesicles having phosphatidylserine and tetraspanin, or the amount of extracellular vesicles having phosphatidylserine and tetraspanin and an amount of extracellular vesicles having tetraspanin, in a biological specimen derived from a subject, and determining that the subject has Alzheimer's disease using the amount of extracellular vesicles having phosphatidylserine and tetraspanin, or a ratio of the amount of extracellular vesicles having phosphatidylserine and tetraspanin to the amount of extracellular vesicles having tetraspanin as an indicator.

[2] The method for assisting the diagnosis of Alzheimer's disease according to [1], in which the determination of Alzheimer's disease is carried out in such a manner that the subject is determined to have Alzheimer's disease in a case where the amount of extracellular vesicles having phosphatidylserine and tetraspanin, or the ratio of the amount of extracellular vesicles having phosphatidylserine and tetraspanin to the amount of extracellular vesicles having tetraspanin is equal to or less than a reference value.

[3] The method for assisting the diagnosis of Alzheimer's disease according to [1] or [2], in which the measurement of the amount of extracellular vesicles is to measure the amount of extracellular vesicles having phosphatidylserine and tetraspanin, the determination of Alzheimer's disease is to determine that the subject has Alzheimer's disease using the amount of extracellular vesicles having phosphatidylserine and tetraspanin as an indicator, and the tetraspanin is selected from CD9, CD63, and CD81.

[4] The method for assisting the diagnosis of Alzheimer's disease according to any one of [1] to [3], in which the measurement of the amount of extracellular vesicles is to measure the amount of extracellular vesicles having phosphatidylserine and tetraspanin, the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine, and the determination of Alzheimer's disease is to determine that the subject has Alzheimer's disease using the amount of extracellular vesicles having phosphatidylserine and tetraspanin as an indicator.

[5] The method for assisting the diagnosis of Alzheimer's disease according to [4], in which the substance having an affinity for phosphatidylserine is a T-cell immunoglobulin-mucin-containing protein.

[6] The method for assisting the diagnosis of Alzheimer's disease according to any one of [1] to [5], in which the measurement of the amount of extracellular vesicles is to measure the amount of extracellular vesicles having phosphatidylserine and tetraspanin, the determination of Alzheimer's disease is to determine that the subject has Alzheimer's disease using the amount of extracellular vesicles having phosphatidylserine and tetraspanin as an indicator, and the biological specimen is a blood specimen or a cerebrospinal fluid.

[7] The method for assisting the diagnosis of Alzheimer's disease according to [1] or [2], in which the measurement of the amount of extracellular vesicles is to measure the amount of extracellular vesicles having phosphatidylserine and tetraspanin, and the amount of extracellular vesicles having tetraspanin, the determination of Alzheimer's disease is to determine that the subject has Alzheimer's disease using the ratio of the amount of extracellular vesicles having phosphatidylserine and tetraspanin to the amount of extracellular vesicles having tetraspanin as an indicator, and the tetraspanin is CD9 or CD63.

[8] The method for assisting the diagnosis of Alzheimer's disease according to any one of [1], [2], and [7], in which the measurement of the amount of extracellular vesicles is to measure the amount of extracellular vesicles having phosphatidylserine and tetraspanin, and the amount of extracellular vesicles having tetraspanin, the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine, the measurement of the amount of extracellular vesicles having tetraspanin is to measure the amount of extracellular vesicles having tetraspanin using the substance having an affinity for tetraspanin, and the determination of Alzheimer's disease is to determine that the subject has Alzheimer's disease using the ratio of the amount of extracellular vesicles having phosphatidylserine and tetraspanin to the amount of extracellular vesicles having tetraspanin as an indicator.

[9] The method for assisting the diagnosis of Alzheimer's disease according to [8], in which the substance having an affinity for phosphatidylserine is a T-cell immunoglobulin-mucin-containing protein.

[10] The method for assisting the diagnosis of Alzheimer's disease according to any one of [1], [2], and [7] to [9], in which the measurement of the amount of extracellular vesicles is to measure the amount of extracellular vesicles having phosphatidylserine and tetraspanin, and the amount of extracellular vesicles having tetraspanin, the determination of Alzheimer's disease is to determine that the subject has Alzheimer's disease using the ratio of the amount of extracellular vesicles having phosphatidylserine and tetraspanin to the amount of extracellular vesicles having tetraspanin as an indicator, and the biological specimen is a blood specimen or a cerebrospinal fluid.

[11] A method for assisting the diagnosis of Alzheimer's disease or mild cognitive impairment, containing measuring an amount of extracellular vesicles having phosphatidylserine and CD9 and an amount of extracellular vesicles having CD9 in a biological specimen derived from a subject, and determining that the subject has Alzheimer's disease or mild cognitive impairment using a ratio of the amount of extracellular vesicles having phosphatidylserine and CD9 to the amount of extracellular vesicles having CD9 as an indicator.

[12] The method for assisting the diagnosis of Alzheimer's disease or mild cognitive impairment according to [11], in which the determination of Alzheimer's disease is to determine that the subject has Alzheimer's disease or mild cognitive impairment in a case where the ratio of the amount of extracellular vesicles having phosphatidylserine and CD9 to the amount of extracellular vesicles having CD9 is equal to or less than a reference value.

[13] The method for assisting the diagnosis of Alzheimer's disease or mild cognitive impairment according to [11] or [12], in which the measurement of the amount of extracellular vesicles having phosphatidylserine and CD9 is to measure the amount of extracellular vesicles having phosphatidylserine and CD9 using a substance having an affinity for CD9 and a substance having an affinity for phosphatidylserine, and the measurement of the amount of extracellular vesicles having CD9 is to measure the amount of extracellular vesicles having CD9 using the substance having an affinity for CD9.

[14] The method for assisting the diagnosis of Alzheimer's disease or mild cognitive impairment according to [13], in which the substance having an affinity for phosphatidylserine is a T-cell immunoglobulin-mucin-containing protein.

[15] The method for assisting the diagnosis of Alzheimer's disease or mild cognitive impairment according to any one of [11] to [14], in which the biological specimen is a blood specimen or a cerebrospinal fluid.

[16] *In vitro* use of a reagent kit for assisting the diagnosis of Alzheimer's disease, the reagent kit containing a substance having an affinity for tetraspanin and a substance having an affinity for phosphatidylserine.

[17] *In vitro* use of a reagent kit for assisting the diagnosis of Alzheimer's disease or mild cognitive impairment, the reagent kit containing a substance having an affinity for CD9 and a substance having an affinity for phosphatidylserine.

[18] *In vitro* use of a biomarker for assisting the diagnosis of Alzheimer's disease, the biomarker containing an extracellular vesicle having phosphatidylserine and tetraspanin.

[19] *In vitro* use of a biomarker set for assisting the diagnosis of Alzheimer's disease or mild cognitive impairment, the biomarker set containing an extracellular vesicle having phosphatidylserine and CD9 and an extracellular vesicle having CD9.

The following items [20] to [38] have not been claimed and are the reference only.

[20] A device for assisting the diagnosis of Alzheimer's disease, containing a measurement unit that measures an amount of extracellular vesicles having phosphatidylserine and tetraspanin in a biological specimen derived from a subject, and a determination unit that determines that the subject has Alzheimer's disease using the amount of extracellular vesicles having phosphatidylserine and tetraspanin as an indicator.

[21] A device for assisting the diagnosis of Alzheimer's disease, containing a measurement unit that measures an amount of extracellular vesicles having phosphatidylserine and tetraspanin and an amount of extracellular vesicles having tetraspanin, in a biological specimen derived from a subject, a calculation unit that calculates a ratio of the amount of extracellular vesicles having phosphatidylserine and tetraspanin to the amount of extracellular vesicles having tetraspanin, and a determination unit that determines that the subject has Alzheimer's disease using the ratio of the amount of extracellular vesicles having phosphatidylserine and tetraspanin to the amount of extracellular vesicles having tetraspanin as an indicator.

[22] A device for assisting the diagnosis of Alzheimer's disease or mild cognitive impairment, containing a measurement unit that measures an amount of extracellular vesicles having phosphatidylserine and CD9 and an amount of extracellular vesicles having CD9, in a biological specimen derived from a subject, a calculation unit that calculates a ratio of the amount of extracellular vesicles having phosphatidylserine and CD9 to the amount of extracellular vesicles having CD9, and a determination unit that determines that the subject has Alzheimer's disease or mild cognitive impairment using the ratio of the amount of extracellular vesicles having phosphatidylserine and CD9 in the biological specimen to the amount of extracellular vesicles having CD9 in the biological specimen as an indicator.

[23] A method for assisting the diagnosis of Alzheimer's disease, containing measuring an amount of extracellular vesicles having phosphatidylserine and tetraspanin and an amount of extracellular vesicles having tetraspanin in a biological specimen derived from a subject, and determining that the subject has Alzheimer's disease using a value obtained by multiplying a ratio of the amount of extracellular vesicles having phosphatidylserine and tetraspanin to the amount of extracellular vesicles having tetraspanin by a ratio of an amount of amyloid β (1-42) to an amount of amyloid β (1-40) as an indicator.

[24] The method for assisting the diagnosis of Alzheimer's disease according to [23], in which the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine, and the measurement of the amount of extracellular vesicles having tetraspanin is to measure the amount of extracellular vesicles having tetraspanin using the substance having an affinity for tetraspanin.

[25] The method for assisting the diagnosis of Alzheimer's disease according to [24], in which the substance having an affinity for phosphatidylserine is a T-cell immunoglobulin-mucin-containing protein.

[26] The method for assisting the diagnosis of Alzheimer's disease according to any one of [23] to [25], in which the biological specimen is a blood specimen or a cerebrospinal fluid.

[27] A method for assisting the diagnosis of Alzheimer's disease or mild cognitive impairment, containing measuring an amount of extracellular vesicles having phosphatidylserine and CD9 and an amount of extracellular vesicles having CD9 in a biological specimen derived from a subject, and determining that the subject has Alzheimer's disease or mild cognitive impairment using a value obtained by multiplying a ratio of the amount of extracellular vesicles having phosphatidylserine and CD9 to the amount of extracellular vesicles having CD9 by a ratio of an amount of amyloid β (1-42) to an amount of amyloid β (1-40) as an indicator.

[28] The method for assisting the diagnosis of Alzheimer's disease or mild cognitive impairment according to [27], in which the determination of Alzheimer's disease is to determine that the subject has Alzheimer's disease or mild cognitive impairment in a case where a value obtained by multiplying the ratio of the amount of extracellular vesicles having phosphatidylserine and CD9 to the amount of extracellular vesicles having CD9 by the ratio of the amount of amyloid β (1-42) to the amount of amyloid β (1-40) is equal to or less than a reference value.

[29] The method for assisting the diagnosis of Alzheimer's disease or mild cognitive impairment according to [27] or [28], in which the measurement of the amount of extracellular vesicles having phosphatidylserine and CD9 is to measure the amount of extracellular vesicles having phosphatidylserine and CD9 using a substance having an affinity for CD9 and a substance having an affinity for phosphatidylserine, and the measurement of the amount of extracellular vesicles having CD9 is to measure the amount of extracellular vesicles having CD9 using the substance having an affinity for CD9.

[30] The method for assisting the diagnosis of Alzheimer's disease or mild cognitive impairment according to [29], in which the substance having an affinity for phosphatidylserine is a T-cell immunoglobulin-mucin-containing protein.

[31] The method for assisting the diagnosis of Alzheimer's disease or mild cognitive impairment according to any one of [27] to [30], in which the biological specimen is a blood specimen or a cerebrospinal fluid.

[32] A reagent kit for assisting the diagnosis of Alzheimer's disease, containing a substance having an affinity for tetraspanin, a substance having an affinity for phosphatidylserine, a substance having an affinity for amyloid β (1-40), and a substance having an affinity for amyloid β (1-42).

[33] A reagent kit for assisting the diagnosis of Alzheimer's disease or mild cognitive impairment, containing a substance having an affinity for CD9, a substance having an affinity for phosphatidylserine, a substance having an affinity for amyloid β (1-40), and a substance having an affinity for amyloid β (1-42).

[34] A biomarker set for assisting the diagnosis of Alzheimer's disease, containing an extracellular vesicle having phosphatidylserine and tetraspanin and an extracellular vesicle having tetraspanin.

[35] A biomarker set for assisting the diagnosis of Alzheimer's disease, containing an extracellular vesicle having phosphatidylserine and tetraspanin, an extracellular vesicle having tetraspanin, amyloid β (1-40), and amyloid β (1-42).

[36] A biomarker set for assisting the diagnosis of Alzheimer's disease or mild cognitive impairment, containing an extracellular vesicle having phosphatidylserine and CD9, an extracellular vesicle having CD9, amyloid β (1-40), and amyloid β (1-42).

[37] A device for assisting the diagnosis of Alzheimer's disease, containing a measurement unit that measures an amount of extracellular vesicles having phosphatidylserine and tetraspanin and an amount of extracellular vesicles having tetraspanin, in a biological specimen derived from a subject, a calculation unit that calculates a value obtained by multiplying a ratio of the amount of extracellular vesicles having phosphatidylserine and tetraspanin to the amount of extracellular vesicles having tetraspanin by a ratio of an amount of amyloid β (1-42) to an amount of amyloid β (1-40), and a determination unit that determines that the subject has Alzheimer's disease using the value obtained by multiplying a ratio of the amount of extracellular vesicles having phosphatidylserine and tetraspanin to the amount of extracellular vesicles having tetraspanin by a ratio of an amount of amyloid β (1-42) to an amount of amyloid β (1-40) as an indicator.

[38] A device for assisting the diagnosis of Alzheimer's disease or mild cognitive impairment, containing a measurement unit that measures an amount of extracellular vesicles having phosphatidylserine and CD9 and an amount of extracellular vesicles having CD9, in a biological specimen derived from a subject, a calculation unit that calculates a value obtained by multiplying a ratio of the amount of extracellular vesicles having phosphatidylserine and CD9 to the amount of extracellular vesicles having CD9 by a ratio of an amount of amyloid β (1-42) to an amount of amyloid β (1-40), and a determination unit that determines that the subject has Alzheimer's disease or mild cognitive impairment using the value obtained by multiplying a ratio of the amount of extracellular vesicles having phosphatidylserine and CD9 in the biological specimen to the amount of extracellular vesicles having CD9 in the biological specimen by a ratio of an amount of amyloid β (1-42) to an amount of amyloid β (1-40) as an indicator.

Advantageous Effects of Invention

According to the present invention, it is possible to easily assist in the diagnosis of Alzheimer's disease or mild cognitive impairment.

(Brief Description of Drawings)

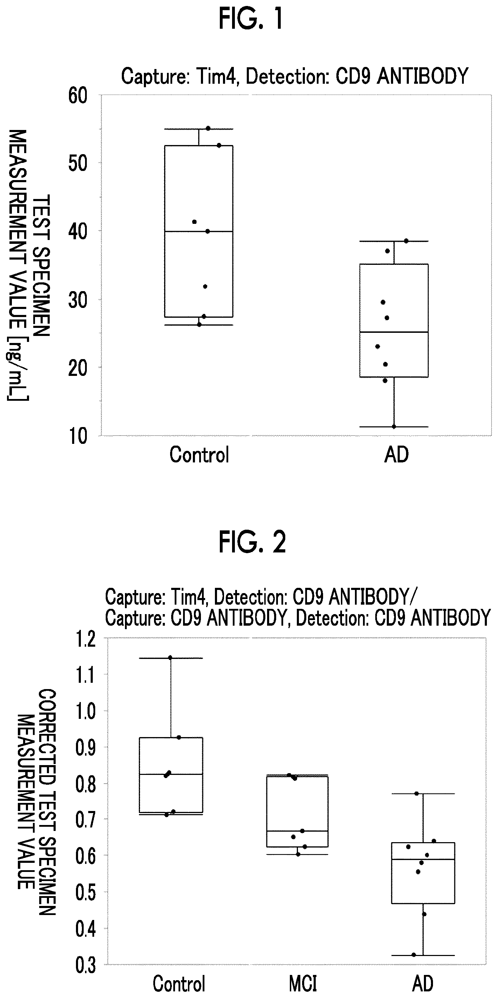

Fig. 1 is a box plot graph of evaluation results of a cerebrospinal fluid (CSF) test specimen derived from a patient with Alzheimer's disease and a CSF test specimen derived from a healthy subject using an amount of exosomes having phosphatidylserine and CD9 as an indicator.

Fig. 2 is a box plot graph of evaluation results of a CSF test specimen derived from a patient with Alzheimer's disease, a CSF test specimen derived from a patient with mild cognitive impairment, and a CSF test specimen derived from a healthy subject using a ratio of an amount of exosomes having phosphatidylserine and CD9 to an amount of extracellular vesicles having CD9 as an indicator.

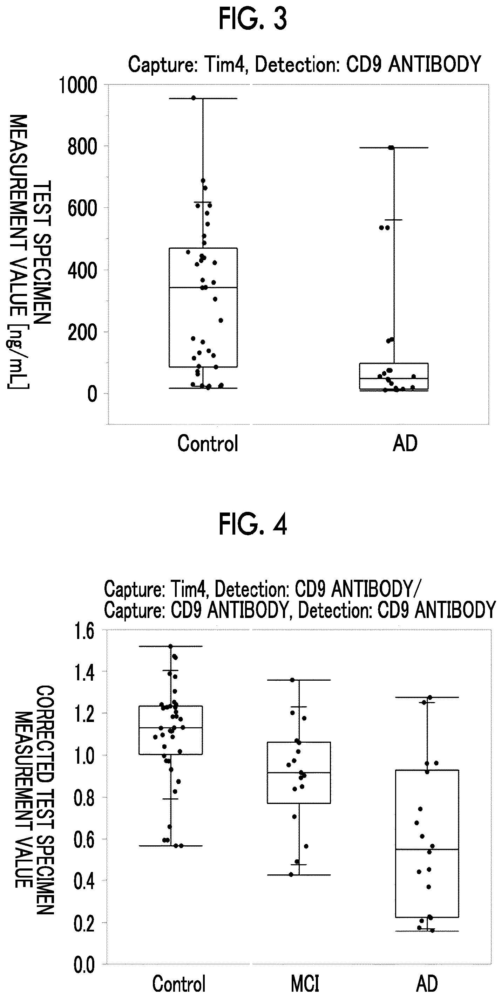

Fig. 3 is a box plot graph of evaluation results of a plasma test specimen derived from a patient with Alzheimer's disease and a plasma test specimen derived from a healthy subject using an amount of exosomes having phosphatidylserine and CD9 as an indicator.

Fig. 4 is a box plot graph of evaluation results of a plasma test specimen derived from a patient with Alzheimer's disease, a plasma test specimen derived from a patient with mild cognitive impairment, and a plasma test specimen derived from a healthy subject using a ratio of an amount of exosomes having phosphatidylserine and CD9 to an amount of extracellular vesicles having CD9 as an indicator.

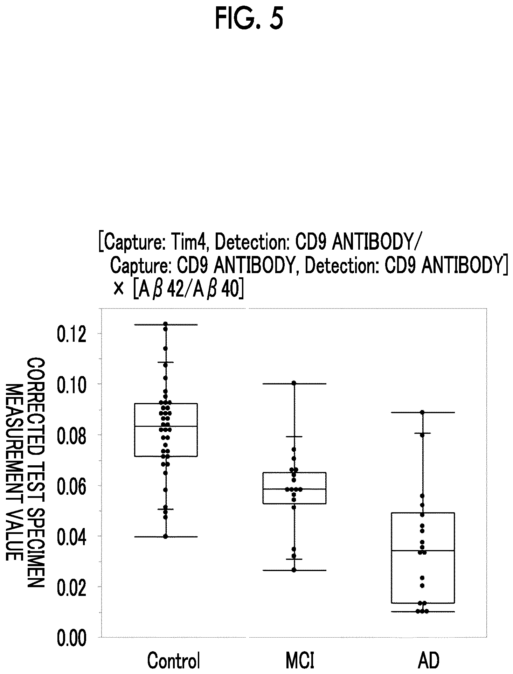

Fig. 5 is a box plot graph of evaluation results of a plasma test specimen derived from a patient with Alzheimer's disease, a plasma test specimen derived from a patient with mild cognitive impairment, and a plasma test specimen derived from a healthy subject using a value obtained by multiplying a ratio of an amount of exosomes having phosphatidylserine and CD9 to an amount of extracellular vesicles having CD9 by a ratio of an amount of amyloid β (1-42) to an amount of amyloid β (1-40) as an indicator.

Description of Embodiments

In a case where the upper limit and the lower limit of the range are shown in the present specification, it means that A to B are A or more and B or less, unless otherwise specified.

The extracellular vesicle is a small membrane vesicle derived from a cell and composed of a lipid bilayer membrane. The extracellular vesicle usually has a diameter of 20 nm to 1,000 nm, preferably 50 nm to 800 nm, more preferably 50 nm to 500 nm, and particularly preferably 50 nm to 200 nm. Examples of the extracellular vesicle include those classified in various ways according to the origin of its development, the size of small membrane vesicle, and the like, as described in Nature Reviews Immunology 9, 581-593 (August, 2009); "Study of Obesity", Vol. 13, No. 2, 2007, topics by Naoto Aoki et al.; and the like. Specific examples of the extracellular vesicle include an exosome, a microvesicle, an ectosome, a membrane particle, an exosome-like vesicle, an apoptotic body, and an adiposome, among which the exosome and the microvesicle are preferable, and the exosome is more preferable.

The exosome is a small membrane vesicle derived from a cell and composed of a lipid bilayer membrane, and examples thereof include those having a diameter of 50 nm to 200 nm, preferably 50 nm to 150 nm, and more preferably 50 nm to 100 nm. It should be noted that the exosome is thought to be derived from a late endosome.

The microvesicle is a small membrane vesicle derived from a cell and composed of a lipid bilayer membrane, and examples thereof include those having a diameter of 100 nm to 1,000 nm, preferably 100 nm to 800 nm, and more preferably 100 nm to 500 nm. It should be noted that the microvesicle is thought to be derived from a cell membrane.

The extracellular vesicle may be contained in a biological specimen derived from a subject or may be isolated from a biological specimen derived from a subject, and is preferably isolated from a biological specimen derived from a subject.

The biological specimen derived from a subject may be any specimen as long as it can contain extracellular vesicles, and examples thereof include a blood-derived specimen such as serum, plasma, whole blood, or buffy coat; and a body fluid specimen such as cerebrospinal fluid, urine, saliva, semen, chest exudate, tears, sputum, mucus, lymph, ascites, pleural effusion, amniotic fluid, bladder lavage fluid, or bronchial alveolar lavage fluid, among which the blood-derived specimen or the cerebrospinal fluid is preferable, the serum, the plasma, or the cerebrospinal fluid is more preferable, the serum or the plasma is still more preferable, and the plasma is particularly preferable. In addition, the blood-derived specimen is more useful from the viewpoint that the burden of sampling on the subject is light.

The biological specimen derived from a subject may be, for example, a specimen directly collected from a subject, or may be specimen that has been subjected to a pretreatment such as recovery, concentration, purification, isolation, dilution with a buffer solution or the like, or filtration sterilization. The pretreatment may be appropriately carried out according to a conventional method. Hereinafter, the biological specimen derived from a subject may be simply referred to as "biological specimen".

The method for isolating extracellular vesicles from the biological specimen may be carried out according to a conventional method, and is not particularly limited. Examples of the method for isolating extracellular vesicles from the biological specimen include an affinity method (for example, a PS affinity method), a differential centrifugation method (for example, an ultracentrifugation method such as a pellet down method, a sucrose cushion method, or a density gradient centrifugation method), an immunoprecipitation method, a chromatography method (for example, an ion exchange chromatography method or a gel permeation chromatography method), a density gradient method (for example, a sucrose density gradient method), an electrophoresis method (for example, an organelle electrophoresis method), a magnetic separation method (for example, a magnetically activated cell sorting (MACS) method), an ultrafiltration concentration method (for example, a nanomembrane ultrafiltration concentration method), a Percoll gradient isolation method, a method using a microfluidic device, and a PEG precipitation method. The affinity method is preferable from the viewpoint that extracellular vesicles having a high degree of purification can be obtained, or the differential centrifugation method is preferable from the viewpoint that it is theoretically possible to recover extracellular vesicles without bias; the affinity method or the ultracentrifugation method is more preferable; and the affinity method is particularly preferable. The PS affinity method, which is an affinity purification for phosphatidylserine, is preferable among the affinity methods. The affinity method and the differential centrifugation method may be carried out according to, for example, the method described in WO2016/088689A.

These isolation methods may be used alone or in combination of two or more thereof. In addition, isolation by one isolation method may be repeated twice or more.

The subject is not particularly limited, and examples thereof include a person diagnosed with Alzheimer's disease based on the diagnostic criteria, a person diagnosed with mild cognitive impairment based on the diagnostic criteria, a person diagnosed at risk of developing Alzheimer's disease based on the diagnostic criteria, a person diagnosed at risk of developing mild cognitive impairment based on the diagnostic criteria, a person who has not been diagnosed mild cognitive impairment based on the diagnostic criteria, a person who has not been diagnosed with Alzheimer's disease or mild cognitive impairment, a person who has not been diagnosed with Alzheimer's disease or mild cognitive impairment based on the diagnostic criteria, and a person who has not been diagnosed at risk of developing Alzheimer's disease or mild cognitive impairment based on the diagnostic criteria, among which the person diagnosed with Alzheimer's disease based on the diagnostic criteria, the person diagnosed with mild cognitive impairment based on the diagnostic criteria, the person diagnosed at risk of developing Alzheimer's disease based on the diagnostic criteria, the person diagnosed at risk of developing mild cognitive impairment based on the diagnostic criteria, or the person who has not been diagnosed with Alzheimer's disease or mild cognitive impairment is preferable.

Examples of the diagnostic criteria include diagnostic criteria used in a case of diagnosing Alzheimer's disease or mild cognitive impairment based on the results of a medical interview, an examination using an imaging apparatus such as amyloid PET diagnosis, an MMSE test, a test on a biomarker or the like of Alzheimer's disease or mild cognitive impairment recommended in the dementia disease treatment guideline such as a decreased level of Aβ42 or an increased level of Tau or phosphorylated Tau in cerebrospinal fluid, a test on candidate substances for the biomarker of Alzheimer's disease or mild cognitive impairment, and the like.

Note that following first invention-3 and second invention-2 are references.

## 1. Assistance in diagnosis of Alzheimer's disease

The first invention in the present invention relates to a method for assisting the diagnosis of Alzheimer's disease, *in vitro* use of a biomarker, and *in vitro* use of a reagent kit.

The first invention of the present invention contains a case of determining that a subject has Alzheimer's disease using a biomarker containing extracellular vesicles having phosphatidylserine and tetraspanin, and using the amount of extracellular vesicles having phosphatidylserine and tetraspanin as an indicator (hereinafter, often referred to simply as "first invention-1"); a case of determining that a subject has Alzheimer's disease using biomarkers containing extracellular vesicles having phosphatidylserine and tetraspanin and extracellular vesicles having tetraspanin in combination and using the ratio of an amount of extracellular vesicles having phosphatidylserine and tetraspanin to an amount of extracellular vesicles having tetraspanin as an indicator (hereinafter, often referred to simply as "first invention-2"); and a case of determining that a subject has Alzheimer's disease using biomarkers containing extracellular vesicles having phosphatidylserine and tetraspanin, extracellular vesicles having tetraspanin, amyloid β (1-40), and amyloid β (1-42) in combination and using the value obtained tetraspanin to an amount of extracellular vesicles having tetraspanin by a ratio of an amount of amyloid β (1-42) to an amount of amyloid β (1-40) (Aβ (1-42)/Aβ (1-40)) as an indicator (hereinafter, often referred to simply as "first invention-3").

### 1-1. First invention-1

**Biomarker for assisting diagnosis of Alzheimer's disease** (the *in vitro* use of a biomarker is claimed)

The biomarker for assisting the diagnosis of Alzheimer's disease in the first invention-1 (hereinafter, often referred to simply as "AD marker") contains an extracellular vesicle having phosphatidylserine and tetraspanin.

The extracellular vesicle in the AD marker is the same as that described above, and preferred ones thereof are also the same.

The extracellular vesicle having phosphatidylserine and tetraspanin in the AD marker has at least one tetraspanin such as CD9, CD63, CD81, and CD151 and phosphatidylserine which is a phospholipid on the membrane surface, preferably at least one tetraspanin selected from CD9, CD63, and CD81 and phosphatidylserine, more preferably at least one tetraspanin selected from CD9 and CD63 and phosphatidylserine, and particularly preferably CD9 and phosphatidylserine.

**Method for assisting diagnosis of Alzheimer's disease**

The method for assisting the diagnosis of Alzheimer's disease in the first invention-1 (hereinafter, often referred to simply as "AD diagnosis assisting method") contains measuring an amount of extracellular vesicles having phosphatidylserine and tetraspanin in a biological specimen, and determining that a subject has Alzheimer's disease using the amount of extracellular vesicles having phosphatidylserine and tetraspanin in the biological specimen as an indicator.

The biological specimen, the subject, and the extracellular vesicle in the AD diagnosis assisting method are the same as those described above, and preferred ones thereof are also the same.

The extracellular vesicle having phosphatidylserine and tetraspanin in the AD diagnosis assisting method is the same as that described above in the AD marker, and preferred ones thereof are also the same.

Examples of the "amount" in the AD diagnosis assisting method include mass and concentration. In addition, the "amount" also contains an actually measured value having a correlation with mass or concentration (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index).

The measurement of the amount of extracellular vesicles having phosphatidylserine and tetraspanin in the AD diagnosis assisting method may be carried out by measuring an amount of only one type of extracellular vesicles having tetraspanin and phosphatidylserine (for example, only extracellular vesicles having CD9 and phosphatidylserine) or by measuring an amount of two or more types of extracellular vesicles having tetraspanin and phosphatidylserine (for example, extracellular vesicles having CD9 and phosphatidylserine, and extracellular vesicles having CD63 and phosphatidylserine). It is preferable to measure the amount of only one type of extracellular vesicles having tetraspanin and phosphatidylserine.

The measurement of the amount of extracellular vesicles having phosphatidylserine and tetraspanin in the AD diagnosis assisting method is not particularly limited as long as it is a method commonly carried out in this field. For example, an immunoassay using a substance having an affinity for tetraspanin and a substance having an affinity for phosphatidylserine, a mass spectrometry, a method combining these methods, or the like may be used for the measurement. Among these methods, the immunoassay is preferable. It should be noted that the immunoassay also contains, in addition to a method using an immune reaction (antigen-antibody reaction), a method using, for example, a binding force between two molecules other than the antigen-antibody reaction (method according to the immunoassay).

The substance having an affinity for tetraspanin may be any substance that specifically binds to tetraspanin, and examples thereof include a protein that specifically binds to tetraspanin, such as an antibody that specifically binds to tetraspanin or a lectin that specifically binds to a sugar chain of tetraspanin, and a nucleic acid that specifically binds to tetraspanin, among which the protein that specifically binds to tetraspanin is preferable, and the antibody that specifically binds to tetraspanin is more preferable. Examples of the antibody that specifically binds to tetraspanin include an anti-CD9 antibody, an anti-CD63 antibody, an anti-CD81 antibody, and an anti-CD151 antibody, among which the anti-CD9 antibody, the anti-CD63 antibody, or the anti-CD81 antibody is preferable, the anti-CD9 antibody or the anti-CD63 antibody is more preferable, and the anti-CD9 antibody is particularly preferable. Only one type of the substance having an affinity for tetraspanin may be used, or two or more types of the substances having an affinity for tetraspanin may be used. It is preferable to use only one type of the substance having an affinity for tetraspanin.

The substance having an affinity for phosphatidylserine may be any substance that specifically binds to phosphatidylserine, and examples thereof include a protein that specifically binds to phosphatidylserine and a nucleic acid that specifically binds to phosphatidylserine, among which the protein that specifically binds to phosphatidylserine is preferable. Examples of the protein that specifically binds to phosphatidylserine include an antibody that specifically binds to phosphatidylserine and a protein with phosphatidylserine affinity, among which the protein with phosphatidylserine affinity is preferable. Examples of the antibody that specifically binds to phosphatidylserine include an anti-phosphatidylserine antibody 1H6 (available from Merck & Co., Inc.). Examples of the protein with phosphatidylserine affinity include a Tim protein such as Tim1 (T-cell immunoglobulin-mucin domain-containing molecule 1, T-cell immunoglobulin-mucin domain 1), Tim2 (T-cell immunoglobulin-mucin domain-containing molecule 2, T-cell immunoglobulin-mucin domain 2), Tim3 (T-cell immunoglobulin-mucin domain-containing molecule 3, T-cell immunoglobulin-mucin domain 3), or Tim4 (T-cell immunoglobulin-mucin domain-containing molecule 4, T-cell immunoglobulin-mucin domain 4); Annexin V; and MFG-E8, among which the Tim protein or the anti-phosphatidylserine antibody is preferable, and the Tim protein is more preferable. In addition, the Tim protein is preferably Tim1 or Tim4 and more preferably Tim4.

Only one type of the substance having an affinity for phosphatidylserine may be used, or two or more types of the substances having an affinity for phosphatidylserine may be used. It is preferable to use only one type of the substance having an affinity for phosphatidylserine.

The substance having an affinity for tetraspanin and the substance having an affinity for phosphatidylserine may be commercially available products or substances appropriately prepared by a conventional method. In addition, the antibody that specifically binds to tetraspanin or the antibody that specifically binds to phosphatidylserine may be either a polyclonal antibody or a monoclonal antibody, and these antibodies may be used alone or in combination thereof as appropriate. In addition, not only an immunoglobulin molecule itself (intact immunoglobulin), but also a fragment antibody such as Fab, F(ab')2, or F(ab'), which is a fragment of the immunoglobulin molecule and has an ability to bind to an antigen, or a synthetic antibody such as a single chain antibody (single chain Fv), a diabody, a triabody, or a tetrabody may be used as the antibody that specifically binds to tetraspanin or the antibody that specifically binds to phosphatidylserine. In addition, in a case where these antibodies are prepared, the antibodies may be prepared, for example, according to the method described in "Immunoassay" (edited by Biochemical Assay Society of JAPAN, Kodansha Ltd., 2014).

The substance having an affinity for tetraspanin or/and the substance having an affinity for phosphatidylserine may be labeled with a labeling substance. Examples of the labeling substance include an enzyme such as peroxidase, microperoxidase, or alkaline phosphatase; a radioactive isotope such as $^{99m}Tc$, $^{131}I$, $^{125}I$, $^{14}C$, $^{3}H$, $^{32}P$, or $^{35}S$; a fluorescent substance such as fluorescein, fluorescein isothiocyanate (FITC), 4-methylumbelliferone, HiLyte, Alexa, CyDye, rhodamine, or a derivative thereof; a luminescent substance such as luciferin, luminol, or ruthenium complex; a substance having absorption in an ultraviolet region, such as phenol, naphthol, anthracene, or a derivative thereof; a substance having properties as a spin labeling agent represented by a compound having an oxyl group such as 4-amino-2,2,6,6-tetramethylpiperidine-1-oxyl; and a nanoparticle such as colloidal gold or quantum dot, among which the enzyme or the fluorescent substance is preferable. The method for labeling the substance having an affinity for tetraspanin or/and the substance having an affinity for phosphatidylserine with the labeling substance is not particularly limited, and may be carried out according to a labeling method known per se. The measurement of these labeling substances may be carried out according to a measuring method known per se depending on the labeling substance.

Using either of the substance having an affinity for tetraspanin or the substance having an affinity for phosphatidylserine as a primary affinity substance, a secondary affinity substance (for example, a secondary antibody) that specifically binds to the primary affinity substance may be further used. The secondary affinity substance may be labeled with a labeling substance and is preferably labeled with a labeling substance and more preferably a secondary antibody labeled with a labeling substance. In addition, the labeling substance and the labeling method are the same as those described above, and preferred ones thereof are also the same.

In addition, labeling with a labeling substance may be carried out taking advantage of avidin-biotin binding using the substance having an affinity for tetraspanin or/and the substance having an affinity for phosphatidylserine to which one of avidin and biotin is bound, and a labeling substance to which the other one of avidin and biotin is bound. Examples of the biotin include biotin, iminobiotin, desthiobiotin, biocytin, and biotin sulfoxide, among which the biotin is preferable. Examples of the avidin include avidin, tamavidin, tamavidin 2, and streptavidin, among which the streptavidin is preferable. The method for binding one of avidin and biotin to the substance having an affinity for tetraspanin or/and the substance having an affinity for phosphatidylserine, and the method for binding the other one of avidin and biotin to the labeling substance may be carried out according to a conventional method.

The substance having an affinity for tetraspanin or/and the substance having an affinity for phosphatidylserine may be immobilized on a solid phase, and it is preferable that the substance having an affinity for phosphatidylserine is immobilized on a solid phase.

Examples of the solid phase include synthetic polymer compounds such as latex, polystyrene, polypropylene, polyacrylic acid, polymethacrylic acid, polyacrylamide, polyglycidyl methacrylate, polyvinyl chloride, polyethylene, polychlorocarbonate, silicone resin, and silicone rubber, and inorganic substances such as porous glass, ground glass, ceramics, alumina, silica gel, activated charcoal, and metal oxide. In addition, two or more of these solid phase substances may be used in combination.

The shape of the solid phase is not particularly limited, and examples thereof include a microtiter plate (ELISA plate), a bead, a tube (microtube), a particle, a dedicated tray in which a large number of tubes are integrally molded, a disk-shaped piece, and a test tube.

The method for immobilizing the substance having an affinity for tetraspanin or/and the substance having an affinity for phosphatidylserine on a solid phase is not particularly limited as long as it is a method commonly used in this field, and may be carried out according to a conventional method.

In the AD diagnosis assisting method, the combination of the substance having an affinity for tetraspanin and the substance having an affinity for phosphatidylserine is not particularly limited, and is preferably a combination of the protein that specifically binds to tetraspanin and the protein that specifically binds to phosphatidylserine, and more preferably a combination of the antibody that specifically binds to tetraspanin and the antibody that specifically binds to phosphatidylserine or the protein with phosphatidylserine affinity.

The combination of the antibody that specifically binds to tetraspanin and the antibody that specifically binds to phosphatidylserine or the protein with phosphatidylserine affinity may be, for example, a combination of an antibody that specifically binds to tetraspanin and a Tim protein or an antibody that specifically binds to phosphatidylserine, which is preferably a combination of an anti-CD9 antibody, an anti-CD63 antibody, or an anti-CD81 antibody and a Tim protein, more preferably a combination of an anti-CD9 antibody or an anti-CD63 antibody and a Tim protein, still more preferably a combination of an anti-CD9 antibody or an anti-CD63 antibody and Tim1 or Tim4, particularly preferably a combination of an anti-CD9 antibody or an anti-CD63 antibody and Tim4, and most preferably a combination of an anti-CD9 antibody and Tim4.

Specific examples of the method for measuring the amount of extracellular vesicles having phosphatidylserine and tetraspanin include immunoassays and mass spectrometries known per se, including enzyme-linked immunosorbent assay (ELISA), enzyme immunoassay (EIA), radioimmunoassay (RIA), fluorescence enzyme immunoassay (FEIA), fluorescence immunoassay (FIA), chemiluminescence enzyme immunoassay (CLEIA), chemiluminescence immunoassay (CLIA), electrochemiluminescence immunoassay (ECLIA), immunochromatography assay (ICA), luminescent oxygen channeling immunoassay (LOCI), capillary electrophoresis such as liquid-phase binding assay-electrokinetic analyte transport assay (LBA-EATA) and lectin electrophoresis, Western blotting, nephelometric immunoassay (NIA) such as latex nephelometric immunoassay, turbidimetric immunoassay (TIA) such as latex turbidimetric immunoassay, immunoagglutination assay such as particle counting immunoassay (PCIA), and surface plasmon resonance (SPR). Among these methods, the enzyme-linked immunosorbent assay (ELISA), the chemiluminescence enzyme immunoassay (CLEIA), the chemiluminescence immunoassay (CLIA), the electrochemiluminescence immunoassay (ECLIA), or the capillary electrophoresis is preferable; the enzyme-linked immunosorbent assay (ELISA), the chemiluminescence enzyme immunoassay (CLEIA), or LBA-EATA is more preferable; and the enzyme-linked immunosorbent assay (ELISA) is particularly preferable.

The principle for measuring the amount of extracellular vesicles having phosphatidylserine and tetraspanin is not particularly limited, and examples thereof include a sandwich method and a competitive method, among which the sandwich method is preferable. In addition, a homogeneous method, a heterogeneous method, or the like may be used as the measurement principle, and the heterogeneous method is preferable.

Examples of the method according to the immunoassay include a method of carrying out the immunoassay using the substance having an affinity for tetraspanin and the substance having an affinity for phosphatidylserine other than the antibody that specifically binds to tetraspanin and the antibody that specifically binds to phosphatidylserine, and examples of the preferred method include the same method.

The measurement of the amount of extracellular vesicles having phosphatidylserine and tetraspanin may be specifically carried out according to the method described in WO2016/088689A.

The measurement of the amount of extracellular vesicles having phosphatidylserine and tetraspanin in the AD diagnosis assisting method specifically includes, for example, a method containing (1) a step of bringing extracellular vesicles having phosphatidylserine and tetraspanin in a biological specimen into contact with a substance having an affinity for tetraspanin and a substance having an affinity for phosphatidylserine to form a complex containing the extracellular vesicles having phosphatidylserine and tetraspanin in the biological specimen, the substance having an affinity for tetraspanin, and the substance having an affinity for phosphatidylserine (hereinafter, often referred to simply as "complex forming step"), and (2) a step of measuring an amount of the complex (hereinafter, often referred to simply as "complex amount measuring step") as a preferable method.

Specific examples and preferred examples of the substance having an affinity for tetraspanin, the substance having an affinity for phosphatidylserine, the combination thereof, and the like are the same as those described above.

The order in which the substance having an affinity for tetraspanin, the substance having an affinity for phosphatidylserine, and the biological specimen are brought into contact with each other in the complex forming step is not particularly limited, and it is preferable to contact the biological specimen with the substance having an affinity for phosphatidylserine and then the substance having an affinity for tetraspanin.

That is, the complex forming step preferably contains a first procedure of bringing a biological specimen into contact with a substance having an affinity for phosphatidylserine to form a first complex composed of extracellular vesicles in the biological specimen and the substance having an affinity for phosphatidylserine, and a second procedure of bringing the first complex into contact with a substance having an affinity for tetraspanin to form a second complex composed of the first complex and the substance having an affinity for tetraspanin.

Specifically, in the complex forming step, for example, an antibody or Tim protein (preferably Tim1 or Tim4 and more preferably Tim4) that specifically binds to phosphatidylserine immobilized on a solid phase and a biological specimen are brought into contact with each other to form a first complex of the antibody or Tim protein that specifically binds to phosphatidylserine and an extracellular vesicle having phosphatidylserine and tetraspanin in the biological specimen, and the first complex and an antibody that specifically binds to tetraspanin are brought into contact with each other to form a second complex of the first complex and the antibody that specifically binds to tetraspanin.

After forming the complex, it is preferable to carry out a washing operation (B/F separation) at least before the complex amount measuring step.

Specifically, for example, the washing operation may be carried out after forming the first complex or/and after forming the second complex in the above method. It is preferable to carry out the washing operation (B/F separation) after forming the first complex, and further carry out the washing operation (B/F separation) after forming the second complex.

The complex amount measuring step is a step of measuring an amount of the complex containing extracellular vesicles having phosphatidylserine and tetraspanin, a substance having an affinity for tetraspanin, and a substance having an affinity for phosphatidylserine obtained in the complex forming step. Any method may be used as long as the amount of the complex can be measured.

More specifically, the complex amount measuring step may detect a labeling substance in the complex containing an antibody or Tim protein (preferably Tim1 or Tim4 and more preferably Tim4) that specifically binds to phosphatidylserine immobilized on a solid phase, an extracellular vesicle having phosphatidylserine and tetraspanin in a biological specimen, an antibody that specifically binds to tetraspanin, and a labeling substance (preferably an enzyme or a fluorescent substance), obtained in the complex forming step using, for example, (1) an antibody that specifically binds to tetraspanin labeled with a labeling substance, or (2) a labeled secondary antibody labeled with a labeling substance, which specifically binds to an "antibody that specifically binds to tetraspanin", or (3) an antibody that specifically binds to tetraspanin to which one of avidin and biotin is bound and a labeling substance to which the other one of avidin and biotin is bound.

In the complex amount measuring step, it is preferable to carry out a washing operation (B/F separation) before detecting a labeling substance.

More specifically, the measurement of the amount of extracellular vesicles having phosphatidylserine and tetraspanin in the AD diagnosis assisting method may be carried out, for example, in such a manner that an antibody or Tim protein (preferably Tim1 or Tim4 and more preferably Tim4) that specifically binds to phosphatidylserine immobilized on a solid phase plate and a biological specimen are brought into contact with each other to form a first complex of the antibody or Tim protein that specifically binds to phosphatidylserine and an extracellular vesicle having phosphatidylserine and tetraspanin in the biological specimen, followed by B/F separation if necessary, (1) the first complex is brought into contact with an antibody that specifically binds to tetraspanin labeled with a labeling substance to form a second complex of the first complex and the antibody that specifically binds to tetraspanin labeled with a labeling substance, (2) the first complex is brought into contact with an antibody that specifically binds to tetraspanin to form a second complex of the first complex and the antibody that specifically binds to tetraspanin, followed by B/F separation if necessary, and the second complex is brought into contact with a labeled secondary antibody labeled with a labeling substance, which specifically binds to "the antibody that specifically binds to tetraspanin", to form a third complex of the second complex and the labeled secondary antibody, or (3) the first complex is brought into contact with an antibody that specifically binds to tetraspanin to which one of avidin and biotin is bound to form a second complex of the first complex and the antibody that specifically binds to tetraspanin to which one of avidin and biotin is bound, followed by B/F separation if necessary, a third complex of the second complex and a labeling substance (preferably an enzyme or a fluorescent substance) to which the other one of avidin and biotin is bound is formed, followed by B/F separation, and then the labeling substance of the obtained second complex or third complex is detected.

The amount of the biological specimen and the amount (concentration) of protein in the biological specimen, the amount (concentration) and number of particles of extracellular vesicles having phosphatidylserine and tetraspanin in the biological specimen, and the amount (concentration) of the substance having an affinity for tetraspanin and the substance having an affinity for phosphatidylserine to react with these extracellular vesicles, the labeling substance and the labeling method, and the like may be appropriately set according to the type of biological specimen, the required measurement sensitivity, the measuring method and measuring device to be used, and the like.

In addition, the amount (concentration) of extracellular vesicles having phosphatidylserine and tetraspanin in the biological specimen may be calculated by creating a calibration curve using a reference standard. Examples of the reference standard include an extracellular vesicle having phosphatidylserine and tetraspanin.

Determination of Alzheimer's disease in the AD diagnosis assisting method contains determining Alzheimer's disease using the amount of extracellular vesicles having phosphatidylserine and tetraspanin as an indicator.

Examples of the determination of Alzheimer's disease include determination of whether or not a subject is likely to have Alzheimer's disease, determination of whether or not a subject may have Alzheimer's disease, determination of whether or not a subject is at high risk of developing Alzheimer's disease, and determination of whether or not a subject is at risk of developing Alzheimer's disease, among which the determination of whether or not a subject is likely to have Alzheimer's disease or the determination of whether or not a subject may have Alzheimer's disease is preferable.

The determination of Alzheimer's disease in the AD diagnosis assisting method is made, for example, by comparing the amount of extracellular vesicles having phosphatidylserine and tetraspanin in a biological specimen derived from a subject, obtained by measuring the amount of extracellular vesicles having phosphatidylserine and tetraspanin, with a predetermined reference value (cutoff value).

Specifically, in a case where the amount of extracellular vesicles having phosphatidylserine and tetraspanin is equal to or less than a predetermined reference value, it can be determined that a subject is likely to have Alzheimer's disease; or a subject may have Alzheimer's disease; or a subject is at high risk of developing Alzheimer's disease; or a subject is at risk of developing Alzheimer's disease.

In addition, in a case where the amount of extracellular vesicles having phosphatidylserine and tetraspanin is larger than a predetermined reference value, it can be determined that a subject is unlikely to have Alzheimer's disease; or a subject may not have Alzheimer's disease; or a subject is at low risk of developing Alzheimer's disease; or a subject is not at risk of developing Alzheimer's disease.

The predetermined reference value (cutoff value) is an amount of extracellular vesicles having phosphatidylserine and tetraspanin in a biological specimen, which is preset to distinguish between a patient with Alzheimer's disease and a healthy subject.

The method for determining the predetermined reference value is not particularly limited. For example, the predetermined reference value can be determined in such a manner that an amount of extracellular vesicles having phosphatidylserine and tetraspanin contained in a biological specimen obtained from a patient suffering from Alzheimer's disease and a healthy subject is measured, and a statistical analysis such as receiver operating characteristic analysis (ROC analysis) is carried out using the obtained amount of extracellular vesicles having phosphatidylserine and tetraspanin. In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

**Reagent kit for assisting diagnosis of Alzheimer's disease** (the *in vitro* use of a kit is claimed)

The reagent kit for assisting the diagnosis of Alzheimer's disease in the first invention-1 (hereinafter, often referred to simply as "reagent kit for assisting the diagnosis of AD") contains a substance having an affinity for tetraspanin and a substance having an affinity for phosphatidylserine.

The substance having an affinity for tetraspanin and the substance having an affinity for phosphatidylserine in the reagent kit for assisting the diagnosis of AD are the same as those described above in the AD diagnosis assisting method, and preferred ones thereof are also the same.

The substance having an affinity for tetraspanin and the substance having an affinity for phosphatidylserine each may be in a solution state, a frozen state, a dried state, or a freeze-dried state. In addition, the substance having an affinity for tetraspanin and the substance having an affinity for phosphatidylserine may be contained in the kit as one reagent or may be contained in the kit as separate reagents.

The combination of the substance having an affinity for tetraspanin and the substance having an affinity for phosphatidylserine in the reagent kit for assisting the diagnosis of AD includes the same combinations as those described above in the AD diagnosis assisting method, and preferred combinations thereof are also the same.

The substance having an affinity for tetraspanin or/and the substance having an affinity for phosphatidylserine in the reagent kit for assisting the diagnosis of AD may be immobilized on a solid phase and may be labeled with a labeling substance. The solid phase, the method for immobilizing the substance on the solid phase, the labeling substance, and the labeling method are the same as those described above in the AD diagnosis assisting method, and preferred ones thereof are also the same.

The reagent kit for assisting the diagnosis of AD may further contain a secondary affinity substance (for example, a secondary antibody) that specifically binds to the substance having an affinity for tetraspanin or/and the substance having an affinity for phosphatidylserine. The secondary affinity substance in the reagent kit for assisting the diagnosis of AD is the same as that described above in the AD diagnosis assisting method, and preferred ones thereof are also the same. In addition, the secondary affinity substance in the reagent kit for assisting the diagnosis of AD may be labeled with a labeling substance. The labeling substance and the labeling method are the same as those described above in the AD diagnosis assisting method and preferred ones thereof are also the same.

The substance having an affinity for tetraspanin or/and the substance having an affinity for phosphatidylserine in the reagent kit for assisting the diagnosis of AD may be a substance to which one of avidin and biotin is bound. The avidin and the biotin are the same as those described above in the AD diagnosis assisting method, and preferred ones thereof are also the same.

The reagent kit for assisting the diagnosis of AD may further contain a labeling substance to which one of avidin and biotin is bound. The labeling substance and labeling method are the same as those described above in the AD diagnosis assisting method, and preferred ones thereof are also the same.

The concentration (amount) of the substance having an affinity for tetraspanin and the substance having an affinity for phosphatidylserine in the reagent kit for assisting the diagnosis of AD may be appropriately set within the range commonly used in this field depending on the measuring method. For example, the substance having an affinity for tetraspanin is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection. In addition, for example, the substance having an affinity for phosphatidylserine is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, reagents commonly used in this field, for example, a buffer, a reaction promoter, a sugar, a protein, a salt, a stabilizer such as surfactant, and a preservative, may coexist with the substance having an affinity for tetraspanin or/and the substance having an affinity for phosphatidylserine. The concentration and pH of these reagents may be appropriately selected from the ranges commonly used in this field.

Further, the reagent kit for assisting the diagnosis of AD may contain, in addition to the substance having an affinity for tetraspanin and the substance having an affinity for phosphatidylserine, a reagent needed to measure an amount of extracellular vesicles having tetraspanin and phosphatidylserine using these affinity substances. Examples of such a reagent include a washing agent, a specimen diluting solution (a reagent for diluting a specimen), a reagent for detecting a labeling substance, a reagent for binding a labeling substance to these affinity substances, a reagent for immobilizing these affinity substances on a solid phase, and a reagent for binding avidin or biotin to these affinity substances and labeling substances. The concentration, pH, and the like of these reagents may be appropriately selected from the ranges commonly used in this field.

The reagent kit for assisting the diagnosis of AD may contain a reference standard used to create a calibration curve for extracellular vesicles having phosphatidylserine and tetraspanin. Examples of the reference standard include an extracellular vesicle having phosphatidylserine and tetraspanin. The reference standard may be in a solution state, a frozen state, a dried state, or a freeze-dried state. In addition, reagents commonly used in this field, for example, a buffer, a reaction promoter, a sugar, a protein, a salt, a stabilizer such as a surfactant, and a preservative, may coexist with the reference standard. The concentration and pH of these reagents may be appropriately selected from the ranges commonly used in this field.

The reagent kit for assisting the diagnosis of AD may contain a package insert or an instruction manual. Examples of the package insert or the instruction manual include a package insert or instruction manual stating that a subject is determined to have Alzheimer's disease by measuring an amount of extracellular vesicles having phosphatidylserine and tetraspanin in a biological specimen derived from a subject or/and using the amount of extracellular vesicles having phosphatidylserine and tetraspanin as an indicator. These package insert and instruction manual may be described separately in a plurality of cases, or may be described collectively in one.

Specifically, the reagent kit for assisting the diagnosis of AD may be, for example, a kit containing one of a substance having an affinity for tetraspanin and a substance having an affinity for phosphatidylserine immobilized on a solid phase and the other one of the substance having an affinity for tetraspanin and the substance having an affinity for phosphatidylserine bound to a labeling substance or the other one affinity substance and a component for indirectly binding the other one affinity substance to a labeling substance. The reagent kit for assisting the diagnosis of AD is preferably, for example, a kit containing either one of the substance having an affinity for tetraspanin and the substance having an affinity for phosphatidylserine immobilized on a solid phase and any one selected from the following (1) to (3). (1) the other one of the substance having an affinity for tetraspanin and the substance having an affinity for phosphatidylserine bound to a labeling substance, (2) the other one of the substance having an affinity for tetraspanin and the substance having an affinity for phosphatidylserine, and a secondary affinity substance that specifically binds to the affinity substance bound to a labeling substance, and (3) the other one of the substance having an affinity for tetraspanin and the substance having an affinity for phosphatidylserine to which one of avidin and biotin is bound, and a labeling substance to which the other one of avidin and biotin is bound.

Preferred specific examples of the reagent kit for assisting the diagnosis of AD include a kit containing a solid phase plate on which an antibody (more preferably an anti-CD9 antibody or an anti-CD63 antibody and particularly preferably the anti-CD9 antibody) or Tim protein (more preferably Tim4 or Tim1 and particularly preferably the Tim4) that specifically binds to phosphatidylserine is immobilized, and any one selected from the following (1) to (3). (1) a solution containing the other one of the antibody or Tim protein that specifically binds to phosphatidylserine bound to a labeling substance (usually 10 to 5,000 ng/mL and preferably 100 to 500 ng/mL), (2) a solution containing the other one of the antibody or Tim protein that specifically binds to phosphatidylserine (usually 10 to 5,000 ng/mL and preferably 100 to 500 ng/mL), and a solution containing a secondary affinity substance that specifically binds to the affinity substance bound to a labeling substance (usually 10 to 5,000 ng/mL and preferably 100 to 500 ng/mL), and (3) a solution containing an antibody (more preferably an anti-CD9 antibody or an anti-CD63 antibody and particularly preferably the anti-CD9 antibody) that specifically binds to tetraspanin to which one of avidin and biotin is bound (usually 10 to 5,000 ng/mL and preferably 100 to 500 ng/mL), and a solution containing a labeling substance to which the other one of avidin and biotin is bound (usually 10 to 5,000 ng/mL and preferably 100 to 500 ng/mL).

**Device for assisting diagnosis of Alzheimer's disease** (not claimed, for reference only)

The device for assisting the diagnosis of Alzheimer's disease in the first invention-1 (hereinafter, often referred to simply as "device for assisting the diagnosis of AD") has a measurement unit that measures an amount of extracellular vesicles having phosphatidylserine and tetraspanin in a biological specimen derived from a subject, and a determination unit that determines that the subject has Alzheimer's disease using the amount of extracellular vesicles having phosphatidylserine and tetraspanin in the biological specimen as an indicator.

The biological specimen, the subject, and the extracellular vesicle in the device for assisting the diagnosis of AD are the same as those described above, and preferred ones thereof are also the same.

The measurement unit, the determination unit, and the like constituting the device for assisting the diagnosis of AD may be arranged in the same device or may be separate bodies.

The measurement unit in the device for assisting the diagnosis of AD is a site for measuring the amount of extracellular vesicles having phosphatidylserine and tetraspanin in the biological specimen introduced into the device. The size and configuration of the measurement unit are not particularly limited. Examples of the measurement unit include an imaging apparatus for imaging using a microplate reader or CCD used for ELISA, an imaging apparatus used for Western blotting or a method using a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The extracellular vesicles having phosphatidylserine and tetraspanin, the amount, the measurement target of the method for measuring the amount of extracellular vesicles having phosphatidylserine and tetraspanin, and the measurement of extracellular vesicles having phosphatidylserine and tetraspanin and the substances used for the measurement in the device for assisting the diagnosis of AD are the same as those of the AD diagnosis assisting method, and preferred ones and specific examples thereof are also the same.

The device for assisting the diagnosis of AD may contain a calculation unit. The calculation unit in the device for assisting the diagnosis of AD is a site for converting an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index) having a correlation with the mass or concentration of extracellular vesicles having phosphatidylserine and tetraspanin in the biological specimen obtained by the measurement unit into mass, concentration, or the like.

It should be noted that the actually measured value, and the mass, concentration, or the like converted by the calculation unit may be stored in a storage device or the like provided in a device such as a memory device or a hard disk.

The determination unit in the device for assisting the diagnosis of AD is a site for determining Alzheimer's disease using the amount of extracellular vesicles having phosphatidylserine and tetraspanin obtained by the measurement unit or the calculation unit as an indicator.

The determination of Alzheimer's disease and the predetermined reference value used for the determination in the device for assisting the diagnosis of AD, and the method for determining the predetermined reference value are the same as those described above in the AD diagnosis assisting method and preferred ones and specific examples thereof are also the same.

The predetermined reference value in the device for assisting the diagnosis of AD may be stored in advance in the device for assisting the diagnosis of AD, or may be input from an input site of the device for assisting the diagnosis of AD at the time of determination.

The device for assisting the diagnosis of AD may contain an output unit. The output unit carries out processing such as displaying or outputting the results of determination to a display device such as a display or a printing device such as a printer.

### 1-2. First invention-2

**Biomarker set for assisting diagnosis of Alzheimer's disease** (the *in vitro* use of a biomarker set is claimed)

The biomarker set for assisting the diagnosis of Alzheimer's disease in the first invention-2 (hereinafter, often referred to simply as "AD marker set") is a combination of biomarkers containing extracellular vesicles having phosphatidylserine and tetraspanin and extracellular vesicles having tetraspanin.

According to the AD marker set, for example, a ratio of the amount of extracellular vesicles having phosphatidylserine and tetraspanin to the amount of extracellular vesicles having tetraspanin (hereinafter, often referred to simply as "AD marker (II)") can be obtained, and the ratio can be used as an indicator for determining that a subject has Alzheimer's disease.

The extracellular vesicle in the AD marker set is the same as the extracellular vesicle described above, and preferred ones thereof are also the same.

The extracellular vesicle having phosphatidylserine and tetraspanin in the AD marker set is the same as that of the AD marker, and preferred ones thereof are also the same.

The extracellular vesicle having tetraspanin in the AD marker set has at least one tetraspanin such as CD9, CD63, CD81, and CD151 on the membrane surface and preferably has at least one tetraspanin selected from CD9, CD63, and CD81, more preferably at least one tetraspanin selected from CD9 and CD63, and particularly preferably CD9.

The tetraspanin in the extracellular vesicle having phosphatidylserine and tetraspanin in the AD marker set may be the same as or different from the tetraspanin in the extracellular vesicle having tetraspanin, and is preferably the same as the tetraspanin in the extracellular vesicle having tetraspanin.

### Method (II) of assisting diagnosis of Alzheimer's disease

The method for assisting the diagnosis of Alzheimer's disease in the first invention-2 (hereinafter, often referred to simply as "AD diagnosis assisting method (II)") contains measuring an amount of extracellular vesicles having phosphatidylserine and tetraspanin in a biological specimen and an amount of extracellular vesicles having tetraspanin in the biological specimen; calculating a ratio of the amount of extracellular vesicles having phosphatidylserine and tetraspanin to the amount of extracellular vesicles having tetraspanin (AD marker (II)); and determining that a subject has Alzheimer's disease using the ratio of the amount of extracellular vesicles having phosphatidylserine and tetraspanin to the amount of extracellular vesicles having tetraspanin as an indicator.

The biological specimen, the subject, and the extracellular vesicle in the AD diagnosis assisting method (II) are the same as those described above, and preferred ones thereof are also the same.

The extracellular vesicles having phosphatidylserine and tetraspanin, the amount, the target for measuring the amount of extracellular vesicles having phosphatidylserine and tetraspanin, and the measurement of the amount of extracellular vesicles having phosphatidylserine and tetraspanin and the substances used for the measurement in the AD diagnosis assisting method (II) are the same as those of the AD diagnosis assisting method, and preferred ones and specific examples thereof are also the same.

The extracellular vesicle having tetraspanin in the AD diagnosis assisting method (II) is the same as that described above in the AD marker set, and preferred ones thereof are also the same.

The tetraspanin in the extracellular vesicle having phosphatidylserine and tetraspanin in the AD diagnosis assisting method (II) may be the same as or different from the tetraspanin in the extracellular vesicle having tetraspanin, and is preferably the same as the tetraspanin in the extracellular vesicle having tetraspanin.

The measurement of the amount of extracellular vesicles having tetraspanin in the AD diagnosis assisting method (II) may be carried out by measuring an amount of one type of extracellular vesicles having tetraspanin (for example, only extracellular vesicles having CD9) or by measuring an amount of two or more types of extracellular vesicles having tetraspanin (for example, extracellular vesicles having CD9 and extracellular vesicles having CD63). It is preferable to measure the amount of only one type of extracellular vesicles having tetraspanin.

The method for measuring the amount of extracellular vesicles having tetraspanin is not particularly limited as long as it is a method commonly used in this field, and examples thereof include an immunoassay using a substance having an affinity for tetraspanin, a mass spectrometry, and a method combining these methods, among which the immunoassay using a substance having an affinity for tetraspanin is preferable. It should be noted that the immunoassay also contains, in addition to a method using an immune reaction (antigen-antibody reaction), a method using a binding force between two molecules other than the antigen-antibody reaction (method according to the immunoassay).

Examples of the substance having an affinity for tetraspanin include the same substances as those described above in the AD diagnosis assisting method, among which a protein that specifically binds to tetraspanin is preferable, and an antibody that specifically binds to tetraspanin is more preferable. Examples of the antibody that specifically binds to tetraspanin include an anti-CD9 antibody, an anti-CD63 antibody, an anti-CD81 antibody, and an anti-CD151 antibody, among which the anti-CD9 antibody or the anti-CD63 antibody is preferable, and the anti-CD9 antibody is more preferable. Only one type of the substance having an affinity for tetraspanin may be used, or two or more types of the substances having an affinity for tetraspanin may be used. It is preferable to use only one type of the substance having an affinity for tetraspanin.

The substance having an affinity for tetraspanin may be a commercially available product or a substance appropriately prepared by a conventional method. In addition, the antibody that specifically binds to tetraspanin may be either a polyclonal antibody or a monoclonal antibody, and these antibodies may be used alone or in combination thereof as appropriate. In addition, not only an immunoglobulin molecule itself (intact immunoglobulin), but also a fragment antibody such as Fab, F(ab')2, or F(ab'), which is a fragment of the immunoglobulin molecule and has an ability to bind to an antigen, or a synthetic antibody such as a single chain antibody (single chain Fv), a diabody, a triabody, or a tetrabody may be used as the antibody that specifically binds to tetraspanin. In addition, in a case where these antibodies are prepared, the antibodies may be prepared, for example, according to the method described in "Immunoassay" (edited by Biochemical Assay Society of JAPAN, Kodansha Ltd., 2014).

The substance having an affinity for tetraspanin in the AD diagnosis assisting method (II) may be labeled with a labeling substance. The labeling substance and the labeling method are the same as those of the AD diagnosis assisting method and preferred ones and specific examples thereof are also the same.

In addition, in the same manner as in the AD diagnosis assisting method, using the substance having an affinity for tetraspanin as a primary affinity substance, a secondary affinity substance (for example, a secondary antibody) that specifically binds to the primary affinity substance may be further used. The secondary affinity substance may be labeled with a labeling substance. The labeling substance and the labeling method are the same as those described above in the AD diagnosis assisting method and preferred ones thereof are also the same.

Further, labeling with a labeling substance may be carried out taking advantage of avidin-biotin binding using the substance having an affinity for tetraspanin to which one of avidin and biotin is bound, and a labeling substance to which the other one of avidin and biotin is bound. The avidin, the biotin, and the method for binding avidin or biotin to the substance having an affinity for tetraspanin are the same as those described above in the AD diagnosis assisting method, and preferred ones and specific examples thereof are also the same.

The substance having an affinity for tetraspanin in the AD diagnosis assisting method (II) may be immobilized on a solid phase. The solid phase and the method for immobilizing the substance having an affinity for tetraspanin on the solid phase are the same as those described above in the AD diagnosis assisting method, and preferred ones and specific examples thereof are also the same.

With regard to the substance having an affinity for tetraspanin used for measuring extracellular vesicles having phosphatidylserine and tetraspanin and the substance having an affinity for tetraspanin used for measuring extracellular vesicles having tetraspanin in the AD diagnosis assisting method (II), asubstance having an affinity for at least one same tetraspanin may be used (for example, a substance having an affinity for at least CD9 is used for the measurement of both), or substances having an affinity for different types of tetraspanins may be used (for example, a substance having an affinity for CD9 is used for the measurement of one, and a substance having an affinity for CD63 is used for the measurement of the other one). It is preferable to use a substance having an affinity for at least one same tetraspanin; it is more preferable to use only a substance having an affinity for the same tetraspanin (for example, only a substance having an affinity for CD9 is used for the measurement of both); and it is particularly preferable to use only one type of substance having an affinity for the same tetraspanin (for example, only an anti-CD9 antibody is used for the measurement of both).

The measurement and measurement principle of the amount of extracellular vesicles having tetraspanin in the AD diagnosis assisting method (II) are the same as those of the AD diagnosis assisting method, and preferred ones thereof are also the same.

The measurement of the amount of extracellular vesicles having tetraspanin in the AD diagnosis assisting method (II) specifically includes, for example, a method containing (1) a step of bringing extracellular vesicles having tetraspanin in a biological specimen into contact with a substance having an affinity for tetraspanin to form a complex containing the extracellular vesicles having tetraspanin in the biological specimen and the substance having an affinity for tetraspanin (complex forming step), and (2) a step of measuring an amount of the complex (complex amount measuring step) as a preferable method.

The complex forming step in the measurement of the amount of extracellular vesicles having tetraspanin in the AD diagnosis assisting method (II) preferably contains a first procedure of bringing a biological specimen into contact with a substance having an affinity for tetraspanin to form a first complex composed of extracellular vesicles in the biological specimen and the substance having an affinity for tetraspanin, and a second procedure of bringing the first complex into contact with a substance having an affinity for tetraspanin to form a second complex composed of the first complex and the substance having an affinity for tetraspanin.

It is preferable that the substance having an affinity for tetraspanin used for forming the first complex and the substance having an affinity for tetraspanin used for forming the second complex are the same.

The complex forming step in the measurement of the amount of extracellular vesicles having tetraspanin in the AD diagnosis assisting method (II) is specifically carried out, for example, in such a manner that an antibody that specifically binds to tetraspanin (preferably an anti-CD9 antibody or an anti-CD63 antibody and more preferably the anti-CD9 antibody) immobilized on a solid phase is brought into contact with a biological specimen to form a first complex of the antibody that specifically binds to tetraspanin and extracellular vesicles having phosphatidylserine and tetraspanin in the biological specimen, and the first complex is brought into contact with an antibody that specifically binds to tetraspanin (preferably an anti-CD9 antibody or an anti-CD63 antibody and more preferably the anti-CD9 antibody) to form a second complex of the first complex and the antibody that specifically binds to tetraspanin.

After forming the complex, it is preferable to carry out a washing operation (B/F separation) at least before the complex amount measuring step.

Specifically, for example, the washing operation may be carried out after forming the first complex or/and after forming the second complex in the above method. It is preferable to carry out the washing operation (B/F separation) after forming the first complex, and further carry out the washing operation (B/F separation) after forming the second complex.

The complex amount measuring step in the measurement of the amount of extracellular vesicles having tetraspanin in the AD diagnosis assisting method (II) is a step of measuring an amount of the complex containing extracellular vesicles having tetraspanin and a substance having an affinity for tetraspanin obtained in the complex forming step. Any method may be used as long as the amount of the complex can be measured.

More specifically, the complex amount measuring step may detect a labeling substance in the complex containing an antibody that specifically binds to tetraspanin (preferably an anti-CD9 antibody or an anti-CD63 antibody and more preferably the anti-CD9 antibody) immobilized on a solid phase plate, an extracellular vesicle having tetraspanin in a biological specimen, an antibody that specifically binds to tetraspanin (preferably an anti-CD9 antibody or an anti-CD63 antibody and more preferably the anti-CD9 antibody), and a labeling substance (preferably an enzyme or a fluorescent substance) obtained in the complex forming step using, for example, (1) an antibody that specifically binds to tetraspanin labeled with a labeling substance, (2) a labeled secondary antibody labeled with a labeling substance, which specifically binds to an "antibody that specifically binds to tetraspanin", or (3) an antibody that specifically binds to tetraspanin to which one of avidin and biotin is bound and a labeling substance to which the other one of avidin and biotin is bound.

In the complex amount measuring step, it is preferable to carry out a washing operation (B/F separation) before detecting a labeling substance.

More specifically, the measurement of the amount of extracellular vesicles having tetraspanin in the AD diagnosis assisting method (II) may be carried out, for example, in such a manner that an antibody that specifically binds to tetraspanin (preferably an anti-CD9 antibody or an anti-CD63 antibody and more preferably the anti-CD9 antibody) immobilized on a solid phase plate and a biological specimen are brought into contact with each other to form a first complex of the antibody that specifically binds to tetraspanin and an extracellular vesicle having tetraspanin in the biological specimen, followed by B/F separation if necessary, (1) the first complex is brought into contact with an antibody that specifically binds to tetraspanin (preferably an anti-CD9 antibody or an anti-CD63 antibody and more preferably the anti-CD9 antibody) labeled with a labeling substance to form a second complex of the first complex and the antibody that specifically binds to tetraspanin labeled with a labeling substance, (2) the first complex is brought into contact with an antibody that specifically binds to tetraspanin (preferably an anti-CD9 antibody or an anti-CD63 antibody and more preferably the anti-CD9 antibody) to form a second complex of the first complex and the antibody that specifically binds to tetraspanin, followed by B/F separation if necessary, and the second complex is brought into contact with a labeled secondary antibody labeled with a labeling substance, which specifically binds to "the antibody that specifically binds to tetraspanin", to form a third complex of the second complex and the labeled secondary antibody, or (3) the first complex is brought into contact with an antibody that specifically binds to tetraspanin (preferably an anti-CD9 antibody or an anti-CD63 antibody and more preferably the anti-CD9 antibody) to which one of avidin and biotin is bound to form a second complex of the first complex and the antibody that specifically binds to tetraspanin to which one of avidin and biotin is bound, followed by B/F separation if necessary, a third complex of the second complex and a labeling substance (preferably an enzyme or a fluorescent substance) to which the other one of avidin and biotin is bound is formed, followed by B/F separation, and then the labeling substance (preferably an enzyme or a fluorescent substance) of the obtained second complex or third complex is detected.

The amount of the biological specimen and the amount (concentration) of protein in the biological specimen, the amount (concentration) and number of particles of extracellular vesicles having tetraspanin in the biological specimen, the amount (concentration) of the substance having an affinity for tetraspanin to react with these extracellular vesicles, the labeling substance and the labeling method, and the like may be appropriately set according to the type of biological specimen, the required measurement sensitivity, the measuring method and measuring device to be used, and the like.

In addition, the amount (concentration) of extracellular vesicles having tetraspanin in the biological specimen may be calculated by creating a calibration curve using a reference standard. Examples of the reference standard include an extracellular vesicle having tetraspanin.

The ratio of the amount of extracellular vesicles having phosphatidylserine and tetraspanin to the amount of extracellular vesicles having tetraspanin may be calculated by dividing the amount (A) of extracellular vesicles having phosphatidylserine and tetraspanin by the amount (B) of extracellular vesicles having tetraspanin ((A)/(B)).

The determination of Alzheimer's disease in the AD diagnosis assisting method (II) contains determining Alzheimer's disease using the ratio ((A)/(B)) of the amount (B) of extracellular vesicles having phosphatidylserine and tetraspanin to the amount (A) of extracellular vesicles having tetraspanin as an indicator.

Examples of the determination of Alzheimer's disease include determination of whether or not a subject is likely to have Alzheimer's disease, determination of whether or not a subject may have Alzheimer's disease, determination of whether or not a subject is at high risk of developing Alzheimer's disease, and determination of whether or not a subject is at risk of developing Alzheimer's disease, among which the determination of whether or not a subject is likely to have Alzheimer's disease or the determination of whether or not a subject may have Alzheimer's disease is preferable.

The determination of Alzheimer's disease in the AD diagnosis assisting method is made, for example, by comparing the ratio ((A)/(B)) of the amount (B) of extracellular vesicles having phosphatidylserine and tetraspanin to the amount (A) of extracellular vesicles having tetraspanin, which is obtained by measuring the amount of extracellular vesicles having tetraspanin and measuring the amount of extracellular vesicles having phosphatidylserine and tetraspanin, with a predetermined reference value (cutoff value).

Specifically, in a case where the ratio ((A)/(B)) of the amount (B) of extracellular vesicles having phosphatidylserine and tetraspanin to the amount (A) of extracellular vesicles having tetraspanin is equal to or less than a predetermined reference value, it can be determined that a subject is likely to have Alzheimer's disease; or a subject may have Alzheimer's disease; or a subject is at high risk of developing Alzheimer's disease; or a subject is at risk of developing Alzheimer's disease.

In addition, in a case where the ratio ((A)/(B)) of the amount (B) of extracellular vesicles having phosphatidylserine and tetraspanin to the amount (A) of extracellular vesicles having tetraspanin is larger than a predetermined reference value, it can be determined that a subject is unlikely to have Alzheimer's disease; or a subject may not have Alzheimer's disease; or a subject is at low risk of developing Alzheimer's disease; or a subject is not at risk of developing Alzheimer's disease.

The predetermined reference value (cutoff value) in the AD diagnosis assisting method (II) is set in advance to distinguish between a patient with Alzheimer's disease and a healthy subject, and is a ratio ((A)/(B)) of an amount (B) of extracellular vesicles having phosphatidylserine and tetraspanin to an amount (A) of extracellular vesicles having tetraspanin in a biological specimen.

The method for determining the predetermined reference value in the AD diagnosis assisting method (II) is not particularly limited. For example, the predetermined reference value can be determined in such a manner that an amount (B) of extracellular vesicles having phosphatidylserine and tetraspanin to an amount (A) of extracellular vesicles having tetraspanin contained in a biological specimen obtained from a patient suffering from Alzheimer's disease and a healthy subject is measured to calculate a ratio ((A)/(B)) of the amount (B) of extracellular vesicles having phosphatidylserine and tetraspanin to the amount (A) of extracellular vesicles having tetraspanin, and a statistical analysis such as receiver operating characteristic analysis (ROC analysis) is carried out using the obtained ratio ((A)/(B)) of the amount (B) of extracellular vesicles having phosphatidylserine and tetraspanin to the amount (A) of extracellular vesicles having tetraspanin. In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

**Reagent kit (II) for assisting diagnosis of Alzheimer's disease** (the *in vitro* use of a kit is claimed)

The reagent kit for assisting the diagnosis of Alzheimer's disease in the first invention-2 (hereinafter, often referred to simply as "reagent kit (II) for assisting the diagnosis of AD") contains a substance having an affinity for tetraspanin and a substance having an affinity for phosphatidylserine.

The substance having an affinity for tetraspanin and the substance having an affinity for phosphatidylserine in the reagent kit (II) for assisting the diagnosis of AD are the same as those in the AD diagnosis assisting method (II), and preferred ones thereof are also the same.

The substance having an affinity for tetraspanin and the substance having an affinity for phosphatidylserine each may be in a solution state, a frozen state, a dried state, or a freeze-dried state. In addition, the substance having an affinity for tetraspanin and the substance having an affinity for phosphatidylserine may be contained in the kit as one reagent or may be contained in the kit as separate reagents.

The combination of the substance having an affinity for tetraspanin and the substance having an affinity for phosphatidylserine in the reagent kit (II) for assisting the diagnosis of AD includes the same combinations as the combinations described above in the AD diagnosis assisting method, and preferred combinations thereof are also the same.

The substance having an affinity for tetraspanin or/and the substance having an affinity for phosphatidylserine in the reagent kit (II) for assisting the diagnosis of AD may be immobilized on a solid phase and may be labeled with a labeling substance. The solid phase, the method for immobilizing the substance on the solid phase, the labeling substance, and the labeling method are the same as those described above in the AD diagnosis assisting method, and preferred ones thereof are also the same.

The reagent kit (II) for assisting the diagnosis of AD may further contain a secondary affinity substance (for example, a secondary antibody) that specifically binds to the substance having an affinity for tetraspanin or/and the substance having an affinity for phosphatidylserine. The secondary affinity substance in the reagent kit (II) for assisting the diagnosis of AD is the same as that described above in the AD diagnosis assisting method, and preferred ones thereof are also the same. In addition, the secondary affinity substance in the reagent kit (II) for assisting the diagnosis of AD may be labeled with a labeling substance. The labeling substance and the labeling method are the same as those described above in the AD diagnosis assisting method and preferred ones thereof are also the same.

The substance having an affinity for tetraspanin or/and the substance having an affinity for phosphatidylserine in the reagent kit (II) for assisting the diagnosis of AD may be a substance to which one of avidin and biotin is bound. The avidin and the biotin are the same as those described above in the AD diagnosis assisting method, and preferred ones thereof are also the same.

The reagent kit (II) for assisting the diagnosis of AD may contain a labeling substance to which one of avidin and biotin is bound. The labeling substance and labeling method are the same as those described above in the AD diagnosis assisting method, and preferred ones thereof are also the same.

The concentration (amount) of the substance having an affinity for tetraspanin and the substance having an affinity for phosphatidylserine in the reagent kit (II) for assisting the diagnosis of AD may be appropriately set within the range commonly used in this field depending on the measuring method. For example, the substance having an affinity for tetraspanin is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection. In addition, for example, the substance having an affinity for phosphatidylserine is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, the substance having an affinity for tetraspanin and the substance having an affinity for phosphatidylserine in the reagent kit (II) for assisting the diagnosis of AD may coexist with a reagent commonly used in this field. The reagent is the same as that of the reagent kit for assisting the diagnosis of AD.

Further, the reagent kit (II) for assisting the diagnosis of AD may contain, in addition to the substance having an affinity for tetraspanin and the substance having an affinity for phosphatidylserine, a reagent needed to measure an amount of extracellular vesicles having tetraspanin and phosphatidylserine or/and amount of extracellular vesicles having phosphatidylserine using these affinity substances. Such a reagent is the same as that of the reagent kit for assisting the diagnosis of AD.

The reagent kit (II) for assisting the diagnosis of AD may contain a reference standard used for creating a calibration curve for extracellular vesicles having phosphatidylserine and tetraspanin. The reference standard is the same as that of the reagent kit for assisting the diagnosis of AD.

In addition, the reagent kit (II) for assisting the diagnosis of AD may contain a reference standard used for creating a calibration curve for extracellular vesicles having tetraspanin. Examples of the reference standard include an extracellular vesicle having tetraspanin. The reference standard may be in a solution state, a frozen state, a dried state, or a freeze-dried state. In addition, reagents commonly used in this field, for example, a buffer, a reaction promoter, a sugar, a protein, a salt, a stabilizer such as a surfactant, and a preservative, may coexist with the reference standard. The concentration and pH of these reagents may be appropriately selected from the ranges commonly used in this field.

The reagent kit (II) for assisting the diagnosis of AD may contain a package insert or an instruction manual. Examples of the package insert or the instruction manual include a package insert or instruction manual stating that a subject is determined to have Alzheimer's disease by measuring an amount of extracellular vesicles having tetraspanin and an amount of extracellular vesicles having phosphatidylserine and tetraspanin in a biological specimen derived from the subject or/and using a ratio of the amount of extracellular vesicles having phosphatidylserine and tetraspanin to the amount of extracellular vesicles having tetraspanin as an indicator. These package insert and instruction manual may be described separately in a plurality of cases, or may be described collectively in one.

Specific examples of the reagent kit (II) for assisting the diagnosis of AD include a kit containing the following (A) and (B).

(A) one of a substance having an affinity for tetraspanin and a substance having an affinity for phosphatidylserine immobilized on a solid phase and the other one of the substance having an affinity for tetraspanin and the substance having an affinity for phosphatidylserine bound to a labeling substance or the other one affinity substance and a component for indirectly binding the other one affinity substance to a labeling substance.

(B) a substance having an affinity for tetraspanin immobilized on a solid phase and a substance having an affinity for tetraspanin bound to a labeling substance or the affinity substance and a component for indirectly binding the affinity substance to a labeling substance.

The reagent kit (II) for assisting the diagnosis of AD is preferably, for example, a kit containing the following (C) and (D).

(C) one of a substance having an affinity for tetraspanin and a substance having an affinity for phosphatidylserine immobilized on a solid phase and one selected from the following (1) to (3).
(1) the other one of the substance having an affinity for tetraspanin and the substance having an affinity for phosphatidylserine bound to a labeling substance, (2) the other one of the substance having an affinity for tetraspanin and the substance having an affinity for phosphatidylserine, and a secondary affinity substance that specifically binds to the affinity substance bound to a labeling substance, and (3) the other one of the substance having an affinity for tetraspanin and the substance having an affinity for phosphatidylserine to which one of avidin and biotin is bound, and a labeling substance to which the other one of avidin and biotin is bound.
(D) a substance having an affinity for tetraspanin immobilized on a solid phase and one selected from the following (4) to (6).

One selected from (4) a substance having an affinity for tetraspanin bound to a labeling substance, (5) a substance having an affinity for tetraspanin, and a secondary affinity substance, which specifically binds to a substance having an affinity for tetraspanin, bound to a labeling substance, and (6) a substance having an affinity for tetraspanin bound to one of avidin and biotin, and the other one of avidin and biotin bound to a labeling substance.

Preferred examples of the reagent kit (II) for assisting the diagnosis of AD include the following.

For example, there is a kit containing a solid phase plate on which an antibody or Tim protein (more preferably Tim4 or Tim1 and particularly preferably the Tim4) that specifically binds to phosphatidylserine is immobilized, a solid phase plate on which a tetraspanin antibody (more preferably an anti-CD9 antibody or an anti-CD63 antibody and particularly preferably the anti-CD9 antibody) is immobilized, and any one selected from the following (1) to (3).

(1) a solution containing a tetraspanin antibody (more preferably an anti-CD9 antibody or an anti-CD63 antibody and particularly preferably the anti-CD9 antibody) bound to a labeling substance (usually 10 to 5,000 ng/mL and preferably 100 to 500 ng/mL), (2) a solution containing a tetraspanin antibody (more preferably an anti-CD9 antibody or an anti-CD63 antibody and particularly preferably the anti-CD9 antibody) (usually 10 to 5,000 ng/mL and preferably 100 to 500 ng/mL), and a solution containing a secondary affinity substance that specifically binds to the tetraspanin antibody bound to a labeling substance (usually 10 to 5,000 ng/mL and preferably 100 to 500 ng/mL), and (3) a solution containing an antibody that specifically binds to tetraspanin (more preferably an anti-CD9 antibody or an anti-CD63 antibody and particularly preferably the anti-CD9 antibody) to which one of avidin and biotin is bound (usually 10 to 5,000 ng/mL and preferably 100 to 500 ng/mL), and a solution containing a labeling substance to which the other one of avidin and biotin is bound (usually 10 to 5,000 ng/mL and preferably 100 to 500 ng/mL).

**Device (II) for assisting diagnosis of Alzheimer's disease** (not claimed, for reference only)

The device for assisting the diagnosis of Alzheimer's disease in the first invention-2 (hereinafter, often referred to simply as "device (II) for assisting the diagnosis of AD") has a measurement unit that measures an amount of extracellular vesicles having phosphatidylserine and tetraspanin and an amount of extracellular vesicles having tetraspanin, in a biological specimen derived from a subject; a calculation unit that calculates a ratio of the amount of extracellular vesicles having phosphatidylserine and tetraspanin to the amount of extracellular vesicles having tetraspanin; and a determination unit that determines that the subject has Alzheimer's disease or mild cognitive impairment using the ratio of the amount of extracellular vesicles having phosphatidylserine and tetraspanin in the biological specimen to the amount of extracellular vesicles having tetraspanin in the biological specimen as an indicator.

The measurement unit, the calculation unit, the determination unit, and the like constituting the device (II) for assisting the diagnosis of AD may be arranged in the same device or may be separate bodies.

The measurement unit in the device (II) for assisting the diagnosis of AD is a site for measuring the amount of extracellular vesicles having phosphatidylserine and tetraspanin and the amount of extracellular vesicles having tetraspanin in the biological specimen introduced into the device. The size and configuration of the measurement unit are not particularly limited.

Examples of the measurement unit include an imaging apparatus for imaging using a microplate reader or CCD used for ELISA, an imaging apparatus used for Western blotting or a method using a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The subject, the biological specimen, and the extracellular vesicle in the device (II) for assisting the diagnosis of AD are the same as those described above, and preferred ones thereof are also the same.

The extracellular vesicle having phosphatidylserine and tetraspanin, the amount, and the target for measuring the amount of extracellular vesicles having phosphatidylserine and tetraspanin in the device (II) for assisting the diagnosis of AD are the same as those described above in the AD diagnosis assisting method, and preferred ones thereof are also the same.

The measurement of the amount of extracellular vesicles having phosphatidylserine and tetraspanin and the substances used for the measurement in the device (II) for assisting the diagnosis of AD are the same as those described above in the AD diagnosis assisting method, and preferred ones and specific examples thereof are also the same.

In addition, the extracellular vesicle having tetraspanin and the target for measuring the amount of extracellular vesicles having tetraspanin in the device (II) for assisting the diagnosis of AD are the same as those described above in the AD diagnosis assisting method (II), and preferred ones thereof are also the same.

The measurement of the amount of extracellular vesicles having tetraspanin and the substances used for the measurement in the device (II) for assisting the diagnosis of AD are the same as those described above in the AD diagnosis assisting method (II), and preferred ones and specific examples thereof are also the same.

The calculation unit in the device (II) for assisting the diagnosis of AD is a site for calculating the ratio of the amount of extracellular vesicles having phosphatidylserine and tetraspanin to the amount of extracellular vesicles having tetraspanin, based on the amount of extracellular vesicles having tetraspanin and the amount of extracellular vesicles having phosphatidylserine and tetraspanin obtained by the measurement unit. The size and configuration of the measurement unit are not particularly limited.

The calculation unit in the device (II) for assisting the diagnosis of AD may convert an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, rate of change in turbidity, scattered light, a rate of change in scattered light, a reflectivity, an amount of change in reflectivity, a refractive index, or an amount of change in refractive index) having a correlation with the mass or concentration of extracellular vesicles having phosphatidylserine and tetraspanin and the extracellular vesicles having tetraspanin in the biological specimen obtained by the measurement unit in the device for assisting the diagnosis of AD into mass, concentration, or the like, and then calculate the ratio of the amount of extracellular vesicles having phosphatidylserine and tetraspanin to the amount of extracellular vesicles having tetraspanin.

It should be noted that the actually measured value, the mass, concentration, or the like converted by the calculation unit, and the ratio of the amount of extracellular vesicles having phosphatidylserine and tetraspanin to the amount of extracellular vesicles having tetraspanin may be stored in a storage device or the like provided in a device such as a memory device or a hard disk.

The determination unit in the device (II) for assisting the diagnosis of AD is a site for determining Alzheimer's disease using the ratio of the amount of extracellular vesicles having phosphatidylserine and tetraspanin to the amount of extracellular vesicles having tetraspanin calculated by the calculation unit as an indicator.

The determination of Alzheimer's disease and the predetermined reference value used for the determination in the device (II) for assisting the diagnosis of AD, and the method for determining the predetermined reference value are the same as those described above in the AD diagnosis assisting method (II) and preferred ones and specific examples thereof are also the same.

The predetermined reference value in the device (II) for assisting the diagnosis of AD may be stored in advance in the device (II) for assisting the diagnosis of AD, or may be input from an input site of the device (II) for assisting the diagnosis of AD at the time of determination.

The device (II) for assisting the diagnosis of AD may contain an output unit. The output unit carries out processing such as displaying or outputting the results of the determination to a display device such as a display or a printing device such as a printer.

### 1-3. First invention-3

**Biomarker set for assisting diagnosis of Alzheimer's disease** (not claimed, for reference only)

The biomarker set for assisting the diagnosis of Alzheimer's disease in the first invention-3 (hereinafter, often referred to simply as "AD marker set (III)") is a combination of biomarkers containing extracellular vesicles having phosphatidylserine and tetraspanin, extracellular vesicles having tetraspanin, amyloid $\beta$ (1-40), and amyloid $\beta$ (1-42). That is, the AD marker set (III) is a combination of biomarkers containing the AD marker set (II), amyloid $\beta$ (1-40), and amyloid $\beta$ (1-42).

According to the AD marker set (III), for example, a value (hereinafter, often referred to simply as "AD marker (III)") obtained by multiplying the ratio of the amount of extracellular vesicles having phosphatidylserine and tetraspanin to the amount of extracellular vesicles having tetraspanin ("AD marker (II)") by a ratio of an amount of amyloid $\beta$ (1-42) to an amount of amyloid $\beta$ (1-40) (hereinafter, often referred to simply as "A$\beta$ (1-42)/A$\beta$ (1-40)") can be obtained, and the AD marker (III) can be used as an indicator for determining that a subject has Alzheimer's disease.

All the descriptions regarding the AD marker set (II) in the AD marker set (III) are the same as those in the AD marker set (II) in the first invention-2, and preferred ones and specific examples thereof are also the same.

**Method (III) for assisting diagnosis of Alzheimer's disease** (not claimed, for reference only)

The method for assisting the diagnosis of Alzheimer's disease in the first invention-3 (hereinafter, often referred to simply as "AD diagnosis assisting method (III)") contains measuring an amount of extracellular vesicles having phosphatidylserine and tetraspanin in a biological specimen and an amount of extracellular vesicles having tetraspanin in the biological specimen; calculating a ratio of the amount of extracellular vesicles having phosphatidylserine and tetraspanin to the amount of extracellular vesicles having tetraspanin (AD marker (II)); multiplying a ratio of an amount of amyloid $\beta$ (1-42) to an amount of amyloid $\beta$ (1-40) (A$\beta$ (1-42)/A$\beta$ (1-40)) by an AD marker (II) to calculate an AD marker (III); and determining that a subject has Alzheimer's disease using the AD marker (III) as an indicator.

All the descriptions regarding the AD marker (II) in the AD diagnosis assisting method (III) are the same as those in the AD marker (II) in the first invention-2, and preferred ones and specific examples thereof are also the same.

The AD diagnosis assisting method (III) may further contain measuring the amount of amyloid $\beta$ (1-40) or/and the amount of amyloid $\beta$ (1-42).

The method for measuring the amount of amyloid $\beta$ (1-40) or/and the amount of amyloid $\beta$ (1-42) is not particularly limited as long as it is a method commonly carried out in this field. The measurement of the amount of amyloid $\beta$ (1-40) or/and the amount of amyloid $\beta$ (1-42) may be carried out according to the measurement of the amount of extracellular vesicles having phosphatidylserine and tetraspanin or the measurement of the amount of extracellular vesicles having tetraspanin. For example, an immunoassay using a substance having an affinity for amyloid $\beta$ (1-40) or a substance having an affinity for amyloid $\beta$ (1-42), a mass spectrometry, a method combining these methods, or the like may be used, among which the immunoassay is preferable. It should be noted that the immunoassay also contains, in addition to a method using an immune reaction (antigen-antibody reaction), a method using, for example, a binding force between two components other than the antigen-antibody reaction (method according to the immunoassay).

Examples of the substance having an affinity for amyloid $\beta$ (1-40) include an antibody that specifically binds to amyloid $\beta$ (1-40), which is preferably an antibody that specifically binds to a C-terminal region of amyloid $\beta$ (1-40),

specifically, for example, BA27 (manufactured by FUJIFILM Wako Pure Chemical Corporation). Examples of the C-terminal region of amyloid β (1-40) include a C-terminal region containing a 40th amino acid at the C-terminus of amyloid β (1-40), among which a 40th amino acid residue and a 39th amino acid residue at the C-terminus of amyloid β (1-40), a 40th amino acid residue, a 38th amino acid residue, and a 39th amino acid residue at the C-terminus of amyloid β (1-40), a 40th amino acid residue and 37th to 39th amino acid residues at the C-terminus of amyloid β (1-40), and a 40th amino acid residue and 36th to 39th amino acid residues at the C-terminus of amyloid β (1-40) are preferable. In addition, the substance having an affinity for amyloid β (1-40) is preferably a substance that does not bind to amyloid β (1-42).

Examples of the substance having an affinity for amyloid β (1-42) include an antibody that specifically binds to amyloid β (1-42), which is preferably an antibody that specifically binds to a C-terminal region of amyloid β (1-42), specifically, for example, BC05 (manufactured by FUJIFILM Wako Pure Chemical Corporation). Examples of the C-terminal region of amyloid β (1-42) include a C-terminal region containing a 42nd amino acid at the C-terminus of amyloid β (1-42), among which a 42nd amino acid residue at the C-terminus of amyloid β (1-42), a 42nd amino acid residue and a 41st amino acid residue at the C-terminus of amyloid β (1-42), and the like are preferable. In addition, the substance having an affinity for amyloid β (1-42) is preferably a substance that does not bind to amyloid β (1-40).

The amount of amyloid β (1-40) and the amount of amyloid β (1-42) each may be measured using only a substance having an affinity for amyloid β (1-40) or a substance having an affinity for amyloid β (1-42), or each may be measured using the substance having an affinity for amyloid β (1-40) or the substance having an affinity for amyloid β (1-42) further in combination with a substance having an affinity for an N-terminal region of amyloid β. It is preferable to use the substance having an affinity for amyloid β (1-40) or the substance having an affinity for amyloid β (1-42) in combination with the substance having an affinity for an N-terminal region of amyloid β. Examples of the N-terminal region of amyloid β include amino acid residues 1 to 28 of amyloid β, among which amino acid residues 1 to 16 of amyloid β are preferable. The substance having an affinity for an N-terminal region of amyloid β is preferably an antibody that specifically binds to the N-terminal region of amyloid β, specific examples of which include BAN50 (manufactured by FUJIFILM Wako Pure Chemical Corporation) and BNT77 (manufactured by FUJIFILM Wako Pure Chemical Corporation).

Examples of the combination of the substance having an affinity for amyloid β (1-40) or the substance having an affinity for amyloid β (1-42) and the substance having an affinity for an N-terminal region of amyloid β include a combination of an antibody that specifically binds to amyloid β (1-40) or an antibody that specifically binds to amyloid β (1-42) and an antibody having an affinity for an N-terminal region of amyloid β, among which, specifically, a combination of BA27 or BC05 and BAN50 or BNT77 is preferable.

A commercially available kit may be used for measuring the amount of amyloid β (1-40), and examples thereof include "Human β Amyloid (1-40) ELISA Kit Wako II" (manufactured by FUJIFILM Wako Pure Chemical Corporation).

A commercially available kit may be used for measuring the amount of amyloid β (1-42), and examples thereof include "Human β Amyloid (1-42) ELISA Kit Wako, High-Sensitive" (manufactured by FUJIFILM Wako Pure Chemical Corporation).

The substance having an affinity for amyloid β (1-40), the substance having an affinity for amyloid β (1-42), and the substance having an affinity for an N-terminal region of amyloid β each may be a commercially available product or a substance appropriately prepared by a conventional method. In addition, the antibody that specifically binds to amyloid β (1-40), the antibody that specifically binds to amyloid β (1-42), and the antibody having an affinity for an N-terminal region of amyloid β each may be either a polyclonal antibody or a monoclonal antibody, which may be used alone or in combination thereof as appropriate. Not only an immunoglobulin molecule itself (intact immunoglobulin), but also a fragment antibody such as Fab, F(ab')2, or F(ab'), which is a fragment of the immunoglobulin molecule and has an ability to bind to an antigen, or a synthetic antibody such as a single chain antibody (single chain Fv), a diabody, a triabody, or a tetrabody may be used. In addition, in a case where these antibodies are prepared, the antibodies may be prepared, for example, according to the method described in "Immunoassay" (edited by Biochemical Assay Society of JAPAN, Kodansha Ltd., 2014).

The substance having an affinity for amyloid β (1-40), the substance having an affinity for amyloid β (1-42), and the substance having an affinity for an N-terminal region of amyloid β each may be labeled with a labeling substance. Examples of the labeling substance include an enzyme such as peroxidase, microperoxidase, or alkaline phosphatase; a radioactive isotope such as $^{99m}$Tc, $^{131}$I, $^{125}$I, $^{14}$C, $^{3}$H, $^{32}$P, or $^{35}$S; a fluorescent substance such as fluorescein, fluorescein isothiocyanate (FITC), 4-methylumbelliferone, HiLyte, Alexa, CyDye, rhodamine, or a derivative thereof; a luminescent substance such as luciferin, luminol, or ruthenium complex; a substance having absorption in an ultraviolet region, such as phenol, naphthol, anthracene, or a derivative thereof; a substance having properties as a spin labeling agent represented by a compound having an oxyl group such as 4-amino-2,2,6,6-tetramethylpiperidine-1-oxyl; and a nanoparticle such as colloidal gold or quantum dot, among which the enzyme or the fluorescent substance is preferable. The method for labeling the substance having an affinity for amyloid β (1-40), the substance having an affinity for amyloid β (1-42), or/and the substance having an affinity for an N-terminal region of amyloid β with the labeling substance is not particularly limited, and may be carried out according to a labeling method known per se. The measurement of these labeling substances may be carried out according to a measuring method known per se depending on the labeling substance.

The substance having an affinity for amyloid β (1-40), the substance having an affinity for amyloid β (1-42), or/and the substance having an affinity for an N-terminal region of amyloid β may be immobilized on a solid phase, and it is preferable that the substance having an affinity for an N-terminal region of amyloid β is immobilized on the solid phase.

Examples of the solid phase include synthetic polymer compounds such as latex, polystyrene, polypropylene, polyacrylic acid, polymethacrylic acid, polyacrylamide, polyglycidyl methacrylate, polyvinyl chloride, polyethylene, polychlorocarbonate, silicone resin, and silicone rubber, and inorganic substances such as porous glass, ground glass, ceramics, alumina, silica gel, activated charcoal, and metal oxide. In addition, two or more of these solid phase substances may be used in combination.

The shape of the solid phase is not particularly limited, and examples thereof include a microtiter plate (ELISA plate), a bead, a tube (microtube), a particle, a dedicated tray in which a large number of tubes are integrally molded, a disk-shaped piece, and a test tube.

The method for immobilizing the substance having an affinity for amyloid β (1-40), the substance having an affinity for amyloid β (1-42), or/and the substance having an affinity for an N-terminal region of amyloid β on the solid phase is not particularly limited as long as it is a method commonly used in this field, and may be carried out according to a conventional method.

Specific examples of the method for measuring the amount of amyloid β (1-40) or/and the amount of amyloid β (1-42) include immunoassays and mass spectrometries known per se, including enzyme-linked immunosorbent assay (ELISA), enzyme immunoassay (EIA), radioimmunoassay (RIA), fluorescence enzyme immunoassay (FEIA), fluorescence immunoassay (FIA), chemiluminescence enzyme immunoassay (CLEIA), chemiluminescence immunoassay (CLIA), electrochemiluminescence immunoassay (ECLIA), immunochromatography assay (ICA), luminescent oxygen channeling immunoassay (LOCI), capillary electrophoresis such as liquid-phase binding assay-electrokinetic analyte transport assay (LBA-EATA) and lectin electrophoresis, Western blotting, nephelometric immunoassay (NIA) such as latex nephelometric immunoassay, turbidimetric immunoassay (TIA) such as latex turbidimetric immunoassay, immunoagglutination assay such as particle counting immunoassay (PCIA), and surface plasmon resonance (SPR). Among these methods, the enzyme-linked immunosorbent assay (ELISA), the chemiluminescence enzyme immunoassay (CLEIA), the chemiluminescence immunoassay (CLIA), the electrochemiluminescence immunoassay (ECLIA), or the capillary electrophoresis is preferable; the enzyme-linked immunosorbent assay (ELISA), the chemiluminescence enzyme immunoassay (CLEIA), or LBA-EATA is more preferable; and the enzyme-linked immunosorbent assay (ELISA) is particularly preferable.

The principle for measuring the amount of amyloid β (1-40) or/and the amount of amyloid β (1-42) is not particularly limited, and examples thereof include a sandwich method and a competitive method, among which the sandwich method is preferable. In addition, a homogeneous method, a heterogeneous method, or the like may be used as the measurement principle, and the heterogeneous method is preferable.

Examples of the method according to immunoassay include a method of carrying out the immunoassay using the substance having an affinity for amyloid β (1-40) other than the antibody that specifically binds to amyloid β (1-40), the substance having an affinity for amyloid β (1-42) other than the antibody that specifically binds to amyloid β (1-42), or/and the substance having an affinity for an N-terminal region of amyloid β other than the antibody that specifically binds to an N-terminal region of amyloid β, and preferred methods thereof are also the same.

Specific examples of the method for measuring the amount of amyloid β (1-40) or/and the amount of amyloid β (1-42) include a method containing (1) a step of forming a complex of amyloid β (1-40) or/and amyloid β (1-42) in a biological specimen and the substance having an affinity for amyloid β (1-40) or the substance having an affinity for amyloid β (1-42) (or a step of forming a complex of amyloid β (1-40) or amyloid β (1-42) in a biological specimen, the substance having an affinity for amyloid β (1-40) or the substance having an affinity for amyloid β (1-42), and the substance having an affinity for an N-terminal region of amyloid (3), and (2) a step of measuring an amount of the complex formed in (1) as a preferred method.

Specific examples of the method for measuring the amount of amyloid β (1-40) or the amount of amyloid β (1-42) in the AD diagnosis assisting method (III) include a method containing (1) a step of bringing amyloid β (1-40) or amyloid β (1-42) in a biological specimen, a substance having an affinity for amyloid β (1-40) or a substance having an affinity for amyloid β (1-42), and a substance having an affinity for an N-terminal region of amyloid β into contact with each other to form a complex containing the substance having an affinity for an N-terminal region of amyloid β, the amyloid β (1-40) or amyloid β (1-42) in a biological specimen, and the substance having an affinity for amyloid β (1-40) or the substance having an affinity for amyloid β (1-42) (complex forming step), and (2) a step of measuring an amount of the complex (complex amount measuring step) as a preferred method.

The complex forming step in measuring the amount of amyloid β (1-40) or the amount of amyloid β (1-42) in the AD diagnosis assisting method (III) preferably contains a first procedure of bringing a biological specimen into contact with a substance having an affinity for an N-terminal region of amyloid β to form a first complex composed of amyloid β (1-40) or amyloid β (1-42) and the substance having an affinity for an N-terminal region of amyloid β, and a second procedure of bringing the first complex into contact with a substance having an affinity for amyloid β (1-40) or a substance having an affinity for amyloid β (1-42) to form a second complex composed of the first complex and the substance having an affinity for amyloid β (1-40) or the substance having an affinity for amyloid β (1-42).

The complex forming step in measuring the amount of amyloid β (1-40) or the amount of amyloid β (1-42) in the AD diagnosis assisting method (III) is specifically carried out, for example, in such a manner that an antibody that specifically binds to an N-terminal region of amyloid β immobilized on a solid phase is brought into contact with a biological specimen to form a first complex of the antibody that specifically binds to an N-terminal region of amyloid β and amyloid β (1-40) or amyloid β (1-42) in the biological specimen, and the first complex is brought into contact with an antibody that specifically binds to amyloid β (1-40) or an antibody that specifically binds to amyloid β (1-42) to form a second complex of the first complex and the antibody that specifically binds to amyloid β (1-40) or the antibody that specifically binds to amyloid β (1-42).

After forming the complex, it is preferable to carry out a washing operation (B/F separation) at least before the complex amount measuring step.

Specifically, for example, the washing operation may be carried out after forming the first complex or/and after forming the second complex in the above method. It is preferable to carry out the washing operation (B/F separation) after forming the first complex, and further carry out the washing operation (B/F separation) after forming the second complex.

The complex amount measuring step in measuring the amount of amyloid β (1-40) or the amount of amyloid β (1-42) in the AD diagnosis assisting method (III) is a step of measuring an amount of the complex containing amyloid β (1-40) or amyloid β (1-42) in a biological specimen, a substance having an affinity for amyloid β (1-40) or a substance having an affinity for amyloid β (1-42), and a substance having an affinity for an N-terminal region of amyloid β obtained in the complex forming step. Any method may be used as long as the amount of the complex can be measured.

More specifically, the complex amount measuring step may detect a labeling substance in the complex containing an antibody having an affinity for an N-terminal region of amyloid β immobilized on a solid phase plate, amyloid β (1-40) or amyloid β (1-42) in a biological specimen, an antibody that specifically binds to amyloid β (1-40) or an antibody that specifically binds to amyloid β (1-42), and a labeling substance (preferably an enzyme or a fluorescent substance) obtained in the complex forming step using, for example, (1) an antibody that specifically binds to amyloid β (1-40) or an antibody that specifically binds to amyloid β (1-42), labeled with a labeling substance, or (2) a labeled secondary antibody labeled with a labeling substance, which specifically binds to "an antibody that specifically binds to amyloid β (1-40) or an antibody that specifically binds to amyloid β (1-42)", or (3) "an antibody that specifically binds to amyloid β (1-40) or an antibody that specifically binds to amyloid β (1-42)" to which one of avidin and biotin is bound and a labeling substance to which the other one of avidin and biotin is bound.

In the complex amount measuring step, it is preferable to carry out a washing operation (B/F separation) before detecting a labeling substance.

The amount of biological specimen and the amount (concentration) of protein in the biological specimen, the amount (concentration) of amyloid β (1-40) or/and the amount (concentration) of amyloid β (1-42) in the biological specimen, the amount (concentration) of a substance having an affinity for amyloid β (1-40), a substance having an affinity for amyloid β (1-42), and a substance having an affinity for an N-terminal region of amyloid β to react with these amyloid β types, the labeling substance and the labeling method, and the like may be appropriately set according to the type of the biological specimen, the required measurement sensitivity, the measuring method and measuring device to be used, and the like.

In addition, the amount of amyloid β (1-40) or/and the amount of amyloid β (1-42) in the biological specimen may be calculated by creating a calibration curve using a reference standard. Examples of the reference standard include amyloid β (1-40) and amyloid β (1-42).

The determination of Alzheimer's disease in the AD diagnosis assisting method (III) contains determining Alzheimer's disease using the AD marker (III) as an indicator.

Examples of the determination of Alzheimer's disease include determination of whether or not a subject is likely to have Alzheimer's disease, determination of whether or not a subject may have Alzheimer's disease, determination of whether or not a subject is at high risk of developing Alzheimer's disease, and determination of whether or not a subject is at risk of developing Alzheimer's disease, among which the determination of whether or not a subject is likely to have Alzheimer's disease or the determination of whether or not a subject may have Alzheimer's disease is preferable.

The determination of Alzheimer's disease in the AD diagnosis assisting method (III) is made, for example, by comparing the AD marker (III) calculated by the above method with a predetermined reference value (cutoff value).

Specifically, in a case where the AD marker (III) is equal to or less than a predetermined reference value, it can be determined that a subject is likely to have Alzheimer's disease; or a subject may have Alzheimer's disease; or a subject is at high risk of developing Alzheimer's disease; or a subject is at risk of developing Alzheimer's disease.

In addition, in a case where the AD marker (III) is larger than a predetermined reference value, it can be determined that a subject is unlikely to have Alzheimer's disease; or a subject may not have Alzheimer's disease; or a subject is at low risk of developing Alzheimer's disease; or a subject is not at risk of developing Alzheimer's disease.

The method for determining the predetermined reference value in the AD diagnosis assisting method (III) is not particularly limited. For example, the predetermined reference value can be determined in such a manner that an AD marker (III) is calculated based on a biological specimen obtained from a patient suffering from Alzheimer's disease and a healthy subject, and a statistical analysis such as receiver operating characteristic analysis (ROC analysis) is carried out using the obtained AD marker (III). In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

**Reagent kit (III) for assisting diagnosis of Alzheimer's disease** (not claimed, for reference only)

The reagent kit for assisting the diagnosis of Alzheimer's disease in the first invention-3 (hereinafter, often referred to simply as "reagent kit (III) for assisting the diagnosis of AD") contains a substance having an affinity for tetraspanin, a substance having an affinity for phosphatidylserine, a substance having an affinity for amyloid β (1-40), and a substance having an affinity for amyloid β (1-42), and if necessary, further a substance having an affinity for an N-terminal region of amyloid β.

That is, the reagent kit (III) for assisting the diagnosis of AD is a reagent kit containing the reagent kit (II) for assisting the diagnosis of AD, a substance having an affinity for amyloid β (1-40), and a substance having an affinity for amyloid β (1-42), and if necessary, further a substance having an affinity for an N-terminal region of amyloid β.

All the descriptions regarding the reagent kit (II) for assisting the diagnosis of AD in the reagent kit (III) for assisting the diagnosis of AD are the same as those in the reagent kit (II) for assisting the diagnosis of AD in the first invention-2, and preferred ones and specific examples thereof are also the same.

The substance having an affinity for amyloid β (1-40), the substance having an affinity for amyloid β (1-42), and the substance having an affinity for an N-terminal region of amyloid β in the reagent kit (III) for assisting the diagnosis of AD are the same as those of the AD diagnosis assisting method (III), and preferred ones thereof are also the same.

The substance having an affinity for amyloid β (1-40), the substance having an affinity for amyloid β (1-42), and the substance having an affinity for an N-terminal region of amyloid β each may be in a solution state, a frozen state, a dried state, or a freeze-dried state. In addition, the substance having an affinity for amyloid β (1-40), the substance having an affinity for amyloid β (1-42), and the substance having an affinity for an N-terminal region of amyloid β may be contained in the kit as one reagent or may be contained in the kit as separate reagents.

The combination of the substance having an affinity for amyloid β (1-40) or the substance having an affinity for amyloid β (1-42) and the substance having an affinity for an N-terminal region of amyloid β in the reagent kit (III) for assisting the diagnosis of AD includes the same combinations as the combinations in the AD diagnosis assisting method (III), and preferred combinations thereof are also the same.

The substance having an affinity for amyloid β (1-40), the substance having an affinity for amyloid β (1-42), or/and the substance having an affinity for an N-terminal region of amyloid β in the reagent kit (III) for assisting the diagnosis of AD may be immobilized on a solid phase and may be labeled with a labeling substance. The solid phase, the method for immobilizing the substance on the solid phase, the labeling substance, and the labeling method are the same as those in the AD diagnosis assisting method (III), and preferred ones thereof are also the same.

The concentration (amount) of the substance having an affinity for amyloid β (1-40) or the substance having an affinity for amyloid β (1-42) in the reagent kit (III) for assisting the diagnosis of AD may be appropriately set within the range commonly used in this field depending on the measuring method. For example, the substance having an affinity for amyloid β (1-40) or the substance having an affinity for amyloid β (1-42) is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, for example, the substance having an affinity for an N-terminal region of amyloid β is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, the substance having an affinity for tetraspanin and the substance having an affinity for phosphatidylserine in the reagent kit (III) for assisting the diagnosis of AD may coexist with a reagent commonly used in this field. The reagent is the same as that of the reagent kit for assisting the diagnosis of AD.

Further, the reagent kit (III) for assisting the diagnosis of AD may contain a reagent needed to measure an amount of amyloid β (1-40) or/and an amount of amyloid β (1-42), in addition to the substance having an affinity for amyloid β (1-40), the substance having an affinity for amyloid β (1-42), or/and the substance having an affinity for an N-terminal region of amyloid β. Such a reagent is the same as that of the reagent kit for assisting the diagnosis of AD.

The reagent kit (III) for assisting the diagnosis of AD may contain a reference standard used for creating a calibration curve for amyloid β (1-40) or/and amyloid β (1-42). Examples of the reference standard include amyloid β (1-40) and amyloid β (1-42). The reference standard may be in a solution state, a frozen state, a dried state, or a freeze-dried state. In addition, reagents commonly used in this field, for example, a buffer, a reaction promoter, a sugar, a protein, a salt, a stabilizer such as a surfactant, and a preservative, may coexist with the reference standard. The concentration and pH of these reagents may be appropriately selected from the ranges commonly used in this field.

The reagent kit (III) for assisting the diagnosis of AD may contain a package insert or an instruction manual. Examples of the package insert or instruction manual include a package insert or instruction manual that describes measuring an amount of amyloid β (1-40) or/and an amount of amyloid β (1-42) in a biological specimen derived from a subject, calculating an AD marker (III), and determining that the subject has Alzheimer's disease using the AD marker (III) as an indicator. These package insert and instruction manual may be described separately in a plurality of cases, or may be described collectively in one.

Specifically, the reagent kit (III) for assisting the diagnosis of AD may be, for example, a kit containing one of "a substance having an affinity for amyloid β (1-40) or a substance having an affinity for amyloid β (1-42)" and a substance having an affinity for an N-terminal region of amyloid β immobilized on a solid phase and the other one of "a substance having an affinity for amyloid β (1-40) or a substance having an affinity for amyloid β (1-42)" and a substance having an affinity for an N-terminal region of amyloid β bound to a labeling substance or the other one affinity substance and a component for indirectly binding the other one affinity substance to a labeling substance.

The reagent kit (III) for assisting the diagnosis of AD is preferably, for example, a kit containing one of "a substance having an affinity for amyloid β (1-40) or a substance having an affinity for amyloid β (1-42)" and a substance having an affinity for an N-terminal region of amyloid β immobilized on a solid phase and any one selected from the following (1) to (3).

(1) the other one of "a substance having an affinity for amyloid β (1-40) or a substance having an affinity for amyloid β (1-42)" and a substance having an affinity for an N-terminal region of amyloid β bound to a labeling substance,
(2) the other one of "a substance having an affinity for amyloid β (1-40) or a substance having an affinity for amyloid β (1-42)" and a substance having an affinity for an N-terminal region of amyloid β, and a secondary affinity substance that specifically binds to the affinity substance bound to a labeling substance, and
(3) the other one of "a substance having an affinity for amyloid β (1-40) or a substance having an affinity for amyloid β (1-42)" and a substance having an affinity for an N-terminal region of amyloid β to which one of avidin and biotin is bound, and a labeling substance to which the other one of avidin and biotin is bound.

**Device** (III) **for assisting diagnosis of Alzheimer's disease** (not claimed, for reference only)

The device for assisting the diagnosis of Alzheimer's disease in the first invention-3 (hereinafter, often referred to simply as "device (III) for assisting the diagnosis of AD") has a measurement unit that measures an amount of extracellular vesicles having phosphatidylserine and tetraspanin and an amount of extracellular vesicles having tetraspanin, in a biological specimen derived from a subject; a calculation unit that multiplies a ratio of the amount of extracellular vesicles having phosphatidylserine and tetraspanin to the amount of extracellular vesicles having tetraspanin (AD marker (II)) by a ratio of an amount of amyloid β (1-42) to an amount of amyloid β (1-40) (Aβ (1-42)/Aβ (1-40)) to calculate an AD marker (III); and a determination unit that determines that the subject has Alzheimer's disease or mild cognitive impairment using the AD marker (III) as an indicator.

The device (III) for assisting the diagnosis of AD may have, if necessary, a measurement unit that measures an amount of amyloid β (1-40) or/and an amount of amyloid β (1-42); an input unit that inputs the amount of amyloid β (1-40) or/and the amount of amyloid β (1-42) or a ratio of the amount of amyloid β (1-42) to the amount of amyloid β (1-40) (Aβ (1-42)/Aβ (1-40)) from the outside; and a calculation unit that calculates the ratio of the amount of amyloid β (1-42) to the amount of amyloid β (1-40) (Aβ (1-42)/Aβ (1-40)).

The measurement unit, the calculation unit, the input unit, the determination unit, and the like constituting the device (III) for assisting the diagnosis of AD may be arranged in the same device or may be separate bodies.

The measurement unit in the device (III) for assisting the diagnosis of AD is a site for measuring the amount of extracellular vesicles having phosphatidylserine and tetraspanin and the amount of extracellular vesicles having tetraspanin in the biological specimen introduced into the device. If necessary, the measurement unit is also a site for further measuring the amount of amyloid β (1-40) or/and the amount of amyloid β (1-42) in the biological specimen introduced into the device.

The size and configuration of the measurement unit are not particularly limited.

Examples of the measurement unit include an imaging apparatus for imaging using a microplate reader or CCD used for ELISA, an imaging apparatus used for Western blotting or a method using a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The subject, the biological specimen, and the extracellular vesicle in the device (III) for assisting the diagnosis of AD are the same as those described above, and preferred ones thereof are also the same.

The extracellular vesicles having phosphatidylserine and tetraspanin, the amount, the target for measuring the amount of extracellular vesicles having phosphatidylserine and tetraspanin, the measurement of the amount of extracellular vesicles having phosphatidylserine and tetraspanin and the substances used for the measurement, the extracellular vesicles having tetraspanin, the target for measuring the amount of extracellular vesicles having tetraspanin, and the measurement of the amount of extracellular vesicles having tetraspanin and the substances used for the measurement in the device (III) for assisting the diagnosis of AD are the same as those of the AD diagnosis assisting method (II), and preferred ones and specific examples thereof are also the same.

The calculation unit in the device (III) for assisting the diagnosis of AD is a site for calculating the AD marker (III) obtained by multiplying the ratio of the amount of extracellular vesicles having phosphatidylserine and tetraspanin to the amount of extracellular vesicles having tetraspanin (AD marker (II)) by the ratio of the amount of amyloid $\beta$ (1-42) to the amount of amyloid $\beta$ (1-40) (A$\beta$ (1-42)/A$\beta$ (1-40)), based on the amount of extracellular vesicles having tetraspanin and the amount of extracellular vesicles having phosphatidylserine and tetraspanin obtained by the measurement unit, and the amount of amyloid $\beta$ (1-40) or/and the amount of amyloid $\beta$ (1-42) obtained by the measurement unit, or the amount of amyloid $\beta$ (1-40) or/and the amount of amyloid $\beta$ (1-42), or the ratio of the amount of amyloid $\beta$ (1-42) to the amount of amyloid $\beta$ (1-40) (A$\beta$ (1-42)/A$\beta$ (1-40)) input from the input unit from the outside.

The size and configuration of the calculation unit are not particularly limited.

The calculation unit in the device (III) for assisting the diagnosis of AD may calculate the AD marker (III) after converting an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index) having a correlation with the measured or input mass or concentration into mass, concentration, or the like.

It should be noted that the actually measured value, the mass, concentration, or the like converted by the calculation unit, the AD marker (II), the ratio of the amount of amyloid $\beta$ (1-42) to the amount of amyloid $\beta$ (1-40) (A$\beta$ (1-42)/A$\beta$ (1-40)), and the AD marker (III) may be stored in a storage device or the like provided in a device such as a memory device or a hard disk.

The determination unit in the device (III) for assisting the diagnosis of AD is a site for determining Alzheimer's disease using the AD marker (III) calculated by the calculation unit as an indicator.

The determination of Alzheimer's disease and the predetermined reference value used for the determination in the device (III) for assisting the diagnosis of AD, and the method for determining the predetermined reference value are the same as those in the AD diagnosis assisting method (III) and preferred ones and specific examples thereof are also the same.

The predetermined reference value in the device (III) for assisting the diagnosis of AD may be stored in advance in the device (III) for assisting the diagnosis of AD, or may be input from an input site of the device (III) for assisting the diagnosis of AD at the time of determination.

The device (III) for assisting the diagnosis of AD may contain an output unit. The output unit carries out processing such as displaying or outputting the results of the determination to a display device such as a display or a printing device such as a printer.

## 2. Assistance in diagnosis of Alzheimer's disease or mild cognitive impairment

The second invention in the present invention relates to a method for assisting the diagnosis of Alzheimer's disease or mild cognitive impairment, *in vitro* uses of a biomarker, and *in vitro* uses of a reagent kit.

The second invention of the present invention contains a case of determining that a subject has Alzheimer's disease or mild cognitive impairment using biomarkers containing extracellular vesicles having phosphatidylserine and CD9 and extracellular vesicles having CD9 in combination, and using the ratio of the amount of extracellular vesicles having phosphatidylserine and CD9 to the amount of extracellular vesicles having CD9 as an indicator (hereinafter, often referred to simply as "second invention-1 "); and a case of determining that a subject has Alzheimer's disease or mild cognitive impairment using biomarkers containing extracellular vesicles having phosphatidylserine and CD9, extracellular vesicles having CD9, amyloid $\beta$ (1-40), and amyloid $\beta$ (1-42) in combination, and using the value obtained by multiplying the ratio of the amount of extracellular vesicles having phosphatidylserine and CD9 to the amount of extracellular vesicles having CD9 by the ratio of the amount of amyloid $\beta$ (1-42) to the amount of amyloid $\beta$ (1-40) (A$\beta$ (1-42)/A$\beta$ (1-40)) as an indicator (hereinafter, often referred to simply as "second invention-2").

### 2-1. **Second invention-1**

**Biomarker set for assisting diagnosis of Alzheimer's disease or mild cognitive impairment** (the *in vitro* use of a biomarker set is claimed)

The biomarker set for assisting the diagnosis of Alzheimer's disease or mild cognitive impairment in the second invention-1 (hereinafter, often referred to simply as "AD/MCI marker set") is a combination of biomarkers containing extracellular vesicles having phosphatidylserine and CD9 and extracellular vesicles having CD9.

According to the AD/MCI marker set, for example, a ratio of the amount of extracellular vesicles having phosphatidylserine and CD9 to the amount of extracellular vesicles having CD9 (hereinafter, often referred to simply as "AD/MCI marker") can be obtained, and the ratio can be used as an indicator for determining that a subject has Alzheimer's disease or mild cognitive impairment.

The extracellular vesicle in the AD/MCI marker set is the same as the extracellular vesicle described above, and preferred ones thereof are also the same.

The extracellular vesicle having phosphatidylserine and CD9 in the AD/MCI marker set has at least phosphatidylserine and CD9 on the membrane surface.

In addition, the extracellular vesicle having CD9 in the AD/MCI marker set has at least CD9 on the membrane surface.

**Method for assisting diagnosis of Alzheimer's disease or mild cognitive impairment**

The method for assisting the diagnosis of Alzheimer's disease or mild cognitive impairment in the second invention-1 (hereinafter, often referred to simply as "AD/MCI diagnosis assisting method") contains measuring an amount of extracellular vesicles having phosphatidylserine and CD9 in a biological specimen and an amount of extracellular vesicles having CD9 in the biological specimen; calculating a ratio of the amount of extracellular vesicles having phosphatidylserine and CD9 to the amount of extracellular vesicles having CD9; and determining that a subject has Alzheimer's disease or mild cognitive impairment using the ratio of the amount of extracellular vesicles having phosphatidylserine and CD9 to the amount of extracellular vesicles having CD9 as an indicator.

The biological specimen, the subject, and the extracellular vesicle in the AD/MCI diagnosis assisting method are the same as those described above, and preferred ones thereof are also the same.

The extracellular vesicle having phosphatidylserine and CD9 and the extracellular vesicle having CD9 in the AD/MCI diagnosis assisting method are the same as those described above in the AD/MCI marker set.

The "amount" in the AD/MCI diagnosis assisting method is the same as that described above in the AD diagnosis assisting method, and specific examples thereof are also the same.

The measurement of the amount of extracellular vesicles having phosphatidylserine and CD9 in the AD/MCI diagnosis assisting method is not particularly limited as long as it is a method commonly carried out in this field. The measurement may be carried out in the same manner as in the measurement of the amount of extracellular vesicles having phosphatidylserine and tetraspanin in the AD diagnosis assisting method, for example, using a substance having an affinity for CD9 as the substance having an affinity for tetraspanin.

The measurement of the amount of extracellular vesicles having CD9 in the AD/MCI diagnosis assisting method is not particularly limited as long as it is a method commonly carried out in this field. The measurement may be carried out in the same manner as in the measurement of the amount of extracellular vesicles having tetraspanin in the AD diagnosis assisting method (II), for example, using a substance having an affinity for CD9 as the substance having an affinity for tetraspanin.

The substance having an affinity for phosphatidylserine in the AD/MCI diagnosis assisting method is the same as that of the AD diagnosis assisting method, and preferred ones thereof are also the same.

The substance having an affinity for CD9 in the AD/MCI diagnosis assisting method may be a substance that specifically binds to CD9, examples of which include a protein that specifically binds to CD9, such as an antibody that specifically binds to CD9 or a lectin that specifically binds to a sugar chain that binds to CD9, and a nucleic acid that specifically binds to CD9, among which the protein that specifically binds to CD9 is preferable, and the antibody that specifically binds to CD9 is more preferable. The antibody that specifically binds to CD9 may be a monoclonal antibody or a polyclonal antibody. Only one type of the substance having an affinity for CD9 may be used, or two or more types of the substances having an affinity for CD9 may be used. It is preferable to use only one type of the substance having an affinity for CD9.

Regarding the substance having an affinity for phosphatidylserine and the substance having an affinity for CD9 in the AD/MCI diagnosis assisting method, the labeling substance and the labeling method, the secondary affinity substance, the binding of avidin and biotin, the solid phase, and the immobilization method may be carried out in the same manner as in the AD diagnosis assisting method (II) by using a substance having an affinity for CD9 as the substance having an affinity for tetraspanin.

The complex forming step, the complex amount measuring step, and the washing operation (B/F separation) in the AD/MCI diagnosis assisting method may be carried out in the same manner as in the AD diagnosis assisting method (II) by using a substance having an affinity for tetraspanin as the substance having an affinity for CD9, and specific examples and preferred examples thereof are also the same as in the AD diagnosis assisting method (II).

Specifically, the measurement of the amount of extracellular vesicles having phosphatidylserine and CD9 in the AD/MCI diagnosis assisting method may be carried out, for example, in such a manner that an antibody or Tim protein (preferably Tim or Tim4 and more preferably Tim4) that specifically binds to phosphatidylserine immobilized on a solid phase and a biological specimen are brought into contact with each other to form a first complex of the antibody or Tim protein that specifically binds to phosphatidylserine and an extracellular vesicle having phosphatidylserine and CD9 in the biological specimen, the first complex and an antibody that specifically binds to CD9 are brought into contact with each other to form a second complex of the first complex, the antibody that specifically binds to CD9, and a labeling substance (preferably an enzyme or a fluorescent substance) of the second complex is detected.

It is preferable that the substance having an affinity for CD9 used for forming the first complex and the substance having an affinity for CD9 used for forming the second complex are the same.

More specifically, the measurement of the amount of extracellular vesicles having phosphatidylserine and CD9 in an AD/MCI diagnosis assisting method may be carried out, for example, in such a manner that an antibody or Tim protein (preferably Tim or Tim4 and more preferably Tim4) that specifically binds to phosphatidylserine immobilized on a solid phase plate and a biological specimen are brought into contact with each other to form a first complex of the antibody or Tim protein that specifically binds to phosphatidylserine and an extracellular vesicle having phosphatidylserine and CD9 in the biological specimen, followed by B/F separation if necessary, (1) the first complex is brought into contact with an antibody that specifically binds to CD9 labeled with a labeling substance to form a second complex of the first complex and the antibody that specifically binds to CD9 labeled with a labeling substance, (2) the first complex is brought into contact with an antibody that specifically binds to CD9 to form a second complex of the first complex and the antibody that specifically binds to CD9, followed by B/F separation if necessary, and the second complex is brought into contact with a labeled secondary antibody labeled with a labeling substance, which specifically binds to "the antibody that specifically binds to CD9", to form a third complex of the second complex and the labeled secondary antibody, or (3) the first complex is brought into contact with an antibody that specifically binds to CD9 to which one of avidin and biotin is bound to form a second complex of the first complex and the antibody that specifically binds to CD9 to which one of avidin and biotin is bound, followed by B/F separation if necessary, the first complex is brought into contact with an antibody that specifically binds to CD9 to which one of avidin and biotin is bound to form a second complex of the first complex and the antibody that specifically binds to CD9 to which one of avidin and biotin is bound, a third complex of the second complex and a labeling substance (preferably an enzyme or a fluorescent substance) to which the other one of avidin and biotin is bound is formed, followed by B/F separation, and then the labeling substance of the obtained second complex or third complex is detected.

Specifically, the measurement of the amount of extracellular vesicles having CD9 in the AD/MCI diagnosis assisting method may be carried out, for example, in such a manner that an antibody that specifically binds to CD9 immobilized on a solid phase and a biological specimen are brought into contact with each other to form a first complex of the antibody that specifically binds to CD9 and an extracellular vesicle having CD9 in the biological specimen, the first complex and an antibody that specifically binds to CD9 are brought into contact with each other to form a second complex of the first complex, the antibody that specifically binds to CD9, and a labeling substance (preferably an enzyme or a fluorescent substance) of the second complex is detected.

More specifically, the measurement of the amount of extracellular vesicles having CD9 in an AD/MCI diagnosis assisting method may be carried out, for example, in such a manner that an antibody that specifically binds to CD9 immobilized on a solid phase plate and a biological specimen are brought into contact with each other to form a first complex of the antibody that specifically binds to CD9 and an extracellular vesicle having CD9 in the biological specimen, followed by B/F separation if necessary, (1) the first complex is brought into contact with an antibody that specifically binds to CD9 labeled with a labeling substance to form a second complex of the first complex and the antibody that specifically binds to CD9 labeled with a labeling substance, (2) the first complex is brought into contact with an antibody that specifically binds to CD9 to form a second complex of the first complex and the antibody that specifically binds to CD9, followed by B/F separation if necessary, and the second complex is brought into contact with a labeled secondary antibody labeled with a labeling substance, which specifically binds to "the antibody that specifically binds to CD9", to form a third complex of the second complex and the labeled secondary antibody, or (3) the first complex is brought into contact with an antibody that specifically binds to CD9 to which one of avidin and biotin is bound to form a second complex of the first complex and the antibody that specifically binds to CD9 to which one of avidin and biotin is bound, followed by B/F separation if necessary, the first complex is brought into contact with an antibody that specifically binds to CD9 to which one of avidin and biotin is bound to form a second complex of the first complex and the antibody that specifically binds to CD9 to which one of avidin and biotin is bound, a third complex of the second complex and a labeling substance (preferably an enzyme or a fluorescent substance) to which the other one of avidin and biotin is bound is formed, followed by B/F separation, and then the labeling substance of the obtained second complex or third complex is detected.

The amount of the biological specimen and the amount (concentration) of protein in the biological specimen, the amount (concentration) and number of particles of extracellular vesicles having phosphatidylserine and CD9 in the biological specimen, and the amount (concentration) of the substance having an affinity for CD9 and the substance having an affinity for phosphatidylserine to react with these extracellular vesicles, the labeling substance and the labeling method, and the like may be appropriately set according to the type of biological specimen, the required measurement sensitivity, the measuring method and measuring device to be used, and the like.

The ratio of the amount of extracellular vesicles having phosphatidylserine and CD9 to the amount of extracellular vesicles having CD9 may be calculated by dividing the amount (A) of extracellular vesicles having phosphatidylserine and CD9 by the amount (B) of extracellular vesicles having CD9 ((A)/(B)).

The determination of Alzheimer's disease or mild cognitive impairment in the AD/MCI diagnosis assisting method contains determining Alzheimer's disease or mild cognitive impairment using the ratio ((A)/(B)) of the amount (B) of extracellular vesicles having phosphatidylserine and CD9 to the amount (A) of extracellular vesicles having CD9 as an indicator.

Examples of the determination of Alzheimer's disease or mild cognitive impairment include determination of whether or not a subject is likely to have Alzheimer's disease or mild cognitive impairment, determination of whether or not a subject may have Alzheimer's disease or mild cognitive impairment, determination of whether or not a subject is at high risk of developing Alzheimer's disease or mild cognitive impairment, and determination of whether or not a subject is at risk of developing Alzheimer's disease or mild cognitive impairment, among which the determination of whether or not a subject is likely to have Alzheimer's disease or mild cognitive impairment or the determination of whether or not a subject may have Alzheimer's disease or mild cognitive impairment is preferable.

The determination of Alzheimer's disease or mild cognitive impairment in the AD/MCI diagnosis assisting method is made, for example, by comparing the ratio ((A)/(B)) of the amount of extracellular vesicles having phosphatidylserine and CD9 to the amount of extracellular vesicles having CD9 with a predetermined reference value (cutoff value).

For example, in a case where a reference value predetermined to distinguish between a healthy subject and Alzheimer's disease or mild cognitive impairment (cutoff value, hereinafter often referred to simply as "healthy/AD-MCI reference value") is used, it is possible to distinguish and determine between a healthy subject and a patient with Alzheimer's disease or a patient with mild cognitive impairment.

That is, for example, using a biological specimen derived from a subject diagnosed with Alzheimer's disease based on diagnostic criteria or a biological specimen derived from a subject diagnosed with a risk of developing Alzheimer's disease based on diagnostic criteria, it is possible to distinguish and determine between a healthy subject and a patient with Alzheimer's disease by comparing the ratio ((A)/(B)) of the amount of extracellular vesicles having phosphatidylserine and CD9 to the amount of extracellular vesicles having CD9 in the biological specimen derived from the subject, which is obtained by measuring the amount of extracellular vesicles having CD9 in the AD/MCI diagnosis assisting method, and measuring the amount of extracellular vesicles having phosphatidylserine and CD9 in the AD/MCI diagnosis assisting method, with a predetermined healthy/AD-MCI reference value.

Specifically, in a case where the ratio ((A)/(B)) of the amount of extracellular vesicles having phosphatidylserine and CD9 to the amount of extracellular vesicles having CD9 is equal to or less than the predetermined healthy/AD-MCI reference value, it can be determined that a subject is likely to have Alzheimer's disease; or a subject may have Alzheimer's disease; or a subject is at high risk of developing Alzheimer's disease; or a subject is at risk of developing Alzheimer's disease.

On the other hand, in a case where the ratio ((A)/(B)) of the amount of extracellular vesicles having phosphatidylserine and CD9 to the amount of extracellular vesicles having CD9 is larger than the predetermined healthy/AD-MCI reference value, it can be determined that a subject is unlikely to have Alzheimer's disease; or a subject may not have Alzheimer's disease; or a subject is at low risk of developing Alzheimer's disease; or a subject is not at risk of developing Alzheimer's disease.

In addition, for example, using a biological specimen derived from a subject diagnosed with mild cognitive impairment based on diagnostic criteria or a biological specimen derived from a subject diagnosed with a risk of developing mild cognitive impairment based on diagnostic criteria, it is possible to distinguish and determine between a healthy subject and a patient with mild cognitive impairment by comparing the ratio ((A)/(B)) of the amount of extracellular vesicles having phosphatidylserine and CD9 to the amount of extracellular vesicles having CD9 in the biological specimen derived from the subject, which is obtained by measuring the amount of extracellular vesicles having CD9 in the AD/MCI diagnosis assisting method, and measuring the amount of extracellular vesicles having phosphatidylserine and CD9 in the AD/MCI diagnosis assisting method, with a predetermined healthy/AD-MCI reference value.

Specifically, in a case where the ratio ((A)/(B)) of the amount of extracellular vesicles having phosphatidylserine and CD9 to the amount of extracellular vesicles having CD9 is equal to or less than the predetermined healthy/AD-MCI reference value, it can be determined that a subject is likely to have mild cognitive impairment; or a subject may have mild cognitive impairment; or a subject is at high risk of developing mild cognitive impairment; or a subject is at risk of developing mild cognitive impairment.

On the other hand, in a case where the ratio ((A)/(B)) of the amount of extracellular vesicles having phosphatidylserine and CD9 to the amount of extracellular vesicles having CD9 is larger than the predetermined healthy/AD-MCI reference value, it can be determined that a subject is unlikely to have mild cognitive impairment; or a subject may not have mild cognitive impairment; or a subject is at low risk of developing mild cognitive impairment; or a subject is not at risk of developing mild cognitive impairment.

In addition, for example, using a biological specimen derived from a subject who has not been diagnosed with Alzheimer's disease or mild cognitive impairment, a biological specimen derived from a subject who has not been diagnosed with Alzheimer's disease or mild cognitive impairment based on diagnostic criteria, or a biological specimen derived from a subject who has not been diagnosed at risk of developing Alzheimer's disease or mild cognitive impairment based on diagnostic criteria, it is possible to distinguish and determine between a healthy subject and a patient with Alzheimer's disease or a patient with mild cognitive impairment by comparing the ratio ((A)/(B)) of the amount of extracellular vesicles having phosphatidylserine and CD9 to the amount of extracellular vesicles having CD9 in the biological specimen derived from the subject, which is obtained by measuring the amount of extracellular vesicles having CD9 in the AD/MCI diagnosis assisting method, and measuring the amount of extracellular vesicles having phosphatidylserine and CD9 in the AD/MCI diagnosis assisting method, with a predetermined healthy/AD-MCI reference value.

Specifically, in a case where the ratio ((A)/(B)) of the amount of extracellular vesicles having phosphatidylserine and CD9 to the amount of extracellular vesicles having CD9 is equal to or less than the predetermined healthy/AD-MCI reference value, it can be determined that a subject is likely to have Alzheimer's disease or mild cognitive impairment; or a subject may have Alzheimer's disease or mild cognitive impairment; or a subject is at high risk of developing Alzheimer's disease or mild cognitive impairment; or a subject is at risk of developing Alzheimer's disease or mild cognitive impairment.

On the other hand, in a case where the ratio ((A)/(B)) of the amount of extracellular vesicles having phosphatidylserine and CD9 to the amount of extracellular vesicles having CD9 is larger than the predetermined healthy/AD-MCI reference value, it can be determined that a subject is unlikely to have Alzheimer's disease or mild cognitive impairment; or a subject may not have Alzheimer's disease or mild cognitive impairment; or a subject is at low risk of developing Alzheimer's disease or mild cognitive impairment; or a subject is not at risk of developing Alzheimer's disease or mild cognitive impairment.

A plurality of reference values (cutoff values) may be used in determining Alzheimer's disease or mild cognitive impairment in the AD/MCI diagnosis assisting method.

By using a plurality of reference values (cutoff values) in the determination of Alzheimer's disease or mild cognitive impairment in the AD/MCI diagnosis assisting method, for example, by using a healthy/AD-MCI reference value and a reference value predetermined to distinguish between AD and MCI (AD/MCI reference value), it is possible to determine whether the subject is a healthy subject, a patient with Alzheimer's disease, or a patient with mild cognitive impairment, and further, the determination can be made for the subject with classification into three types: a healthy subject, a patient with Alzheimer's disease, and a patient with mild cognitive impairment.

Examples of the determination of Alzheimer's disease or mild cognitive impairment include determining whether a subject is likely to have Alzheimer's disease, determining whether a subject may have Alzheimer's disease, determining whether a subject is likely to have mild cognitive impairment, determining whether a subject may have mild cognitive impairment, determining whether a subject is at high risk of developing Alzheimer's disease, determining whether a subject is at risk of developing Alzheimer's disease, determining whether a subject is at high risk of developing mild cognitive impairment, and determining whether a subject is at risk of developing mild cognitive impairment, among which determining whether a subject is likely to have Alzheimer's disease, determining whether a subject may have Alzheimer's disease, determining whether a subject is likely to have mild cognitive impairment, or determining whether a subject may have mild cognitive impairment is preferable.

Specifically, it can be determined that, in a case where the ratio ((A)/(B)) of the amount of extracellular vesicles having phosphatidylserine and CD9 to the amount of extracellular vesicles having CD9 is equal to or less than the AD/MCI reference value, a subject is likely to have Alzheimer's disease, or a subject is at high risk of developing Alzheimer's disease; in a case where the ratio ((A)/(B)) is larger than the AD/MCI reference value and is equal to or less than the healthy/AD-MCI reference value, a subject is likely to have mild cognitive impairment, or a subject is at high risk of developing mild cognitive impairment; and in a case where the ratio ((A)/(B)) is larger than the healthy/AD-MCI reference value, a subject is likely to be a healthy subject (the subject is unlikely to have Alzheimer's disease and mild cognitive impairment), or a subject is at low risk of developing Alzheimer's disease and mild cognitive impairment.

The healthy/AD-MCI reference value is preset to distinguish between a patient with Alzheimer's disease or/and a patient with mild cognitive impairment and a healthy subject, and is a ratio of the amount of extracellular vesicles having phosphatidylserine and CD9 to the amount of extracellular vesicles having CD9 ((A)/(B)) in a biological specimen.

The method for determining the healthy/AD-MCI reference value is not particularly limited as long as it is a method used in this field. For example, the healthy/AD-MCI reference value can be determined in such a manner that an amount of extracellular vesicles having CD9 and an amount of extracellular vesicles having phosphatidylserine and CD9 contained in a biological specimen obtained from a patient with Alzheimer's disease or/and a patient with mild cognitive impairment, and a healthy subject are measured; a ratio of the amount of extracellular vesicles having phosphatidylserine and CD9 to the amount of extracellular vesicles having CD9 ((A)/(B)) is calculated based on the measurement results; and a statistical analysis such as receiver operating characteristic analysis (ROC analysis) is carried out using the obtained ratio of the amount of extracellular vesicles having phosphatidylserine and CD9 to the amount of extracellular vesicles having CD9 ((A)/(B)). In setting the healthy/AD-MCI reference value, it is preferable to consider sensitivity, specificity, positive predictive value, negative predictive value, and the like. For example, the healthy/AD-MCI reference value can be set such that the sensitivity is 60% or more, preferably 70% or more, more preferably 80% or more, and particularly preferably 90% or more. For example, the healthy/AD-MCI reference value can be set such that the specificity is 60% or more, preferably 70% or more, more preferably 80% or more, and still more preferably 90% or more.

The AD/MCI reference value is preset to distinguish between a patient with Alzheimer's disease and a patient with mild cognitive impairment, and is a ratio of the amount of extracellular vesicles having phosphatidylserine and CD9 to the amount of extracellular vesicles having CD9 ((A)/(B)) in a biological specimen.

The method for determining the AD/MCI reference value is not particularly limited. For example, the AD/MCI reference value can be determined in such a manner that an amount of extracellular vesicles having CD9 and an amount of extracellular vesicles having phosphatidylserine and CD9 contained in a biological specimen obtained from a patient suffering from Alzheimer's disease and a patient suffering from mild cognitive impairment are measured; a ratio of the amount of extracellular vesicles having phosphatidylserine and CD9 to the amount of extracellular vesicles having CD9 ((A)/(B)) in the biological specimen derived from the subject is calculated based on the measurement results; and a statistical analysis such as receiver operating characteristic analysis (ROC analysis) is carried out using the obtained ratio of the amount of extracellular vesicles having phosphatidylserine and CD9 to the amount of extracellular vesicles having CD9 ((A)/(B)) in the biological specimen derived from the subject. In setting the AD/MCI reference value, it is preferable to consider sensitivity, specificity, positive predictive value, negative predictive value, and the like. For example, the AD/MCI reference value can be set such that the sensitivity is 60% or more, preferably 70% or more, more preferably 80% or more, and particularly preferably 90% or more. For example, the AD/MCI reference value can be set such that the specificity is 60% or more, preferably 70% or more, more preferably 80% or more, and still more preferably 90% or more.

**Reagent kit for assisting diagnosis of Alzheimer's disease or mild cognitive impairment** (the *in vitro* use of the kit is claimed)

The reagent kit for assisting the diagnosis of Alzheimer's disease or mild cognitive impairment in the second invention-1 (hereinafter, often referred to simply as "reagent kit for assisting the diagnosis of AD/MCI") contains a substance having an affinity for CD9 and a substance having an affinity for phosphatidylserine.

The reagent kit for assisting the diagnosis of AD/MCI may be configured in the same manner as the reagent kit (II) for assisting the diagnosis of AD, except that, for example, a substance having an affinity for CD9 is used as the substance having an affinity for tetraspanin.

The substance having an affinity for CD9 and the substance having an affinity for phosphatidylserine in the reagent kit for assisting the diagnosis of AD/MCI are the same as those described above in the AD/MCI diagnosis assisting method, and preferred ones thereof are also the same.

The substance having an affinity for CD9 and the substance having an affinity for phosphatidylserine in the reagent kit for assisting the diagnosis of AD/MCI each may be in a solution state, a frozen state, a dried state, or a freeze-dried state. In addition, the substance having an affinity for CD9 and the substance having an affinity for phosphatidylserine may be contained in the kit as one reagent or may be contained in the kit as separate reagents.

The substance having an affinity for CD9 and the substance having an affinity for phosphatidylserine in the reagent kit for assisting the diagnosis of AD/MCI may be immobilized on a solid phase and may be labeled with a labeling substance. The solid phase, the method for immobilizing the substance on the solid phase, the labeling substance, and the labeling method are the same as those described above in the AD diagnosis assisting method, and preferred ones thereof are also the same.

The reagent kit for assisting the diagnosis of AD/MCI may further contain a secondary affinity substance that specifically binds to the substance having an affinity for CD9 or/and the substance having an affinity for phosphatidylserine. The secondary affinity substance, the labeling substance, and the labeling method in the reagent kit for assisting the diagnosis of AD/MCI are the same as those described above in the AD diagnosis assisting method, and preferred ones thereof are also the same.

The substance having an affinity for CD9 or/and the substance having an affinity for phosphatidylserine in the reagent kit for assisting the diagnosis of AD/MCI may be a substance to which one of avidin and biotin is bound. The avidin and the biotin are the same as those described above in the AD diagnosis assisting method, and preferred ones thereof are also the same.

The reagent kit for assisting the diagnosis of AD/MCI may contain a labeling substance to which one of avidin and biotin is bound. The labeling substance and labeling method are the same as those described above in the AD diagnosis assisting method, and preferred ones thereof are also the same.

The concentration (amount) of the substance having an affinity for CD9 and the substance having an affinity for phosphatidylserine in the reagent kit for assisting the diagnosis of AD/MCI may be appropriately set within the range commonly used in this field depending on the measuring method.

For example, the substance having an affinity for CD9 is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection. In addition, for example, the substance having an affinity for phosphatidylserine is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, the substance having an affinity for CD9 and the substance having an affinity for phosphatidylserine in the reagent kit for assisting the diagnosis of AD/MCI may coexist with a reagent commonly used in this field. The reagent is the same as that described above in the reagent kit for assisting the diagnosis of AD.

Further, the reagent kit for assisting the diagnosis of AD/MCI may contain, in addition to the substance having an affinity for CD9 and the substance having an affinity for phosphatidylserine, a reagent needed to measure an amount of extracellular vesicles having CD9 and phosphatidylserine or/and the amount of extracellular vesicles having CD9 using these affinity substances. Such a reagent is the same as that described above in the reagent kit (II) for assisting the diagnosis of AD.

The reagent kit for assisting the diagnosis of AD/MCI may contain a reference standard used for creating a calibration curve for extracellular vesicles having phosphatidylserine and CD9. The reference standard is the same as that described above in the reagent kit (II) for assisting the diagnosis of AD.

The reagent kit for assisting the diagnosis of AD/MCI may contain a reference standard used for creating a calibration curve for extracellular vesicles having CD9. Examples of the reference standard include an extracellular vesicle having CD9, and a state of the reference standard and a reagent which may coexist with the reference standard are the same as those described above in the reagent kit (II) for assisting the diagnosis of AD.

The reagent kit for assisting the diagnosis of AD/MCI may contain a package insert or an instruction manual. Examples of the package insert or the instruction manual include a package insert or instruction manual stating that a subject is determined to have Alzheimer's disease or mild cognitive impairment by measuring an amount of extracellular vesicles having CD9 and an amount of extracellular vesicles having phosphatidylserine and CD9 in a biological specimen derived from the subject or/and using a ratio of the amount of extracellular vesicles having phosphatidylserine and CD9 to the amount of extracellular vesicles having CD9 as an indicator. These package insert and instruction manual may be described separately in a plurality of cases, or may be described collectively in one.

Specific examples of the reagent kit for assisting the diagnosis of AD/MCI are the same as those described above in the reagent kit (II) for assisting the diagnosis of AD, except that a substance having an affinity for CD9 is used as the substance having an affinity for tetraspanin, and preferred ones thereof are also the same.

Preferred examples of the reagent kit for assisting the diagnosis of AD/MCI include a kit containing a solid phase plate on which an antibody or Tim protein (more preferably Tim4 or Tim1 and particularly preferably the Tim4) that specifically binds to phosphatidylserine is immobilized, a solid phase plate on which an anti-CD9 antibody is immobilized, and any one selected from the following (1) to (3).

(1) a solution containing an anti-CD9 antibody bound to a labeling substance (usually 10 to 5,000 ng/mL and preferably 100 to 500 ng/mL), (2) a solution containing an anti-CD9 antibody (usually 10 to 5,000 ng/mL and preferably 100 to 500 ng/mL), and a solution containing a secondary affinity substance that specifically binds to the anti-CD9 antibody bound to a labeling substance (usually 10 to 5,000 ng/mL and preferably 100 to 500 ng/mL), and (3) a solution containing an anti-CD9 antibody to which one of avidin and biotin is bound (usually 10 to 5,000 ng/mL and more preferably 100 to 500 ng/mL), and a solution containing a labeling substance to which the other one of avidin and biotin is bound (usually 10 to 5,000 ng/mL and preferably 100 to 500 ng/mL).

**Device for assisting diagnosis of Alzheimer's disease or mild cognitive impairment** (not claimed, for reference only)

The device for assisting the diagnosis of Alzheimer's disease or mild cognitive impairment in the second invention-1 (hereinafter, often referred to simply as "device for assisting the diagnosis of AD/MCI") has a measurement unit that measures an amount of extracellular vesicles having phosphatidylserine and CD9 and an amount of extracellular vesicles having CD9, in a biological specimen derived from a subject; a calculation unit that calculates a ratio of the amount of extracellular vesicles having phosphatidylserine and CD9 in the biological specimen derived from the subject to the amount of extracellular vesicles having CD9 in the biological specimen derived from the subject; and a determination unit that determines that the subject has Alzheimer's disease or mild cognitive impairment using the ratio of the amount of extracellular vesicles having phosphatidylserine and CD9 to the amount of extracellular vesicles having CD9 in the biological specimen derived from the subject as an indicator.

The subject, the biological specimen, and the extracellular vesicle in the device for assisting the diagnosis of AD/MCI are the same as those described above, and preferred ones thereof are also the same. The extracellular vesicle having phosphatidylserine and CD9, the amount, and the extracellular vesicle having CD9 in the device for assisting the diagnosis of AD/MCI are the same as those described above in the AD/MCI diagnosis assisting method, and preferred ones thereof are also the same.

The measurement unit, the calculation unit, the determination unit, and the like constituting the device for assisting the diagnosis of AD/MCI may be arranged in the same device or may be separate bodies.

The measurement unit in the device for assisting the diagnosis of AD/MCI is a site for measuring the amount of extracellular vesicles having phosphatidylserine and CD9 and the amount of extracellular vesicles having CD9 in a biological specimen derived from a subject introduced into the device. The size and configuration of the measurement unit are not particularly limited.

Examples of the measurement unit include an imaging apparatus for imaging using a microplate reader or CCD used for ELISA, an imaging apparatus used for Western blotting or a method using a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The measurement of the amount of extracellular vesicles having phosphatidylserine and CD9, and the measurement of the amount of the extracellular vesicles having CD9 in the device for assisting the diagnosis of AD/MCI are the same as those described above in the AD/MCI diagnosis assisting method, and preferred ones and specific examples thereof are also the same.

The calculation unit in the device for assisting the diagnosis of AD/MCI is a site for calculating a ratio of an amount of extracellular vesicles having phosphatidylserine and CD9 in a biological specimen derived from a subject to an amount of extracellular vesicles having CD9 in the biological specimen derived from the subject. The size and configuration of the measurement unit are not particularly limited.

The amount of extracellular vesicles having CD9 in the biological specimen derived from the subject and the amount of extracellular vesicles having phosphatidylserine and CD9 in the biological specimen derived from the subject may be calculated in such a manner that an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index) having a correlation with the mass or concentration of extracellular vesicles having CD9 in the biological specimen derived from the subject and extracellular vesicles having phosphatidylserine and CD9 in the biological specimen derived from the subject, obtained by the measurement unit in the device for assisting the diagnosis of AD/MCI, is converted into mass, concentration, or the like, and then the ratio of the amount of extracellular vesicles having phosphatidylserine and CD9 to the amount of extracellular vesicles having CD9 is calculated.

It should be noted that the actually measured value, the mass, concentration, or the like converted by the calculation unit, and the ratio of the amount of extracellular vesicles having phosphatidylserine and CD9 to the amount of extracellular vesicles having CD9 may be stored in a storage device or the like provided in a device such as a memory device or a hard disk.

The determination unit in the device for assisting the diagnosis of AD/MCI is a site for determining Alzheimer's disease or mild cognitive impairment using the ratio of the amount of extracellular vesicles having phosphatidylserine and CD9 to the amount of extracellular vesicles having CD9 obtained by the calculation unit as an indicator.

The determination of Alzheimer's disease or mild cognitive impairment in the device for assisting the diagnosis of AD/MCI is the same as the determination of Alzheimer's disease or mild cognitive impairment in the AD/MCI diagnosis assisting method, and preferred ones and specific examples thereof are also the same.

The determination of Alzheimer's disease or mild cognitive impairment in the device for assisting the diagnosis of AD/MCI is made, for example, by comparing the ratio ((A)/(B)) of the amount of extracellular vesicles having phosphatidylserine and CD9 to the amount of extracellular vesicles having CD9 in the biological specimen derived from the subject obtained by the calculation unit with a predetermined reference value (cutoff value, hereinafter often referred to simply as "healthy/AD-MCI reference value"). The predetermined reference value may be stored in advance in the device for assisting the diagnosis of AD/MCI, or may be input from an input site of the device for assisting the diagnosis of AD/MCI at the time of determination.

A plurality of reference values (cutoff values) may be used in determining Alzheimer's disease or mild cognitive impairment in the device for assisting the diagnosis of AD/MCI.

In the determination of Alzheimer's disease or mild cognitive impairment in the device for assisting the diagnosis of AD/MCI, the determination in a case where a plurality of reference values (cutoff values) are used is the same as the determination in the AD/MCI diagnosis assisting method, and preferred ones and specific examples thereof are also the same.

The healthy/AD-MCI reference value, the method for determining the healthy/AD-MCI reference value, the AD/MCI reference value, and the method for determining the AD/MCI reference value in the device for assisting the diagnosis of AD/MCI are also the same as those described in the AD/MCI diagnosis assisting method, and preferred ones thereof are also the same.

The device for assisting the diagnosis of AD/MCI may contain an output unit. The output unit carries out processing such as displaying or outputting the results of the determination to a display device such as a display or a printing device such as a printer.

2-2. Second invention-2

Biomarker set for assisting diagnosis of Alzheimer's disease or **mild** cognitive impairment (not claimed, for reference only)

The biomarker set for assisting the diagnosis of Alzheimer's disease in the second invention-2 (hereinafter, often referred to simply as "AD/MCI marker set (II)") is a combination of biomarkers containing extracellular vesicles having phosphatidylserine and CD9, extracellular vesicles having CD9, amyloid β (1-40), and amyloid β (1-42). That is, the AD/MCI marker set (II) is a combination of biomarkers containing the AD/MCI marker set, amyloid β (1-40), and amyloid β (1-42).

According to the AD/MCI marker set (II), for example, a value (hereinafter, often referred to simply as "AD/MCI marker (II)") obtained by multiplying the ratio of the amount of extracellular vesicles having phosphatidylserine and CD9 to the amount of extracellular vesicles having CD9 (AD/MCI marker) by a ratio of the amount of amyloid β (1-42) to the amount of amyloid β (1-40) (hereinafter, often referred to simply as "Aβ (1-42)/Aβ (1-40)") can be obtained, and the AD/MCI marker (II) can be used as an indicator for determining that a subject has Alzheimer's disease or mild cognitive impairment.

All the descriptions regarding the AD/MCI marker set in the AD/MCI marker set (II) are the same as those in the AD/MCI marker set of the second invention-1, and preferred ones and specific examples thereof are also the same.

**Method for assisting diagnosis of Alzheimer's disease or mild cognitive impairment** (not claimed, for reference only)

The method for assisting the diagnosis of Alzheimer's disease or mild cognitive impairment in the second invention-2 (hereinafter, often referred to simply as "AD/MCI diagnosis assisting method (II)") contains measuring an amount of extracellular vesicles having phosphatidylserine and CD9 in a biological specimen and an amount of extracellular vesicles having CD9 in the biological specimen; calculating a ratio of the amount of extracellular vesicles having phosphatidylserine and CD9 to the amount of extracellular vesicles having CD9 (AD/MCI marker); and determining that a subject has Alzheimer's disease or mild cognitive impairment using a value (AD/MCI marker (II)) obtained by multiplying the ratio of the amount of extracellular vesicles having phosphatidylserine and CD9 to the amount of extracellular vesicles having CD9 by the ratio of the amount of amyloid β (1-42) to the amount of amyloid β (1-40) ("Aβ (1-42)/Aβ (1-40)") as an indicator.

All the descriptions regarding the AD/MCI marker in the AD/MCI diagnosis assisting method (II) are the same as those in the AD/MCI marker in the second invention-1, and preferred ones and specific examples thereof are also the same.

The AD/MCI diagnosis assisting method (II) may further contain measuring the amount of amyloid β (1-40) or/and the amount of amyloid β (1-42).

All the descriptions regarding the measurement of the amount of amyloid β (1-40) or/and the amount of amyloid β (1-42) are the same as those in the measurement of the amount of amyloid β (1-40) or/and the amount of amyloid β (1-42) in the first invention-3, and preferred ones and specific examples thereof are also the same.

The determination of Alzheimer's disease or mild cognitive impairment in the AD/MCI diagnosis assisting method (II) contains determining Alzheimer's disease or mild cognitive impairment using the AD/MCI marker (II) as an indicator.

The determination of Alzheimer's disease or mild cognitive impairment may be carried out using the AD/MCI marker (II) as an indicator, according to the AD/MCI diagnosis assisting method (determination of Alzheimer's disease or mild cognitive impairment using the AD/MCI marker as an indicator) in the second invention-1, and preferred ones and specific examples thereof are also the same.

**Reagent kit** (II) **for assisting diagnosis of Alzheimer's disease or mild cognitive impairment** (not claimed, for reference only)

The reagent kit for assisting the diagnosis of Alzheimer's disease or mild cognitive impairment in the second invention-2 (hereinafter, often referred to simply as "reagent kit (II) for assisting the diagnosis of AD/MCI") contains a

**EP 4 067 906 B1**

substance having an affinity for CD9, a substance having an affinity for phosphatidylserine, a substance having an affinity for amyloid β (1-40), and a substance having an affinity for amyloid β (1-42), and if necessary, further a substance having an affinity for an N-terminal region of amyloid β.

That is, the reagent kit (II) for assisting the diagnosis of AD/MCI is a reagent kit containing the reagent kit for assisting the diagnosis of AD/MCI, a substance having an affinity for amyloid β (1-40), and a substance having an affinity for amyloid β (1-42), and if necessary, further a substance having an affinity for an N-terminal region of amyloid β.

All the descriptions regarding the reagent kit for assisting the diagnosis of AD/MCI in the reagent kit (II) for assisting the diagnosis of AD/MCI are the same as those in the reagent kit for assisting the diagnosis of AD/MCI in the second invention-1, and preferred ones and specific examples thereof are also the same.

The substance having an affinity for amyloid β (1-40), the substance having an affinity for amyloid β (1-42), the substance having an affinity for an N-terminal region of amyloid β, the combinations of these substances having affinity, and the like in the reagent kit (II) for assisting the diagnosis of AD/MCI are the same as those in the reagent kit (III) for assisting the diagnosis of AD, and preferred ones thereof are also the same.

The concentration (amount) of the substance having an affinity for amyloid β (1-40) or the substance having an affinity for amyloid β (1-42) in the reagent kit (II) for assisting the diagnosis of AD/MCI may be appropriately set within the range commonly used in this field depending on the measuring method. For example, the substance having an affinity for amyloid β (1-40) or the substance having an affinity for amyloid β (1-42) is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, for example, the substance having an affinity for an N-terminal region of amyloid β is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

Further, the reagent kit (II) for assisting the diagnosis of AD/MCI may contain a reagent needed to measure an amount of amyloid β (1-40) or/and an amount of amyloid β (1-42), in addition to the reagent kit for assisting the diagnosis of AD/MCI, the substance having an affinity for amyloid β (1-40), the substance having an affinity for amyloid β (1-42), or/and the substance having an affinity for an N-terminal region of amyloid β. Such a reagent is the same as that of the reagent kit for assisting the diagnosis of AD.

The reagent kit (II) for assisting the diagnosis of AD/MCI may contain a reference standard used for creating a calibration curve for amyloid β (1-40) or/and amyloid β (1-42). Examples of the reference standard include amyloid β (1-40) and amyloid β (1-42). The reference standard may be in a solution state, a frozen state, a dried state, or a freeze-dried state. In addition, reagents commonly used in this field, for example, a buffer, a reaction promoter, a sugar, a protein, a salt, a stabilizer such as a surfactant, and a preservative, may coexist with the reference standard. The concentration and pH of these reagents may be appropriately selected from the ranges commonly used in this field.

The reagent kit (II) for assisting the diagnosis of AD/MCI may contain a package insert or an instruction manual. Examples of the package insert or instruction manual include a package insert or instruction manual that describes measuring an amount of amyloid β (1-40) or/and an amount of amyloid β (1-42) in a biological specimen derived from a subject, calculating an AD/MCI marker (II), and determining that the subject has Alzheimer's disease or mild cognitive impairment using the AD/MCI marker (II) as an indicator. These package insert and instruction manual may be described separately in a plurality of cases, or may be described collectively in one.

Specifically, the reagent kit (II) for assisting the diagnosis of AD/MCI may be, for example, a kit containing one of "a substance having an affinity for amyloid β (1-40) or a substance having an affinity for amyloid β (1-42)" and a substance having an affinity for an N-terminal region of amyloid β immobilized on a solid phase and the other one of "a substance having an affinity for amyloid β (1-40) or a substance having an affinity for amyloid β (1-42)" and a substance having an affinity for an N-terminal region of amyloid β bound to a labeling substance or the other one affinity substance and a component for indirectly binding the other one affinity substance to a labeling substance.

The reagent kit (II) for assisting the diagnosis of AD/MCI is preferably, for example, a kit containing one of "a substance having an affinity for amyloid β (1-40) or a substance having an affinity for amyloid β (1-42)" and a substance having an affinity for an N-terminal region of amyloid β immobilized on a solid phase and any one selected from the following (1) to (3).

(1) the other one of "a substance having an affinity for amyloid β (1-40) or a substance having an affinity for amyloid β (1-42)" and a substance having an affinity for an N-terminal region of amyloid β bound to a labeling substance,
(2) the other one of "a substance having an affinity for amyloid β (1-40) or a substance having an affinity for amyloid β (1-42)" and a substance having an affinity for an N-terminal region of amyloid β, and a secondary affinity substance that specifically binds to the affinity substance bound to a labeling substance, and
(3) the other one of "a substance having an affinity for amyloid β (1-40) or a substance having an affinity for amyloid β (1-42)" and a substance having an affinity for an N-terminal region of amyloid β to which one of avidin and biotin is bound, and a labeling substance to which the other one of avidin and biotin is bound.

**Device** (II) **for assisting diagnosis of Alzheimer's disease or mild cognitive impairment** (not claimed, for reference only)

The device for assisting the diagnosis of Alzheimer's disease or mild cognitive impairment in the second invention-2 (hereinafter, often referred to simply as "device (II) for assisting the diagnosis of AD/MCI") has a measurement unit that measures an amount of extracellular vesicles having phosphatidylserine and CD9 and an amount of extracellular vesicles having CD9, in a biological specimen derived from a subject; a calculation unit that multiplies a ratio of the amount of extracellular vesicles having phosphatidylserine and CD9 to the amount of extracellular vesicles having CD9 (AD/MCI marker) by a ratio of an amount of amyloid β (1-42) to an amount of amyloid β (1-40) (Aβ (1-42)/Aβ (1-40)) to calculate an AD/MCI marker (II); and a determination unit that determines that the subject has Alzheimer's disease or mild cognitive impairment using the AD/MCI marker (II) as an indicator.

The device (II) for assisting the diagnosis of AD/MCI may have, if necessary, a measurement unit that measures an amount of amyloid β (1-40) or/and an amount of amyloid β (1-42); an input unit that inputs the amount of amyloid β (1-40) or/and the amount of amyloid β (1-42) or a ratio of the amount of amyloid β (1-42) to the amount of amyloid β (1-40) (Aβ (1-42)/Aβ (1-40)) from the outside; and a calculation unit that calculates the ratio of the amount of amyloid β (1-42) to the amount of amyloid β (1-40) (Aβ (1-42)/Aβ (1-40)).

The measurement unit, the calculation unit, the input unit, the determination unit, and the like constituting the device (II) for assisting the diagnosis of AD/MCI may be arranged in the same device or may be separate bodies.

The measurement unit in the device (II) for assisting the diagnosis of AD/MCI is a site for measuring the amount of extracellular vesicles having phosphatidylserine and CD9 and the amount of extracellular vesicles having CD9 in a biological specimen introduced into the device. If necessary, the measurement unit is also a site for further measuring the amount of amyloid β (1-40) or/and the amount of amyloid β (1-42) in the biological specimen introduced into the device.

The size and configuration of the measurement unit are not particularly limited.

Examples of the measurement unit include an imaging apparatus for imaging using a microplate reader or CCD used for ELISA, an imaging apparatus used for Western blotting or a method using a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The subject, the biological specimen, and the extracellular vesicle in the device (II) for assisting the diagnosis of AD/MCI are the same as those described above, and preferred ones thereof are also the same.

The extracellular vesicles having phosphatidylserine and CD9, the amount, the target for measuring the amount of extracellular vesicles having phosphatidylserine and CD9, the measurement of the amount of extracellular vesicles having phosphatidylserine and CD9 and the substances used for the measurement, the extracellular vesicles having CD9, the target for measuring the amount of extracellular vesicles having CD9, and the measurement of the amount of extracellular vesicles having CD9 and the substances used for the measurement in the device (II) for assisting the diagnosis of AD/MCI are the same as those of the AD/MCI diagnosis assisting method (II), and preferred ones and specific examples thereof are also the same.

The calculation unit in the device (II) for assisting the diagnosis of AD/MCI is a site for calculating the AD/MCI marker (II) obtained by multiplying the ratio of the amount of extracellular vesicles having phosphatidylserine and CD9 to the amount of extracellular vesicles having CD9 (AD/MCI marker) by the ratio of the amount of amyloid β (1-42) to the amount of amyloid β (1-40) (Aβ (1-42)/Aβ (1-40)), based on the amount of extracellular vesicles having CD9 and the amount of extracellular vesicles having phosphatidylserine and CD9 obtained by the measurement unit, and the amount of amyloid β (1-40) or/and the amount of amyloid β (1-42) measured by the measurement unit, or the amount of amyloid β (1-40) or/and the amount of amyloid β (1-42), or the ratio of the amount of amyloid β (1-42) to the amount of amyloid β (1-40) (Aβ (1-42)/Aβ (1-40)) input from the input unit from the outside.

The size and configuration of the calculation unit are not particularly limited.

The calculation unit in the device (II) for assisting the diagnosis of AD/MCI may calculate the AD/MCI marker (II) after converting an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index) having a correlation with the measured or input mass or concentration into mass, concentration, or the like.

It should be noted that the actually measured value, the mass, concentration, or the like converted by the calculation unit, the AD/MCI marker, the ratio of the amount of amyloid β (1-42) to the amount of amyloid β (1-40) (Aβ (1-42)/Aβ (1-40)), and the AD/MCI marker (II) may be stored in a storage device or the like provided in a device such as a memory device or a hard disk.

The determination unit in the device (II) for assisting the diagnosis of AD/MCI is a site for determining Alzheimer's disease or mild cognitive impairment using the AD/MCI marker (II) calculated by the calculation unit as an indicator.

The determination of Alzheimer's disease or mild cognitive impairment and the predetermined reference value used for the determination in the device (II) for assisting the diagnosis of AD/MCI, and the method for determining the predetermined reference value are the same as those in the AD/MCI diagnosis assisting method (II) and preferred ones and specific examples thereof are also the same.

The predetermined reference value in the device (II) for assisting the diagnosis of AD/MCI may be stored in advance in the device (II) for assisting the diagnosis of AD/MCI, or may be input from an input site of the device (II) for assisting the diagnosis of AD/MCI at the time of determination.

The device (II) for assisting the diagnosis of AD/MCI may contain an output unit. The output unit carries out processing such as displaying or outputting the results of the determination to a display device such as a display or a printing device such as a printer.

According to the present invention, data (determination result) for assisting the diagnosis of a subject regarding Alzheimer's disease or mild cognitive impairment by a physician can be obtained.

In a case where the subject is determined to have Alzheimer's disease or mild cognitive impairment by the present invention, the physician may further carry out, for example, a medical interview, a diagnostic method using an imaging apparatus such as amyloid PET diagnosis, an MMSE test, a test for a biomarker or the like of Alzheimer's disease or mild cognitive impairment recommended in the dementia disease treatment guideline such as a decreased level of Aβ42 or an increased level of Tau or phosphorylated Tau in cerebrospinal fluid, or a test using a candidate substance for a biomarker of Alzheimer's disease or mild cognitive impairment, and can make a diagnosis of Alzheimer's disease or mild cognitive impairment of the subject in consideration of the results thereof and the like.

In addition, in a case where the subject is determined to have Alzheimer's disease or mild cognitive impairment by the present invention, a drug or therapeutic agent that delays the progression of Alzheimer's disease or mild cognitive impairment such as a cholinesterase inhibitor may be administered, or surgery may be carried out.

Industrial Applicability

The method for assisting the diagnosis of Alzheimer's disease or mild cognitive impairment, the *in vitro* use of the biomarker, and the *in vitro* use of the reagent kit according to the present invention can assist in the diagnosis of Alzheimer's disease or mild cognitive impairment and are therefore useful in the field of clinical examination. According to the present invention, it is possible to assist in the diagnosis of Alzheimer's disease or mild cognitive impairment with high accuracy.

Examples

Hereinafter, the present invention will be described in more detail based on Examples and Comparative Examples, but the present invention is not limited to these examples.

Example 1. Evaluation of Alzheimer's disease test specimen using amount of exosome having PS and CD9 as indicator

Exosomes were measured by sandwich ELISA of Tim protein-anti-CD9 antibody, and AUC and p-value were calculated.

**(1) Preparation of calibrator**

Using MagCapture (registered trademark) Exosome Isolation Kit PS (manufactured by FUJIFILM Wako Pure Chemical Corporation, hereinafter referred to as "Akit"), exosomes were isolated from COLO201 cell culture supernatant according to the instruction manual attached to the kit, and the exosomes were eluted using an eluent attached to the kit. Using a protein assay BCA kit (manufactured by FUJIFILM Wako Pure Chemical Corporation), a protein concentration in the obtained exosome solution was measured by a bicinchoninic acid method (BCA method).

Next, using Reaction Buffer attached to PS Capture Exosome ELISA Kit, Streptavidin HRP (manufactured by FUJIFILM Wako Pure Chemical Corporation, hereinafter referred to as "B kit"), the obtained exosome solution was diluted to 0.156, 0.313, 0.625, 1.25, 2.50, 5.00, 10.0, and 20.0 ng/mL, based on the protein concentration measured by the BCA method, whereby an exosome dilution series consisting of 8-point concentrations (hereinafter, referred to as "calibrator") derived from the COLO201 cell culture supernatant was obtained.

## (2) Pretreatment of test specimen for measurement

Cerebrospinal fluid (CSF) from 8 test specimens of a patient with Alzheimer's disease (AD) purchased from PrecisionMed, LLC. and cerebrospinal fluid (CSF) from seven test specimens of a healthy subject were each centrifuged at 10,000 g for 20 minutes, and the supernatants were collected. Each of the obtained supernatants was diluted 100-fold with Reaction Buffer attached to the A kit to obtain a "test specimen diluted solution for measurement".

## (3) Measurement by ELISA

The test specimen diluted solution for measurement prepared in (2) was measured by sandwich ELISA of Tim4 protein-anti-CD9 antibody in which the Tim4 protein was immobilized on a solid phase and the anti-CD9 antibody was used as an antibody for detection. Specifically, an anti-CD9 mouse monoclonal antibody (1K, manufactured by FUJIFILM Wako Pure Chemical Corporation) was reduced with 1.6 mM DTT and then reacted with Biotin-PEAC5-maleimide (manufactured by Dojindo Laboratories) at 37°C for 1.5 hours to prepare a biotin-labeled anti-CD9 mouse monoclonal antibody. The reagents attached to the B kit were used, except for the antibody for detection.

First, Washing Buffer ($10\times$) attached to the B kit was diluted 10-fold with purified water (distilled water), and then Exosome Binding Enhancer ($100\times$) attached to the B kit was added in an amount of 1/100 to the obtained diluted solution. The obtained solution is referred to as "washing solution ($1\times$)". Next, each well of a "96-well plate in which the Tim4 protein was immobilized on a solid phase" attached to the B kit was washed 3 times with 300 to 350 $\mu$L of the washing solution ($1\times$).

Next, the test specimen diluted solution for measurement prepared in (2) (8 test specimens, n = 2, 16 wells), the calibrator prepared in (2) (8 points, n = 2, 16 wells), and Reaction Buffer (n = 2, 2 wells) attached to the B kit as a blank were dispensed at an amount of 100 $\mu$L/well into each well of the plate. Then, a plate seal was attached to the plate, followed by reaction at room temperature for 2 hours while stirring at about 500 rpm using a microplate shaker. After the reaction was completed, the reaction solution was discarded and each well was washed 3 times with 300 to 350 $\mu$L of the washing solution ($\times$ 1).

Then, using Reaction Buffer attached to the B kit, the biotin-labeled anti-CD9 mouse monoclonal antibodies were diluted to a final concentration of 250 ng/mL to obtain a biotin-labeled antibody reaction solution. The obtained biotin-labeled antibody reaction solution was dispensed at an amount of 100 $\mu$L/well into each well of the plate, and a plate seal was attached to the plate, followed by reaction at room temperature for 1 hour while stirring at about 500 rpm using a microplate shaker. After the reaction was completed, the reaction solution was discarded and each well was washed 3 times with 300 to 350 $\mu$L of the washing solution ($\times$1).

HRP-conjugated streptavidin ($100\times$) in an amount of 1/100 was added to Reaction Buffer attached to the B kit, which was then thoroughly mixed, the prepared HRP-labeled streptavidin reaction solution ($1\times$) was dispensed at an amount of 100 $\mu$L/well into each well of the plate, and a plate seal was attached to the plate, followed by reaction at room temperature for 2 hours while stirring at about 500 rpm using a microplate shaker. After the reaction was completed, the reaction solution was discarded and each well was washed 5 times with 300 to 350 $\mu$L of the washing solution ($1\times$).

Next, the 3,3',5,5'-tetramethylbenzidine (TMB) solution attached to the B kit, which was returned to room temperature, was dispensed at an amount of 100 $\mu$L/well into each well of the plate, followed by stirring for about 1 minute using a microplate shaker. After that, a plate seal was attached to the plate which was then allowed to stand at room temperature (20°C to 25°C) for 30 minutes. Then, the stop solution attached to the B kit, which was returned to room temperature, was dispensed at an amount of 100 $\mu$L/well into each well of the plate, followed by stirring for about 5 seconds using a microplate shaker, and the absorbance at 450 nm and the absorbance at a sub-wavelength of 620 nm were immediately measured using a 96-well microplate reader (Safire2, available from Tecan Group Ltd.). The value obtained by subtracting the absorbance value at a sub-wavelength of 620 nm from the absorbance value at 450 nm was defined as an "absorbance value", and the value obtained by subtracting the blank absorbance value from the absorbance value of the test specimen diluted solution for measurement was defined as a "corrected test specimen absorbance value". Then, a standard curve was created from the value obtained by subtracting the blank absorbance value from the absorbance value of the exosome dilution series (calibrator) derived from COLO201 cell culture supernatant and the protein concentration of the calibrator. The corrected test specimen absorbance value was converted into a protein concentration using the standard curve, and the value obtained by multiplying the obtained corresponding value by the test specimen dilution rate was defined as a "test specimen measurement value" [ng/mL].

## (4) Calculation of AUC

Based on the test specimen measurement value obtained in (3), a significant difference test between a patient with Alzheimer's disease and a healthy subject was carried out by the Wilcoxon/Kruskal-Wallis test (rank sum) using JMP (registered trademark) 11 (available from SAS Institute Inc., Cary, NC, USA), and a p-value was calculated. In addition, based on the test specimen measurement value obtained in (3), a logistic regression analysis was carried out using JMP (registered trademark) 11 (available from SAS Institute Inc., Cary, NC, USA), and an area under the curve (AUC) value was calculated from the obtained receiver operating characteristic curve (ROC curve).

The obtained results are shown in Table 1 which will be given later. In addition, Fig. 1 shows a box plot graph created based on the test specimen measurement value. In the figure, the vertical axis shows the test specimen measurement value, and Control on the horizontal axis shows the results of a healthy subject, and AD on the horizontal axis shows the results of a patient with Alzheimer's disease.

Comparative Example 1. Evaluation of Alzheimer's disease test specimen using amount of exosome having CD9 as indicator

Exosomes were measured by sandwich ELISA of anti-CD9 antibody-anti-CD9 antibody in the same manner as in Example 1, except that an anti-CD9 antibody was immobilized on a solid phase instead of the Tim4 protein. Then, a significant difference test between a patient with Alzheimer's disease and a healthy subject was carried out, and the AUC and p-value were calculated.

A plate in which the anti-CD9 antibody was immobilized on a solid phase was prepared by the following method. Anti-CD9 mouse monoclonal antibodies (1K) (manufactured by FUJIFILM Wako Pure Chemical Corporation) were diluted with 50 mM MOPS (pH 7.5) to a concentration of 10 $\mu$g/mL, and were added at an amount of 100 $\mu$L/well to wells of a 96-well microplate (available from Nunc Inc.) which was then incubated overnight in a refrigerator. The wells were washed three times with TBST (Tris Buffered Saline, pH 7.4), 300 $\mu$L of TBS (Tris Buffered Saline, pH 7.4) containing 10 mg/mL of Block Ace was added thereto, followed by incubation overnight in a refrigerator. The thus-treated plate was used as an antibody-immobilized plate.

As the anti-CD9 antibody of the detection antibody, a biotin-labeled antibody was used in the same manner as in Example 1.

The obtained results are shown in Table 1 which will be given later.

Example 2. Evaluation of Alzheimer's disease test specimen using amount of exosome having PS and CD63 as indicator

Exosomes were measured by sandwich ELISA of Tim protein-anti-CD63 antibody in the same manner as in Example 1, except that an anti-CD63 antibody was used instead of the anti-CD9 antibody as the detection antibody. Then, a significant difference test between a patient with Alzheimer's disease and a healthy subject was carried out, and the AUC and p-value were calculated.

The biotin-labeled anti-CD63 antibody attached to PS Capture Exosome ELISA Kit, Streptavidin HRP (manufactured by FUJIFILM Wako Pure Chemical Corporation, the "B kit" described above) was used as the anti-CD63 antibody of the detection antibody.

The obtained results are shown in Table 1 which will be given later.

Comparative Example 2. Evaluation of Alzheimer's disease test specimen using CD63 as indicator

Exosomes were measured by sandwich ELISA of anti-CD63 antibody-anti-CD63 antibody in the same manner as in Comparative Example 1, except that an anti-CD63 mouse monoclonal antibody (3-13) (manufactured by FUJIFILM Wako Pure Chemical Corporation) was immobilized on a solid phase instead of the anti-CD9 antibody and used as the detection antibody. Then, a significant difference test between a patient with Alzheimer's disease and a healthy subject was carried out, and the AUC and p-value were calculated.

A plate in which the anti-CD63 antibody was immobilized on a solid phase was prepared in the same manner as in Comparative Example 1. The biotin-labeled anti-CD63 antibody attached to PS Capture Exosome ELISA Kit, Streptavidin HRP (manufactured by FUJIFILM Wako Pure Chemical Corporation, the "B kit" described above) was used as the anti-CD63 antibody of the detection antibody.

The obtained results are shown in Table 1 which will be given later.

Example 3. Evaluation of Alzheimer's disease test specimen using amount of exosome having PS and CD81 as indicator

Exosomes were measured by sandwich ELISA of Tim protein-anti-CD81 antibody in the same manner as in Example 1, except that an anti-CD81 mouse monoclonal antibody (17B1) (manufactured by FUJIFILM Wako Pure Chemical Corporation) was used instead of the anti-CD9 antibody as the detection antibody. Then, a significant difference test between a patient with Alzheimer's disease and a healthy subject was carried out, and the AUC and p-value were calculated.

As the anti-CD81 antibody of the detection antibody, a biotin-labeled antibody was used in the same manner as in Example 1.

The obtained results are shown in Table 1 which will be given later.

Comparative Example 3. Evaluation of Alzheimer's disease test specimen using amount of exosome having CD81 as indicator

Exosomes were measured by sandwich ELISA of anti-CD81 antibody-anti-CD81 antibody in the same manner as in Example 1, except that an anti-CD81 mouse monoclonal antibody (17B1) (manufactured by FUJIFILM Wako Pure Chemical Corporation) was immobilized on a solid phase instead of the anti-CD9 antibody and used as the detection antibody. Then, a significant difference test between a patient with Alzheimer's disease and a healthy subject was carried out, and the AUC and p-value were calculated.

A plate in which the anti-CD81 antibody was immobilized on a solid phase was prepared in the same manner as in Comparative Example 1.

As the anti-CD81 antibody of the detection antibody, a biotin-labeled antibody was used in the same manner as in Example 1.

The obtained results are shown in Table 1 which will be given later.

Example 4. Evaluation of Alzheimer's disease test specimen using ratio of amount of exosomes having PS and CD9 to amount of exosomes having CD9 as indicator

The "test specimen measurement value" (value (A)) obtained in Example 1 (sandwich ELISA of Tim protein-anti-CD9 antibody) was divided by the "test specimen measurement value" (value (B)) obtained in Comparative Example 1 (sandwich ELISA of anti-CD9 antibody-anti-CD9 antibody) to obtain (A)/(B) (hereinafter, referred to as "corrected test specimen measurement value").

Based on the obtained corrected test specimen measurement value, a significant difference test between a patient with Alzheimer's disease and a healthy subject was carried out in the same manner as in Example 1 (4), and the AUC and p-value were calculated.

The obtained results are shown in Table 1 which will be given later. In addition, Fig. 2 shows a box plot graph created based on the corrected test specimen measurement value together with the results of Example 6 and Example 7. In the figure, the vertical axis shows the corrected test specimen measurement value ((A)/(B)), and Control on the horizontal axis shows the results of a healthy subject, MCI on the horizontal axis shows the results of a patient with mild cognitive impairment, and AD on the horizontal axis shows the results of a patient with Alzheimer's disease.

Example 5. Evaluation of Alzheimer's disease test specimen using ratio of amount of exosomes having PS and CD63 to amount of exosomes having CD63 as indicator

(A) the "test specimen measurement value" obtained in Example 2 (sandwich ELISA of Tim protein-anti-CD63 antibody) was divided by (B) the "test specimen measurement value" obtained in Comparative Example 2 (sandwich ELISA of anti-CD63 antibody-anti-CD63 antibody) to obtain (A)/(B) (corrected test specimen measurement value).

Based on the obtained corrected test specimen measurement value, a significant difference test between a patient with Alzheimer's disease and a healthy subject was carried out in the same manner as in Example 1 (4), and the AUC and p-value were calculated.

The obtained results are shown in Table 1 which will be given later.

Table 1

| | Specimen | Solid phase | Detection | Test specimen measurement value used for analysis | Healthy subject-AD | |
|---|---|---|---|---|---|---|
| | | | | | AUC | p-value |
| Example 1 | | Tim4 | αCD9 | Value (A) | 0.839 | 0.032 |
| Comparative Example 1 | | αCD9 | αCD9 | Value (B) | 0.589 | 0.603 |
| Example 4 | | | | Value (A)/Value (B) | 0.964 | 0.003 |
| Example 2 | Cerebrospinal fluid (CSF) | Tim4 | αCD63 | Value (A) | 0.839 | 0.032 |
| Comparative Example 2 | | αCD63 | αCD63 | Value (B) | 0.741 | 0.132 |
| Example 5 | | | | Value (A)/Value (B) | 0.839 | 0.032 |

(continued)

| | Specimen | Solid phase | Detection | Test specimen measurement value used for analysis | Healthy subject-AD | |
|---|---|---|---|---|---|---|
| | | | | | AUC | p-value |
| Example 3 | | Tim4 | αCD81 | Value (A) | 0.857 | 0.024 |
| Comparative Example 3 | | αCD81 | αCD81 | Value (B) | 0.714 | 0.183 |

Comparative Example 4. Evaluation of mild cognitive impairment test specimen using amount of exosome having PS and CD9 as indicator

Exosomes were measured by sandwich ELISA of Tim protein-anti-CD9 antibody in the same manner as in Example 1, except that the cerebrospinal fluid from 7 test specimens of a patient with mild cognitive impairment (MCI) purchased from PrecisionMed, LLC. was used instead of the test specimen of a patient with Alzheimer's disease (AD). Then, a significant difference test between a patient with mild cognitive impairment and a healthy subject was carried out, and the AUC and p-value were calculated.
The obtained results are shown in Table 2 which will be given later.

Comparative Example 5. Evaluation of mild cognitive impairment test specimen using amount of exosome having CD9 as indicator

Exosomes were measured by sandwich ELISA of anti-CD9 antibody-anti-CD9 antibody in the same manner as in Comparative Example 1, except that the cerebrospinal fluid from 7 test specimens of a patient with mild cognitive impairment (MCI) purchased from PrecisionMed, LLC. was used instead of the test specimen of a patient with Alzheimer's disease (AD). Then, a significant difference test between a patient with mild cognitive impairment and a healthy subject was carried out, and the AUC and p-value were calculated.
The obtained results are shown in Table 2 which will be given later.

Example 6. Evaluation of mild cognitive impairment test specimen using ratio of amount of exosomes having PS and CD9 to amount of exosomes having CD9 as indicator

The "test specimen measurement value" obtained in Comparative Example 4 (sandwich ELISA of Tim protein-anti-CD9 antibody) was divided by the "test specimen measurement value" obtained in Comparative Example 5 (sandwich ELISA of anti-CD9 antibody-anti-CD9 antibody) to obtain (A)/(B) (corrected test specimen measurement value).
Based on the obtained corrected test specimen measurement value, a significant difference test between a patient with mild cognitive impairment and a healthy subject was carried out in the same manner as in Example 1 (4), and the AUC and p-value were calculated.
The obtained results are shown in Table 2 which will be given later. In addition, Fig. 2 shows a box plot graph created based on the corrected test specimen measurement value together with the results of Example 4 and Example 7. In the figure, the vertical axis shows the corrected test specimen measurement value ((A)/(B)), and Control on the horizontal axis shows the results of a healthy subject, MCI on the horizontal axis shows the results of a patient with mild cognitive impairment, and AD on the horizontal axis shows the results of a patient with Alzheimer's disease.

Table 2

| | Specimen | Solid phase | Detection | Test specimen measurement value used for analysis | Healthy subject-MCI | |
|---|---|---|---|---|---|---|
| | | | | | AUC | p-value |
| Comparative Example 4 | Cerebrospinal fluid (CSF) | Tim4 | αCD9 | Value (A) | 0.694 | 0.250 |
| Comparative Example 5 | | αCD9 | αCD9 | Value (B) | 0.408 | 0.609 |
| Example 6 | | | | Value (A)/Value (B) | 0.857 | 0.030 |

Comparative Example 6. Evaluation of Alzheimer's disease test specimen and mild cognitive impairment test specimen using amount of exosome having PS and CD9 as indicator

Exosomes were measured by sandwich ELISA of Tim protein-anti-CD9 antibody in the same manner as in Example 1, except that the cerebrospinal fluid from 7 test specimens of a patient with mild cognitive impairment (MCI) purchased from PrecisionMed, LLC. was used instead of the test specimen of a healthy subject. Then, a significant difference test between a patient with Alzheimer's disease and a patient with mild cognitive impairment was carried out, and the AUC and p-value were calculated.

The obtained results are shown in Table 3 which will be given later.

Comparative Example 7. Evaluation of Alzheimer's disease test specimen and mild cognitive impairment test specimen using amount of exosome having CD9 as indicator

Exosomes were measured by sandwich ELISA of anti-CD9 antibody-anti-CD9 antibody in the same manner as in Comparative Example 1, except that the cerebrospinal fluid from 7 test specimens of a patient with mild cognitive impairment (MCI) purchased from PrecisionMed, LLC. was used instead of the test specimen of a healthy subject. Then, a significant difference test between a patient with Alzheimer's disease and a patient with mild cognitive impairment was carried out, and the AUC and p-value were calculated.

The obtained results are shown in Table 3 which will be given later.

Example 7. Evaluation of Alzheimer's disease test specimen and mild cognitive impairment test specimen using ratio of amount of exosomes having phosphatidylserine and CD9 to amount of exosomes having CD9 as indicator

(A) the "test specimen measurement value" obtained in Comparative Example 6 (sandwich ELISA of Tim protein-anti-CD9 antibody) was divided by (B) the "test specimen measurement value" obtained in Comparative Example 7 (sandwich ELISA of anti-CD9 antibody-anti-CD9 antibody) to obtain (A)/(B) (corrected test specimen measurement value).

Based on the obtained corrected test specimen measurement value, a significant difference test between a patient with Alzheimer's disease and a patient with mild cognitive impairment was carried out in the same manner as in Example 1 (4), and the AUC and p-value were calculated.

The obtained results are shown in Table 3 which will be given later. In addition, Fig. 2 shows a box plot graph created based on the corrected test specimen measurement value together with the results of Example 4 and Example 6. In the figure, the vertical axis shows the corrected test specimen measurement value ((A)/(B)), and Control on the horizontal axis shows the results of a healthy subject, MCI on the horizontal axis shows the results of a patient with mild cognitive impairment, and AD on the horizontal axis shows the results of a patient with Alzheimer's disease.

Table 3

| | Specimen | Solid phase | Detection | Test specimen measurement value used for analysis | MCI-AD | |
| --- | --- | --- | --- | --- | --- | --- |
| | | | | | AUC | p-value |
| Comparative Example 6 | Cerebrospinal fluid (CSF) | Tim4 | $\alpha$CD9 | Value (A) | 0.625 | 0.452 |
| Comparative Example 7 | | $\alpha$CD9 | $\alpha$CD9 | Value (B) | 0.411 | 0.603 |
| Example 7 | | | | Value (A)/Value (B) | 0.875 | 0.018 |

From Table 1, in a case where Alzheimer's disease was evaluated using CSFs derived from a patient with Alzheimer's disease and a healthy subject as test specimens and using the amount of exosomes having tetraspanin of CD9, CD63, or CD81 as an indicator, the AUCs were 0.589, 0.741, and 0.714. In addition, in a case where a correlation coefficient R between the Mini-Mental State Examination (MMSE), which is a test method for Alzheimer's disease, and the amount of exosomes having CD9 was calculated, the obtained correlation coefficient R was 0.048, which is less than 0.2 that is determined to have a correlation, and therefore no correlation was observed.

On the other hand, in a case where Alzheimer's disease was evaluated using CSFs derived from a patient with Alzheimer's disease and a healthy subject as test specimens and using the amount of exosomes having phosphatidylserine to which tetraspanin of CD9, CD63, or CD81 and Tim protein bind as an indicator, the AUCs were 0.839, 0.839, and 0.857, and therefore it was found that Alzheimer's disease can be detected with high accuracy in any of those cases. In addition, in a case where the correlation coefficient R between the MMSE and the amount of exosomes having phosphatidylserine and CD9 was calculated, the obtained correlation coefficient R was 0.363, which is greater than 0.2 that is determined to have a correlation, and therefore there was a correlation.

In addition, in a case where Alzheimer's disease was evaluated using the corrected test specimen measurement value ((A)/(B)) obtained by dividing the test specimen measurement value (A) of the exosome having phosphatidylserine and CD9 by the test specimen measurement value (B) of the exosome having CD9 as an indicator, the AUC was 0.964, and therefore it was found that Alzheimer's disease can be detected with extremely high accuracy.

Further, from Table 2, in a case where mild cognitive impairment was evaluated using CSFs derived from a patient with mild cognitive impairment and a healthy subject as test specimens and using the corrected test specimen measurement value ((A)/(B)) obtained by dividing the test specimen measurement value (A) of the exosome having phosphatidylserine and CD9 by the test specimen measurement value (B) of the exosome having CD9 as an indicator, the AUC was 0.857, and therefore it was found that mild cognitive impairment can be detected with high accuracy. In addition, from Table 3, in a case where Alzheimer's disease or mild cognitive impairment was evaluated using CSFs of a patient with Alzheimer's disease and a patient with mild cognitive impairment as test specimens and using the corrected test specimen measurement value as an indicator, the AUC was 0.875, and therefore it was found that Alzheimer's disease and mild cognitive impairment can be distinguished and detected with high accuracy.

In addition, the correlation coefficient R between the MMSE and the corrected test specimen measurement value ((A)/(B)) obtained by dividing the test specimen measurement value (A) of the exosome having phosphatidylserine and CD9 by the test specimen measurement value (B) of the exosome having CD9 was 0.561, and therefore a correlation was observed.

Examples 8 to 10. Evaluation of Alzheimer's disease test specimen using amount of exosome having PS and tetraspanin as indicator

Exosomes were measured by sandwich ELISA of Tim protein-anti-tetraspanin antibody shown in Table 4 which will be given later in the same manner as in Example 1-3, except that 18 test specimens of EDTA plasma of a patient with Alzheimer's disease (AD) purchased from PrecisionMed, LLC. were used instead of the cerebrospinal fluid test specimen of a patient with Alzheimer's disease, and 37 test specimens of EDTA plasma of a healthy subject purchased from PrecisionMed, LLC. were used instead of the cerebrospinal fluid test specimen of a healthy subject. Then, a significant difference test between a patient with Alzheimer's disease and a healthy subject was carried out, and the AUC and p-value were calculated.

The obtained results are shown in Table 4 which will be given later. In addition, Fig. 3 shows a box plot graph created based on the test specimen measurement value. In the figure, the vertical axis shows the test specimen measurement value, and Control on the horizontal axis shows the results of a healthy subject, and AD on the horizontal axis shows the results of a patient with Alzheimer's disease.

Comparative Examples 8 to 10. Evaluation of Alzheimer's disease test specimen using amount of exosome having tetraspanin as indicator

Exosomes were measured by sandwich ELISA of anti-CD9 antibody-anti-CD9 antibody, sandwich ELISA of anti-CD63 antibody-anti-CD63 antibody, and sandwich ELISA of anti-CD81 antibody-anti-CD81 antibody in the same manner as in Comparative Example 1-3, except that 18 test specimens of EDTA plasma of a patient with Alzheimer's disease (AD) purchased from PrecisionMed, LLC. were used instead of the cerebrospinal fluid test specimen of a patient with Alzheimer's disease, and 37 test specimens of EDTA plasma of a healthy subject purchased from PrecisionMed, LLC. were used instead of the cerebrospinal fluid test specimen of a healthy subject. Then, a significant difference test between a patient with Alzheimer's disease and a healthy subject was carried out, and the AUC and p-value were calculated.

The obtained results are shown in Table 4 which will be given later.

Example 11. Evaluation of Alzheimer's disease test specimen using ratio of amount of exosomes having PS and CD9 to amount of exosomes having CD9 as indicator

The "test specimen measurement value" (value (A)) obtained in Example 8 (sandwich ELISA of Tim protein-anti-CD9 antibody) was divided by the "test specimen measurement value" (value (B)) obtained in Comparative Example 8 (sandwich ELISA of anti-CD9 antibody-anti-CD9 antibody) to obtain (A)/(B) (corrected test specimen measurement value).

Based on the obtained corrected test specimen measurement value, a significant difference test between a patient with Alzheimer's disease and a healthy subject was carried out in the same manner as in Example 1 (4), and the AUC and p-value were calculated.

The obtained results are shown in Table 4 which will be given later. In addition, Fig. 4 shows a box plot graph created based on the corrected test specimen measurement value together with the results of Example 13 and Example 14. In the figure, the vertical axis shows the corrected test specimen measurement value ((A)/(B)), and Control on the horizontal axis shows the results of a healthy subject, MCI on the horizontal axis shows the results of a patient with mild cognitive impairment, and AD on the horizontal axis shows the results of a patient with Alzheimer's disease.

Example 12. Evaluation of Alzheimer's disease test specimen and mild cognitive impairment test specimen using ratio of amount of exosomes having PS and CD63 to amount of exosomes having CD63 as indicator

The "test specimen measurement value" (value (A)) obtained in Example 9 (sandwich ELISA of Tim protein-anti-CD63 antibody) was divided by the "test specimen measurement value" (value (B)) obtained in Comparative Example 9 (sandwich ELISA of anti-CD63 antibody-anti-CD63 antibody) to obtain (A)/(B) (corrected test specimen measurement value).

Based on the obtained corrected test specimen measurement value, a significant difference test between a patient with Alzheimer's disease and a healthy subject was carried out in the same manner as in Example 1 (4), and the AUC and p-value were calculated.

The obtained results are shown in Table 4 which will be given later.

Table 4

| | Specimen | Solid phase | Detection | Test specimen measurement value used for analysis | Healthy subject-AD | |
|---|---|---|---|---|---|---|
| | | | | | AUC | p-value |
| Example 8 | Plasma | Tim4 | αCD9 | Value (A) | 0.775 | 0.001 |
| Comparative Example 8 | | αCD9 | αCD9 | Value (B) | 0.671 | 0.042 |
| Example 11 | | | | Value (A)/Value (B) | 0.871 | <0.0001 |
| Example 9 | | Tim4 | αCD63 | Value (A) | 0.704 | 0.015 |
| Comparative Example 9 | | αCD63 | αCD63 | Value (B) | 0.485 | 0.352 |
| Example 12 | | | | Value (A)/Value (B) | 0.734 | 0.005 |
| Example 10 | | Tim4 | αCD81 | Value (A) | 0.711 | 0.012 |
| Comparative Example 10 | | αCD81 | αCD81 | Value (B) | 0.554 | 0.524 |

Comparative Examples 11 and 12. Evaluation of mild cognitive impairment test specimen using amount of exosome having PS and tetraspanin as indicator

Exosomes were measured by sandwich ELISA of Tim protein-anti-tetraspanin antibody shown in Table 5 which will be given later in the same manner as in Example 1, except that 18 test specimens of EDTA plasma of a patient with mild dementia (MCI) purchased from PrecisionMed, LLC. were used instead of the cerebrospinal fluid test specimen of a patient with Alzheimer's disease (AD), and 37 test specimens of EDTA plasma of a healthy subject purchased from PrecisionMed, LLC. were used as the cerebrospinal fluid test specimen of a healthy subject. Then, a significant difference test between a patient with mild dementia and a healthy subject was carried out, and the AUC and p-value were calculated.

The obtained results are shown in Table 5 which will be given later.

Comparative Examples 13 and 14. Evaluation of mild cognitive impairment test specimen using amount of exosome having PS and tetraspanin as indicator

Exosomes were measured by sandwich ELISA of anti-tetraspanin antibody-anti-tetraspanin antibody shown in Table 5 which will be given later in the same manner as in Comparative Example 1, except that 18 test specimens of EDTA plasma of a patient with mild dementia (MCI) purchased from PrecisionMed, LLC. were used instead of the cerebrospinal fluid test specimen of a patient with Alzheimer's disease (AD), and 37 test specimens of EDTA plasma of a healthy subject purchased from PrecisionMed, LLC. were used as the cerebrospinal fluid test specimen of a healthy subject. Then, a significant difference test between a patient with mild dementia and a healthy subject was carried out, and the AUC and p-value were calculated.

The obtained results are shown in Table 5 which will be given later.

Example 13. Evaluation of mild cognitive impairment test specimen using ratio of amount of exosomes having PS and CD9 to amount of exosomes having CD9 as indicator

The "test specimen measurement value" (value (A)) obtained in Comparative Example 11 (sandwich ELISA of Tim protein-anti-CD9 antibody) was divided by the "test specimen measurement value" (value (B)) obtained in Comparative Example 13 (sandwich ELISA of anti-CD9 antibody-anti-CD9 antibody) to obtain (A)/(B) (corrected test specimen measurement value).

Based on the obtained corrected test specimen measurement value, a significant difference test between a patient with mild dementia and a healthy subject was carried out in the same manner as in Example 1 (4), and the AUC and p-value were calculated.

The obtained results are shown in Table 5 which will be given later. In addition, Fig. 4 shows a box plot graph created based on the corrected test specimen measurement value together with the results of Example 11 and Example 14. In the figure, the vertical axis shows the corrected test specimen measurement value ((A)/(B)), and Control on the horizontal axis shows the results of a healthy subject, MCI on the horizontal axis shows the results of a patient with mild cognitive impairment, and AD on the horizontal axis shows the results of a patient with Alzheimer's disease.

Table 5

| | Specimen | Solid phase | Detection | Test specimen measurement value used for analysis | Healthy subject-MCI | |
|---|---|---|---|---|---|---|
| | | | | | AUC | p-value |
| Comparative Example 11 | Plasma | Tim4 | αCD9 | Value (A) | 0.661 | 0.046 |
| Comparative Example 13 | | αCD9 | αCD9 | Value (B) | 0.615 | 0.438 |
| Example 13 | | | | Value (A)/Value (B) | 0.766 | 0.014 |
| Comparative Example 12 | | Tim4 | αCD81 | Value (A) | 0.598 | 0.283 |
| Comparative Example 14 | | αCD81 | αCD81 | Value (B) | 0.544 | 0.458 |

Comparative Examples 15 and 16. Evaluation of Alzheimer's disease test specimen and mild cognitive impairment test specimen using amount of exosome having PS and tetraspanin as indicator

Exosomes were measured by sandwich ELISA of Tim protein-anti-tetraspanin antibody in the same manner as in Example 1, except that 18 test specimens of EDTA plasma of a patient with Alzheimer's disease (AD) purchased from PrecisionMed, LLC. were used instead of the cerebrospinal fluid test specimen of a patient with Alzheimer's disease (AD), and 18 test specimens of EDTA plasma of a patient with mild cognitive impairment (MCI) purchased from PrecisionMed, LLC. were used instead of the test specimen of a healthy subject. Then, a significant difference test between a patient with Alzheimer's disease and a patient with mild dementia was carried out, and the AUC and p-value were calculated.

The obtained results are shown in Table 6 which will be given later.

Comparative Examples 17 and 18. Evaluation of mild cognitive impairment test specimen using amount of exosome having tetraspanin as indicator

Exosomes were measured by sandwich ELISA of anti-tetraspanin antibody-anti-tetraspanin antibody shown in Table 6 which will be given later in the same manner as in Comparative Example 1, except that 18 test specimens of EDTA plasma of a patient with Alzheimer's disease (AD) purchased from PrecisionMed, LLC. were used instead of the cerebrospinal fluid test specimen of a patient with Alzheimer's disease (AD), and the cerebrospinal fluid from 18 test specimens of a patient with mild cognitive impairment (MCI) purchased from PrecisionMed, LLC. was used instead of the test specimen of a healthy subject. Then, a significant difference test between a patient with Alzheimer's disease and a patient with mild dementia was carried out, and the AUC and p-value were calculated.

The obtained results are shown in Table 6 which will be given later.

Example 14. Evaluation of Alzheimer's disease test specimen and mild cognitive impairment test specimen using ratio of amount of exosomes having PS and CD9 to amount of exosomes having CD9 as indicator

The "test specimen measurement value" (value (A)) of a patient with Alzheimer's disease obtained in Comparative Example 15 (sandwich ELISA of Tim protein-anti-CD9 antibody) was divided by the "test specimen measurement value" (value (B)) of a patient with Alzheimer's disease obtained in Comparative Example 17 (sandwich ELISA of anti-CD9 antibody-anti-CD9 antibody) to obtain (A)/(B) (corrected test specimen measurement value).

Based on the obtained corrected test specimen measurement value, a significant difference test between a patient with Alzheimer's disease and a patient with mild dementia was carried out in the same manner as in Example 1 (4), and the AUC and p-value were calculated.

The obtained results are shown in Table 6 which will be given later. In addition, Fig. 4 shows a box plot graph created based on the corrected test specimen measurement value together with the results of Example 11 and Example 13. In the figure, the vertical axis shows the corrected test specimen measurement value ((A)/(B)), and Control on the horizontal axis shows the results of a healthy subject, MCI on the horizontal axis shows the results of a patient with mild cognitive impairment, and AD on the horizontal axis shows the results of a patient with Alzheimer's disease.

Table 6

| | Specimen | Solid phase | Detection | Test specimen measurement value used for analysis | MCI-AD | |
|---|---|---|---|---|---|---|
| | | | | | AUC | p-value |
| Comparative Example 15 | Plasma | Tim4 | αCD9 | Value (A) | 0.699 | 0.060 |
| Comparative Example 17 | | αCD9 | αCD9 | Value (B) | 0.578 | 0.180 |
| Example 14 | | | | Value (A)/Value (B) | 0.745 | 0.002 |
| Comparative Example 16 | | Tim4 | αCD81 | Value (A) | 0.608 | 0.256 |
| Comparative Example 18 | | αCD81 | αCD81 | Value (B) | 0.487 | 0.865 |

From Table 4, in a case where Alzheimer's disease was evaluated using plasma derived from a patient with Alzheimer's disease and a healthy subject as the test specimen and using the amount of exosomes having tetraspanin of CD9, CD63, or CD81 as an indicator, the AUCs were 0.671, 0.485, and 0.554. In addition, in a case where the correlation coefficient R between the MMSE, which is a test method for Alzheimer's disease, and the amount of exosomes having CD9 was calculated, the obtained correlation coefficient R was 0.206, and therefore almost no correlation was observed.

On the other hand, in a case where Alzheimer's disease was evaluated using plasma derived from a patient with Alzheimer's disease and a healthy subject as the test specimen and using the amount of exosomes having phosphatidylserine to which tetraspanin of CD9, CD63, or CD81 and Tim protein bind as an indicator, the AUCs were 0.775, 0.704, and 0.711, all of which are greater than 0.7, and therefore it was found that Alzheimer's disease can be detected. In addition, in a case where the correlation coefficient R between the MMSE and the amount of exosomes having phosphatidylserine and CD9 was calculated, the obtained correlation coefficient R was 0.326, which is greater than 0.2 that is determined to have a correlation, and therefore there was a correlation.

In addition, in a case where the corrected test specimen measurement value ((A)/(B)) obtained by dividing the test specimen measurement value (A) of the exosome having phosphatidylserine and CD9 by the test specimen measurement value (B) of the exosome having CD9 was used, the AUC was 0.871, and therefore it was found that Alzheimer's disease can be detected with high accuracy.

Further, from Table 5, in a case where mild cognitive impairment was evaluated using plasma derived from a patient with mild cognitive impairment and a healthy subject as the test specimen and using the corrected test specimen measurement value ((A)/(B)) obtained by dividing the test specimen measurement value (A) of the exosome having phosphatidylserine and CD9 by the test specimen measurement value (B) of the exosome having CD9 as an indicator, the AUC was 0.766, and therefore it was found that mild cognitive impairment can be detected. In addition, from Table 6, in a case where Alzheimer's disease or mild cognitive impairment was evaluated using plasma of a patient with Alzheimer's disease and a patient with mild cognitive impairment as the test specimen and using the corrected test specimen measurement value as an indicator, the AUC was 0.745, and therefore it was found that Alzheimer's disease and mild cognitive impairment can be distinguished and detected.

In addition, the correlation coefficient R between the MMSE and the corrected test specimen measurement value obtained by dividing the test specimen measurement value (A) of the exosome having phosphatidylserine and CD9 by the test specimen measurement value (B) of the exosome having CD9 was 0.635, and therefore a correlation was observed.

Any exosome having phosphatidylserine and tetraspanin was capable of detecting Alzheimer's disease or/and mild cognitive impairment and in particular, according to an exosome having phosphatidylserine and CD9, Alzheimer's disease could be detected with high accuracy regardless of the type of specimen.

Comparative Example 19. Evaluation of Alzheimer's disease test specimen using amount of amyloid β (1-40) as indicator

**(1) Preparation of calibrator**

The standard solution (human β amyloid (1-40), 100 pmol/L) attached to "Human β Amyloid (1-40) ELISA Kit Wako II" (manufactured by FUJIFILM Wako Pure Chemical Corporation, hereinafter referred to as "C kit") was diluted with the standard diluting solution attached to the C kit to 1.0, 2.5, 5.0, 10.0, 25.0, 50.0, and 100.0 pmol/L to thereby obtain a standard dilution series consisting of 7-point concentrations (hereinafter, referred to as "calibrator").

EP 4 067 906 B1

## (2) Preparation of test specimen for measurement

18 test specimens of EDTA plasma of patients with Alzheimer's disease (AD), 18 test specimens of EDTA plasma of patients with mild dementia (MCI), and 37 test specimens of EDTA plasma of healthy subjects purchased from PrecisionMed, LLC. were diluted 4.44-fold with the standard diluting solution attached to the C kit to obtain "test specimen diluted solutions for measurement".

## (3) Measurement by ELISA

The test specimen diluted solutions for measurement prepared in (2) were measured using the C kit. Specifically, first, a solution obtained by diluting the washing solution ($20\times$) attached to the C kit 20-fold with purified water (distilled water) was prepared and used as a "washing solution ($1\times$)". Next, the calibrator prepared in (1), the test specimen diluted solution for measurement prepared in (2), and the standard diluting solution attached to the C kit as a blank were dispensed at an amount of 95 $\mu$L/well (n = 2) into each well of the antibody (BAN50)-immobilized microplate attached to the C kit. Next, a plate seal was attached to the plate, followed by reaction overnight in a refrigerator. After the reaction was completed, the reaction solution was discarded and each well was washed 4 times with 300 to 350 $\mu$L of the washing solution ($1\times$).
Next, the HRP-labeled antibody (BA27) solution attached to the C kit was dispensed at an amount of 100 $\mu$L/well into each well of the plate, and a plate seal was attached to the plate, followed by reaction for 2 hours in a refrigerator. After the reaction was completed, the reaction solution was discarded and each well was washed 5 times with 300 to 350 $\mu$L of the washing solution ($1\times$).
Next, the 3,3',5,5'-tetramethylbenzidine (TMB) solution attached to the C kit, which was returned to room temperature, was dispensed at an amount of 100 $\mu$L/well into each well of the plate, followed by stirring for about 1 minute using a microplate shaker. After that, a plate seal was attached to the plate which was then allowed to stand at room temperature (20°C to 25°C) for 30 minutes. Then, the stop solution attached to the C kit, which was returned to room temperature, was dispensed at an amount of 100 $\mu$L/well into each well of the plate, followed by stirring for about 5 seconds using a microplate shaker, and the absorbance at 450 nm and the absorbance at a sub-wavelength of 620 nm were immediately measured using a 96-well microplate reader (SPARK, available from Tecan Group Ltd.). The value obtained by subtracting the absorbance value at a sub-wavelength of 620 nm from the absorbance value at 450 nm was defined as an "absorbance value", and the value obtained by subtracting the blank absorbance value from the absorbance value of the test specimen diluted solution for measurement was defined as a "corrected test specimen absorbance value". Then, a standard curve was created from the value obtained by subtracting the blank absorbance value from the absorbance value of the standard dilution series (calibrator) and the concentration of the calibrator. The corrected test specimen absorbance value was converted into a protein concentration using the standard curve, and the value obtained by multiplying the obtained corresponding value by the test specimen dilution rate was defined as a "test specimen measurement value" [pmol/L].

## (4) Calculation of AUC

Based on the test specimen measurement value obtained in (3), a significant difference test was carried out between a patient with Alzheimer's disease and a healthy subject, between a patient with Alzheimer's disease and a patient with mild dementia, and between a patient with mild dementia and a healthy subject, in the same manner as in Example 1, and the AUC and p-value were calculated.
The obtained results are shown in Table 7 which will be given later.

Comparative Example 20. Evaluation of Alzheimer's disease test specimen using amount of amyloid $\beta$ (1-42) as indicator

## (1) Preparation of calibrator

The standard solution (human $\beta$ amyloid (1-42), 20 pmol/L) attached to "Human $\beta$ Amyloid (1-42) ELISA Kit Wako, High-Sensitive" (manufactured by FUJIFILM Wako Pure Chemical Corporation, hereinafter referred to as "D kit") was diluted with the standard diluting solution attached to the D kit to 0.1, 0.5, 1.0, 2.0, 5.0, 10.0, and 20.0 pmol/L to thereby obtain a standard dilution series consisting of 7-point concentrations (hereinafter, referred to as "calibrator").

## (2) Preparation of test specimen for measurement

18 test specimens of EDTA plasma of patients with Alzheimer's disease (AD), 18 test specimens of EDTA plasma of patients with mild dementia (MCI), and 37 test specimens of EDTA plasma of healthy subjects purchased from PrecisionMed, LLC. were diluted 4.44-fold with the standard diluting solution attached to the D kit to obtain "test specimen diluted solutions for measurement".

**(3) Measurement by ELISA**

The test specimen diluted solutions for measurement prepared in (2) were measured using the D kit. Specifically, first, a solution obtained by diluting the washing solution (20×) attached to the D kit 20-fold with purified water (distilled water) was prepared and used as a "washing solution (1×)". Next, the calibrator prepared in (1), the test specimen diluted solution for measurement prepared in (2), and the standard diluting solution attached to the D kit as a blank were dispensed at an amount of 95 μL/well (n = 2) into each well of the antibody (BAN50)-immobilized microplate attached to the D kit. Next, a plate seal was attached to the plate, followed by reaction overnight in a refrigerator. After the reaction was completed, the reaction solution was discarded and each well was washed 4 times with 300 to 350 μL of the washing solution (1×).
Next, the HRP-labeled antibody (BC05) solution attached to the D kit was dispensed at an amount of 100 μL/well into each well of the plate, and a plate seal was attached to the plate, followed by reaction for 2 hours in a refrigerator. After the reaction was completed, the reaction solution was discarded and each well was washed 5 times with 300 to 350 μL of the washing solution (1×).
Next, the 3,3',5,5'-tetramethylbenzidine (TMB) solution attached to the D kit, which was returned to room temperature, was dispensed at an amount of 100 μL/well into each well of the plate, followed by stirring for about 1 minute using a microplate shaker. After that, a plate seal was attached to the plate which was then allowed to stand at room temperature (20°C to 25°C) for 30 minutes. Then, the stop solution attached to the D kit, which was returned to room temperature, was dispensed at an amount of 100 μL/well into each well of the plate, followed by stirring for about 5 seconds using a microplate shaker, and the absorbance at 450 nm and the absorbance at a sub-wavelength of 620 nm were immediately measured using a 96-well microplate reader (SPARK, available from Tecan Group Ltd.). The value obtained by subtracting the absorbance value at a sub-wavelength of 620 nm from the absorbance value at 450 nm was defined as an "absorbance value", and the value obtained by subtracting the blank absorbance value from the absorbance value of the test specimen diluted solution for measurement was defined as a "corrected test specimen absorbance value". Then, a standard curve was created from the value obtained by subtracting the blank absorbance value from the absorbance value of the standard dilution series (calibrator) and the concentration of the calibrator. The corrected test specimen absorbance value was converted into a protein concentration using the standard curve, and the value obtained by multiplying the obtained corresponding value by the test specimen dilution rate was defined as a "test specimen measurement value" [pmol/L].

**(4) Calculation of AUC**

Based on the test specimen measurement value obtained in (3), a significant difference test was carried out between a patient with Alzheimer's disease and a healthy subject, between a patient with Alzheimer's disease and a patient with mild dementia, and between a patient with mild dementia and a healthy subject, in the same manner as in Example 1, and the AUC and p-value were calculated.
The obtained results are shown in Table 7 which will be given later.

Comparative Example 21. Evaluation of Alzheimer's disease test specimen using ratio of amount of amyloid β (1-42) to amount of amyloid β (1-40) as indicator

The "test specimen measurement value" (value (D)) of amyloid β (1-42) obtained in Comparative Example 19 was divided by the "test specimen measurement value" (value (C)) of amyloid β (1-40) obtained in Comparative Example 20 to obtain (D)/(C) (hereinafter referred to as "corrected test specimen measurement value").
Based on the obtained corrected test specimen measurement value, a significant difference test was carried out between a patient with Alzheimer's disease and a healthy subject, between a patient with Alzheimer's disease and a patient with mild dementia, and between a patient with mild dementia and a healthy subject, in the same manner as in Example 1, and the AUC and p-value were calculated.
The obtained results are shown in Table 7 which will be given later.
Example 15. Evaluation of Alzheimer's disease test specimen using value obtained by multiplying ratio of amount of exosomes having PS and CD9 to amount of exosomes having CD9 by ratio of amount of amyloid β (1-42) to amount of amyloid β (1-40) as indicator
The product calculated by multiplying the ratio of the amount of exosomes having PS and CD9 to the amount of exosomes having CD9 (corrected test specimen measurement value ((A)/(B)) obtained in Example 11 by the ratio of the amount of amyloid β (1-42) to the amount of amyloid β (1-40) (corrected test specimen measurement value ((D)/(C)) obtained in Comparative Example 21 was obtained.

51

Based on the value of the calculated product, a significant difference test was carried out between a patient with Alzheimer's disease and a healthy subject, between a patient with Alzheimer's disease and a patient with mild dementia, and between a patient with mild dementia and a healthy subject, in the same manner as in Example 1, and the AUC and p-value were calculated.

The obtained results are shown in Table 7 which will be given later. In addition, Fig. 5 shows a box plot graph created based on the corrected test specimen measurement value. In the figure, the vertical axis shows the corrected test specimen measurement value ((A)/(B)) of Example 11 × the corrected test specimen measurement value ((D)/(C)) of Comparative Example 21, and Control on the horizontal axis shows the results of a healthy subject, MCI on the horizontal axis shows the results of a patient with mild cognitive impairment, and AD on the horizontal axis shows the results of a patient with Alzheimer's disease.

Table 7

| | Specimen | Measurement target | Test specimen measurement value used for analysis | Healthy subject-AD | | Healthy subject-MCI | | MCI-AD | |
|---|---|---|---|---|---|---|---|---|---|
| | | | | AUC | p-value | AUC | p-value | AUC | p-value |
| Comparative Example 19 | | Aβ (1-40) | Value (C) | 0.536 | 0.6734 | 0.528 | 0.7515 | 0.497 | 0.9868 |
| Comparative Example 20 | | Aβ (1-42) | Value (D) | 0.781 | 0.0008 | 0.730 | 0.0073 | 0.611 | 0.2689 |
| Comparative Example 21 | Plasma | | Value (D)/Value (C) | 0.878 | <0.0001 | 0.824 | 0.0002 | 0.686 | 0.0622 |
| Example 15 [Value (A)/Value (B)] × | | | \|Value (D)/Value (C)\| | 0.923 | <0.0001 | 0.833 | <0.0001 | 0.804 | 0.0023 |

From Table 7, in a case where Alzheimer's disease and mild dementia were evaluated using plasma derived from a patient with Alzheimer's disease, a patient with mild dementia, and a healthy subject as the test specimen and using the corrected test specimen measurement value (D)/(C) of Comparative Example 21 as an indicator, the AUC was 0.878 between the healthy subject and the patient with Alzheimer's disease, 0.824 between the healthy subject and the patient with mild dementia, and 0.686 between the patient with Alzheimer's disease and the patient with mild dementia.

In addition, in a case where the correlation coefficient R between the MMSE, which is a test method for Alzheimer's disease, and the corrected test specimen measurement value (A)/(B) was calculated, the obtained correlation coefficient R was 0.5781.

From Table 7, in a case where Alzheimer's disease and mild dementia were evaluated using the value obtained by multiplying the corrected test specimen measurement value ((A)/(B)) of Example 11 by the corrected test specimen measurement value ((D)/(C)) of Comparative Example 21 as an indicator, the AUC was 0.923 between the healthy subject and the patient with Alzheimer's disease, 0.833 between the healthy subject and the patient with mild dementia, and 0.804 between the patient with Alzheimer's disease and the patient with mild dementia.

In addition, in a case where the correlation coefficient R between the MMSE, which is a test method for Alzheimer's disease, and the corrected test specimen measurement value (A)/(B) was calculated, the obtained correlation coefficient R was 0.7023.

Alzheimer's disease or/and mild cognitive impairment could be detected with higher accuracy by using the value obtained by multiplying the ratio of the amount of exosomes having PS and CD9 to the amount of exosomes having CD9 (corrected test specimen measurement value ((A)/(B)) of Example 11) by the ratio of the amount of amyloid $\beta$ (1-42) to the amount of amyloid $\beta$ (1-40) (corrected test specimen measurement value ((D)/(C)) of Comparative Example 21) as an indicator. In addition, it was found that using this value makes it possible to distinguish and detect particularly a healthy subject and a patient with Alzheimer's disease, and a patient with Alzheimer's disease and a patient with mild dementia with higher accuracy.

**Claims**

1. A method for assisting the diagnosis of Alzheimer's disease, comprising:

   measuring an amount of extracellular vesicles having phosphatidylserine and tetraspanin, or the amount of extracellular vesicles having phosphatidylserine and tetraspanin and an amount of extracellular vesicles having tetraspanin, in a biological specimen derived from a subject; and
   determining that the subject has Alzheimer's disease using the amount of extracellular vesicles having phosphatidylserine and tetraspanin, or
   a ratio of the amount of extracellular vesicles having phosphatidylserine and tetraspanin to the amount of extracellular vesicles having tetraspanin as an indicator.

2. The method for assisting the diagnosis of Alzheimer's disease according to claim 1,
   wherein the determination of Alzheimer's disease is carried out in such a manner that the subject is determined to have Alzheimer's disease in a case where the amount of extracellular vesicles having phosphatidylserine and tetraspanin, or the ratio of the amount of extracellular vesicles having phosphatidylserine and tetraspanin to the amount of extracellular vesicles having tetraspanin is equal to or less than a reference value.

3. The method for assisting the diagnosis of Alzheimer's disease according to claim 1,

   wherein the measurement of the amount of extracellular vesicles is to measure the amount of extracellular vesicles having phosphatidylserine and tetraspanin,
   the determination of Alzheimer's disease is to determine that the subject has Alzheimer's disease using the amount of extracellular vesicles having phosphatidylserine and tetraspanin as an indicator, and
   the tetraspanin is selected from CD9, CD63, and CD81.

4. The method for assisting the diagnosis of Alzheimer's disease according to claim 1,

   wherein the measurement of the amount of extracellular vesicles is to measure the amount of extracellular vesicles having phosphatidylserine and tetraspanin,
   the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine,

the determination of Alzheimer's disease is to determine that the subject has Alzheimer's disease using the amount of extracellular vesicles having phosphatidylserine and tetraspanin as an indicator.

5. The method for assisting the diagnosis of Alzheimer's disease according to claim 4, wherein the substance having an affinity for phosphatidylserine is a T-cell immunoglobulin-mucin-containing protein.

6. The method for assisting the diagnosis of Alzheimer's disease according to claim 1,

wherein the measurement of the amount of extracellular vesicles is to measure the amount of extracellular vesicles having phosphatidylserine and tetraspanin,
the determination of Alzheimer's disease is to determine that the subject has Alzheimer's disease using the amount of extracellular vesicles having phosphatidylserine and tetraspanin as an indicator, and
the biological specimen is a blood specimen or a cerebrospinal fluid.

7. The method for assisting the diagnosis of Alzheimer's disease according to claim 1,

wherein the measurement of the amount of extracellular vesicles is to measure the amount of extracellular vesicles having phosphatidylserine and tetraspanin, and the amount of extracellular vesicles having tetraspanin,
the determination of Alzheimer's disease is to determine that the subject has Alzheimer's disease using the ratio of the amount of extracellular vesicles having phosphatidylserine and tetraspanin to the amount of extracellular vesicles having tetraspanin as an indicator, and
the tetraspanin is CD9 or CD63.

8. The method for assisting the diagnosis of Alzheimer's disease according to claim 1,

wherein the measurement of the amount of extracellular vesicles is to measure the amount of extracellular vesicles having phosphatidylserine and tetraspanin, and the amount of extracellular vesicles having tetraspanin,
the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine,
the measurement of the amount of extracellular vesicles having tetraspanin is to measure the amount of extracellular vesicles having tetraspanin using the substance having an affinity for tetraspanin, and
the determination of Alzheimer's disease is to determine that the subject has Alzheimer's disease using the ratio of the amount of extracellular vesicles having phosphatidylserine and tetraspanin to the amount of extracellular vesicles having tetraspanin as an indicator.

9. The method for assisting the diagnosis of Alzheimer's disease according to claim 8, wherein the substance having an affinity for phosphatidylserine is a T-cell immunoglobulin-mucin-containing protein.

10. The method for assisting the diagnosis of Alzheimer's disease according to claim 1,

wherein the measurement of the amount of extracellular vesicles is to measure the amount of extracellular vesicles having phosphatidylserine and tetraspanin, and the amount of extracellular vesicles having tetraspanin,
the determination of Alzheimer's disease is to determine that the subject has Alzheimer's disease using the ratio of the amount of extracellular vesicles having phosphatidylserine and tetraspanin to the amount of extracellular vesicles having tetraspanin as an indicator, and
the biological specimen is a blood specimen or a cerebrospinal fluid.

11. A method for assisting the diagnosis of Alzheimer's disease or mild cognitive impairment, comprising:

measuring an amount of extracellular vesicles having phosphatidylserine and CD9 and an amount of extracellular vesicles having CD9 in a biological specimen derived from a subject; and
determining that the subject has Alzheimer's disease or mild cognitive impairment using a ratio of the amount of extracellular vesicles having phosphatidylserine and CD9 to the amount of extracellular vesicles having CD9 as an indicator.

12. The method for assisting the diagnosis of Alzheimer's disease or mild cognitive impairment according to claim 11, wherein the determination of Alzheimer's disease is to determine that the subject has Alzheimer's disease or mild cognitive impairment in a case where the ratio of the amount of extracellular vesicles having phosphatidylserine and CD9 to the amount of extracellular vesicles having CD9 is equal to or less than a reference value.

13. The method for assisting the diagnosis of Alzheimer's disease or mild cognitive impairment according to claim 11,

wherein the measurement of the amount of extracellular vesicles having phosphatidylserine and CD9 is to measure the amount of extracellular vesicles having phosphatidylserine and CD9 using a substance having an affinity for CD9 and a substance having an affinity for phosphatidylserine, and
the measurement of the amount of extracellular vesicles having CD9 is to measure the amount of extracellular vesicles having CD9 using the substance having an affinity for CD9.

14. The method for assisting the diagnosis of Alzheimer's disease or mild cognitive impairment according to claim 13, wherein the substance having an affinity for phosphatidylserine is a T-cell immunoglobulin-mucin-containing protein.

15. The method for assisting the diagnosis of Alzheimer's disease or mild cognitive impairment according to claim 11, wherein the biological specimen is a blood specimen or a cerebrospinal fluid.

16. *In vitro* use of a reagent kit for assisting the diagnosis of Alzheimer's disease, the reagent kit comprising:

a substance having an affinity for tetraspanin; and
a substance having an affinity for phosphatidylserine.

17. *In vitro* use of a reagent kit for assisting the diagnosis of Alzheimer's disease or mild cognitive impairment, the reagent kit comprising:

a substance having an affinity for CD9; and
a substance having an affinity for phosphatidylserine.

18. *In vitro* use of a biomarker for assisting the diagnosis of Alzheimer's disease, the biomarker comprising:
an extracellular vesicle having phosphatidylserine and tetraspanin.

19. *In vitro* use of a biomarker set for assisting the diagnosis of Alzheimer's disease or mild cognitive impairment, the biomarker set comprising:

an extracellular vesicle having phosphatidylserine and CD9; and
an extracellular vesicle having CD9.

**Patentansprüche**

1. Verfahren zur Unterstützung der Diagnose der Alzheimer-Krankheit, umfassend:

Messen einer Menge extrazellulärer Vesikel mit Phosphatidylserin und Tetraspanin oder der Menge extrazellulärer Vesikel mit Phosphatidylserin und Tetraspanin und einer Menge extrazellulärer Vesikel mit Tetraspanin in einer von einem Subjekt stammenden biologischen Probe; und
Bestimmen, dass das Subjekt die Alzheimer-Krankheit hat, unter Verwendung der Menge extrazellulärer Vesikel mit Phosphatidylserin und Tetraspanin oder
eines Verhältnisses der Menge extrazellulärer Vesikel mit Phosphatidylserin und Tetraspanin zu der Menge extrazellulärer Vesikel mit Tetraspanin als Indikator.

2. Verfahren zur Unterstützung der Diagnose der Alzheimer-Krankheit nach Anspruch 1, wobei die Bestimmung der Alzheimer-Krankheit in der Weise durchgeführt wird, dass bestimmt wird, dass das Subjekt die Alzheimer-Krankheit hat, falls die Menge extrazellulärer Vesikel mit Phosphatidylserin und Tetraspanin oder das Verhältnis der Menge extrazellulärer Vesikel mit Phosphatidylserin und Tetraspanin zu der Menge extrazellulärer Vesikel mit Tetraspanin gleich oder kleiner als ein Referenzwert ist.

3. Verfahren zur Unterstützung der Diagnose der Alzheimer-Krankheit nach Anspruch 1, wobei das Messen der Menge extrazellulärer Vesikel ein Messen der Menge extrazellulärer Vesikel mit Phosphatidylserin und Tetraspanin ist,

die Bestimmung der Alzheimer-Krankheit ein Bestimmen ist, dass das Subjekt die Alzheimer-Krankheit hat, unter Verwendung der Menge extrazellulärer Vesikel mit Phosphatidylserin und Tetraspanin als Indikator, und das Tetraspanin ausgewählt ist aus CD9, CD63 und CD81.

4. Verfahren zur Unterstützung der Diagnose der Alzheimer-Krankheit nach Anspruch 1, wobei das Messen der Menge extrazellulärer Vesikel ein Messen der Menge extrazellulärer Vesikel mit Phosphatidylserin und Tetraspanin ist,

das Messen der Menge extrazellulärer Vesikel mit Phosphatidylserin und Tetraspanin ein Messen der Menge extrazellulärer Vesikel mit Phosphatidylserin und Tetraspanin unter Verwendung einer Substanz mit einer Affinität für Tetraspanin und einer Substanz mit einer Affinität für Phosphatidylserin ist,
die Bestimmung der Alzheimer-Krankheit ein Bestimmen ist, dass das Subjekt die Alzheimer-Krankheit hat, unter Verwendung der Menge extrazellulärer Vesikel mit Phosphatidylserin und Tetraspanin als Indikator.

5. Verfahren zur Unterstützung der Diagnose der Alzheimer-Krankheit nach Anspruch 4, wobei die Substanz mit einer Affinität für Phosphatidylserin ein T-Zell-Immunglobulin-Mucin-enthaltendes Protein ist.

6. Verfahren zur Unterstützung der Diagnose der Alzheimer-Krankheit nach Anspruch 1, wobei das Messen der Menge extrazellulärer Vesikel ein Messen der Menge extrazellulärer Vesikel mit Phosphatidylserin und Tetraspanin ist,

die Bestimmung der Alzheimer-Krankheit ein Bestimmen ist, dass das Subjekt die Alzheimer-Krankheit hat, unter Verwendung der Menge extrazellulärer Vesikel mit Phosphatidylserin und Tetraspanin als Indikator, und die biologische Probe eine Blutprobe oder eine Zerebrospinalflüssigkeit ist.

7. Verfahren zur Unterstützung der Diagnose der Alzheimer-Krankheit nach Anspruch 1,

wobei das Messen der Menge extrazellulärer Vesikel ein Messen der Menge extrazellulärer Vesikel mit Phosphatidylserin und Tetraspanin und der Menge extrazellulärer Vesikel mit Tetraspanin ist, die Bestimmung der Alzheimer-Krankheit ein Bestimmen ist, dass das Subjekt die Alzheimer-Krankheit hat, unter Verwendung des Verhältnisses der Menge extrazellulärer Vesikel mit Phosphatidylserin und Tetraspanin zu der Menge extrazellulärer Vesikel mit Tetraspanin als Indikator, und das Tetraspanin CD9 oder CD63 ist.

8. Verfahren zur Unterstützung der Diagnose der Alzheimer-Krankheit nach Anspruch 1, wobei das Messen der Menge extrazellulärer Vesikel ein Messen der Menge extrazellulärer Vesikel mit Phosphatidylserin und Tetraspanin und der Menge extrazellulärer Vesikel mit Tetraspanin ist, das Messen der Menge extrazellulärer Vesikel mit Phosphatidylserin und Tetraspanin ein Messen der Menge extrazellulärer Vesikel mit Phosphatidylserin und Tetraspanin unter Verwendung einer Substanz mit einer Affinität für Tetraspanin und einer Substanz mit einer Affinität für Phosphatidylserin ist,

das Messen der Menge extrazellulärer Vesikel mit Tetraspanin ein Messen der Menge extrazellulärer Vesikel mit Tetraspanin unter Verwendung der Substanz mit einer Affinität für Tetraspanin ist, und die Bestimmung der Alzheimer-Krankheit ein Bestimmen ist, dass das Subjekt die Alzheimer-Krankheit hat, unter Verwendung des Verhältnisses der Menge extrazellulärer Vesikel mit Phosphatidylserin und Tetraspanin zu der Menge extrazellulärer Vesikel mit Tetraspanin als Indikator.

9. Verfahren zur Unterstützung der Diagnose der Alzheimer-Krankheit nach Anspruch 8, wobei die Substanz mit einer Affinität für Phosphatidylserin ein T-Zell-Immunglobulin-Mucin-enthaltendes Protein ist.

10. Verfahren zur Unterstützung der Diagnose der Alzheimer-Krankheit nach Anspruch 1, wobei das Messen der Menge extrazellulärer Vesikel ein Messen der Menge extrazellulärer Vesikel mit Phosphatidylserin und Tetraspanin und der Menge extrazellulärer Vesikel mit Tetraspanin ist, die Bestimmung der Alzheimer-Krankheit ein Bestimmen ist, dass das Subjekt die Alzheimer-Krankheit hat, unter Verwendung des Verhältnisses der Menge extrazellulärer Vesikel mit Phosphatidylserin und Tetraspanin zu der Menge extrazellulärer Vesikel mit Tetraspanin als Indikator, und die biologische Probe eine Blutprobe oder eine Zerebrospinalflüssigkeit ist.

11. Verfahren zur Unterstützung der Diagnose der Alzheimer-Krankheit oder einer leichten kognitiven Beeinträchtigung, umfassend:

Messen einer Menge extrazellulärer Vesikel mit Phosphatidylserin und CD9 und einer Menge extrazellulärer Vesikel mit CD9 in einer von einem Subjekt stammenden biologischen Probe; und

Bestimmen, dass das Subjekt die Alzheimer-Krankheit oder eine leichte kognitive Beeinträchtigung hat, unter Verwendung eines Verhältnisses der Menge extrazellulärer Vesikel mit Phosphatidylserin und CD9 zu der Menge extrazellulärer Vesikel mit CD9 als Indikator.

12. Verfahren zur Unterstützung der Diagnose der Alzheimer-Krankheit oder einer leichten kognitiven Beeinträchtigung nach Anspruch 11,
wobei die Bestimmung der Alzheimer-Krankheit ein Bestimmen ist, dass das Subjekt die Alzheimer-Krankheit oder eine leichte kognitive Beeinträchtigung hat, falls das Verhältnis der Menge extrazellulärer Vesikel mit Phosphatidylserin und CD9 zu der Menge extrazellulärer Vesikel mit CD9 gleich oder kleiner als ein Referenzwert ist.

13. Verfahren zur Unterstützung der Diagnose der Alzheimer-Krankheit oder einer leichten kognitiven Beeinträchtigung nach Anspruch 11,

wobei das Messen der Menge extrazellulärer Vesikel mit Phosphatidylserin und CD9 ein Messen der Menge extrazellulärer Vesikel mit Phosphatidylserin und CD9 unter Verwendung einer Substanz mit einer Affinität für CD9 und einer Substanz mit einer Affinität für Phosphatidylserin ist, und
das Messen der Menge extrazellulärer Vesikel mit CD9 ein Messen der Menge extrazellulärer Vesikel mit CD9 unter Verwendung der Substanz mit einer Affinität für CD9 ist.

14. Verfahren zur Unterstützung der Diagnose der Alzheimer-Krankheit oder einer leichten kognitiven Beeinträchtigung nach Anspruch 13,
wobei die Substanz mit einer Affinität für Phosphatidylserin ein T-Zell-Immunglobulin-Mucin-enthaltendes Protein ist.

15. Verfahren zur Unterstützung der Diagnose der Alzheimer-Krankheit oder einer leichten kognitiven Beeinträchtigung nach Anspruch 11,
wobei die biologische Probe eine Blutprobe oder eine Zerebrospinalflüssigkeit ist.

16. Verwendung eines Reagenzienkits zur Unterstützung der Diagnose der Alzheimer-Krankheit, wobei das Reagenzienkit umfasst:

eine Substanz mit einer Affinität für Tetraspanin; und
eine Substanz mit einer Affinität für Phosphatidylserin.

17. Verwendung eines Reagenzienkits zur Unterstützung der Diagnose der Alzheimer-Krankheit oder einer leichten kognitiven Beeinträchtigung, wobei das Reagenzienkit umfasst:

eine Substanz mit einer Affinität für CD9; und
eine Substanz mit einer Affinität für Phosphatidylserin.

18. Verwendung eines Biomarkers zur Unterstützung der Diagnose der Alzheimer-Krankheit, wobei der Biomarker umfasst: ein extrazelluläres Vesikel mit Phosphatidylserin und Tetraspanin.

19. Verwendung eines Biomarkersatzes zur Unterstützung der Diagnose der Alzheimer-Krankheit oder einer leichten kognitiven Beeinträchtigung, wobei der Biomarkersatz umfasst: ein extrazelluläres Vesikel mit Phosphatidylserin und CD9; und ein extrazelluläres Vesikel mit CD9.

**Revendications**

1. Procédé d'assistance au diagnostic de la maladie d'Alzheimer, comprenant les étapes suivantes :

mesurer une quantité de vésicules extracellulaires présentant de la phosphatidylsérine et de la tétraspanine, ou la quantité de vésicules extracellulaires présentant de la phosphatidylsérine et de la tétraspanine et une quantité de vésicules extracellulaires présentant de la tétraspanine, dans un spécimen biologique dérivé d'un sujet, et déterminer que le sujet est affecté de la maladie d'Alzheimer à l'aide de la quantité de vésicules extracellulaires présentant de la phosphatidylsérine et de la tétraspanine, ou

un rapport entre la quantité de vésicules extracellulaires présentant de la phosphatidylsérine et de la tétraspanine et la quantité de vésicules extracellulaires présentant de la tétraspanine comme indicateur.

2. Procédé d'assistance au diagnostic de la maladie d'Alzheimer selon la revendication 1, dans lequel la détermination de la maladie d'Alzheimer est réalisée de telle sorte que le sujet est déterminé comme étant affecté de la maladie d'Alzheimer dans un cas où la quantité de vésicules extracellulaires présentant de la phosphatidylsérine et de la tétraspanine, ou le rapport entre la quantité de vésicules extracellulaires présentant de la phosphatidylsérine et de la tétraspanine et la quantité de vésicules extracellulaires présentant de la tétraspanine, est inférieur ou égal à une valeur de référence.

3. Procédé d'assistance au diagnostic de la maladie d'Alzheimer selon la revendication 1,

dans lequel la mesure de la quantité de vésicules extracellulaires consiste en la mesure de la quantité de vésicules extracellulaires présentant de la phosphatidylsérine et de la tétraspanine, et
la détermination de la maladie d'Alzheimer consiste en la détermination que le sujet est affecté de la maladie d'Alzheimer à l'aide de la quantité de vésicules extracellulaires présentant de la phosphatidylsérine et de la tétraspanine comme indicateur, et
la tétraspanine est sélectionnée parmi CD9, CD63, et CD81.

4. Procédé d'assistance au diagnostic de la maladie d'Alzheimer selon la revendication 1,

dans lequel la mesure de la quantité de vésicules extracellulaires consiste en la mesure de la quantité de vésicules extracellulaires présentant de la phosphatidylsérine et de la tétraspanine ;
la mesure de la quantité de vésicules extracellulaires présentant de la phosphatidylsérine et de la tétraspanine consiste en la mesure de la quantité de vésicules extracellulaires présentant de la phosphatidylsérine et de la tétraspanine à l'aide d'une substance présentant une affinité pour la tétraspanine et une substance présentant une affinité pour la phosphatidylsérine, et
la détermination de la maladie d'Alzheimer consiste en la détermination que le sujet est affecté de la maladie d'Alzheimer à l'aide de la quantité de vésicules extracellulaires présentant de la phosphatidylsérine et de la tétraspanine comme indicateur.

5. Procédé d'assistance au diagnostic de la maladie d'Alzheimer selon la revendication 4, dans lequel la substance présentant une affinité pour la phosphatidylsérine est une protéine contenant de l'immunoglobuline-mucine de lymphocyte T.

6. Procédé d'assistance au diagnostic de la maladie d'Alzheimer selon la revendication 1,

dans lequel la mesure de la quantité de vésicules extracellulaires consiste en la mesure de la quantité de vésicules extracellulaires présentant de la phosphatidylsérine et de la tétraspanine ;
la détermination de la maladie d'Alzheimer consiste en la détermination que le sujet est affecté de la maladie d'Alzheimer à l'aide de la quantité de vésicules extracellulaires présentant de la phosphatidylsérine et de la tétraspanine comme indicateur, et
le spécimen biologique est un échantillon de sang ou un liquide cérébrospinal.

7. Procédé d'assistance au diagnostic de la maladie d'Alzheimer selon la revendication 1,

dans lequel la mesure de la quantité de vésicules extracellulaires consiste en la mesure de la quantité de vésicules extracellulaires présentant de la phosphatidylsérine et de la tétraspanine et de la quantité de vésicules extracellulaires présentant de la tétraspanine ;
la détermination de la maladie d'Alzheimer consiste en la détermination que le sujet est affecté de la maladie d'Alzheimer à l'aide du rapport entre la quantité de vésicules extracellulaires présentant de la phosphatidylsérine et de la tétraspanine et la quantité de vésicules extracellulaires présentant de la tétraspanine comme indicateur, et
la tétraspanine est CD9 ou CD63.

8. Procédé d'assistance au diagnostic de la maladie d'Alzheimer selon la revendication 1,

dans lequel la mesure de la quantité de vésicules extracellulaires consiste en la mesure de la quantité de vésicules extracellulaires présentant de la phosphatidylsérine et de la tétraspanine et de la quantité de vésicules extracellulaires présentant de la tétraspanine ;

la mesure de la quantité de vésicules extracellulaires présentant de la phosphatidylsérine et de la tétraspanine consiste en la mesure de la quantité de vésicules extracellulaires présentant de la phosphatidylsérine et de la tétraspanine à l'aide d'une substance présentant une affinité pour la tétraspanine et une substance présentant une affinité pour la phosphatidylsérine,

la mesure de la quantité de vésicules extracellulaires présentant de la tétraspanine consiste en la mesure de la quantité de vésicules extracellulaires présentant de la tétraspanine à l'aide d'une substance présentant une affinité pour la tétraspanine, et

la détermination de la maladie d'Alzheimer consiste en la détermination que le sujet est affecté de la maladie d'Alzheimer à l'aide du rapport entre la quantité de vésicules extracellulaires présentant de la phosphatidylsérine et de la tétraspanine et la quantité de vésicules extracellulaires présentant de la tétraspanine comme indicateur.

9. Procédé d'assistance au diagnostic de la maladie d'Alzheimer selon la revendication 8,
dans lequel la substance présentant une affinité pour la phosphatidylsérine est une protéine contenant de l'immunoglobuline-mucine de lymphocyte T.

10. Procédé d'assistance au diagnostic de la maladie d'Alzheimer selon la revendication 1,

dans lequel la mesure de la quantité de vésicules extracellulaires consiste en la mesure de la quantité de vésicules extracellulaires présentant de la phosphatidylsérine et de la tétraspanine et de la quantité de vésicules extracellulaires présentant de la tétraspanine ;

la détermination de la maladie d'Alzheimer consiste en la détermination que le sujet est affecté de la maladie d'Alzheimer à l'aide du rapport entre la quantité de vésicules extracellulaires présentant de la phosphatidylsérine et de la tétraspanine et la quantité de vésicules extracellulaires présentant de la tétraspanine comme indicateur, et

le spécimen biologique est un échantillon de sang ou un liquide cérébrospinal.

11. Procédé d'assistance au diagnostic de la maladie d'Alzheimer ou d'un trouble cognitif léger, comprenant les étapes suivantes :

mesurer une quantité de vésicules extracellulaires présentant de la phosphatidylsérine et CD9 et une quantité de vésicules extracellulaires présentant CD9, dans un spécimen biologique dérivé d'un sujet, et

déterminer que le sujet est affecté de la maladie d'Alzheimer ou d'un trouble cognitif léger à l'aide d'un rapport entre la quantité de vésicules extracellulaires présentant de la phosphatidylsérine et CD9 et la quantité de vésicules extracellulaires présentant CD9 comme indicateur.

12. Procédé d'assistance au diagnostic de la maladie d'Alzheimer ou d'un trouble cognitif léger selon la revendication 11, dans lequel la détermination de la maladie d'Alzheimer consiste à déterminer que le sujet est affecté de la maladie d'Alzheimer ou d'un trouble cognitif léger dans un cas où le rapport entre la quantité de vésicules extracellulaires présentant de la phosphatidylsérine et CD9 et la quantité de vésicules extracellulaires présentant CD9 est inférieur ou égal à une valeur de référence.

13. Procédé d'assistance au diagnostic de la maladie d'Alzheimer ou d'un trouble cognitif léger selon la revendication 11,

dans lequel la mesure de la quantité de vésicules extracellulaires présentant de la phosphatidylsérine et de la tétraspanine consiste en la mesure de la quantité de vésicules extracellulaires présentant de la phosphatidylsérine et CD9 à l'aide d'une substance présentant une affinité pour CD9 et d'une substance présentant une affinité pour la phosphatidylsérine, et

la mesure de la quantité de vésicules extracellulaires présentant CD9 consiste en la mesure de la quantité de vésicules extracellulaires présentant CD9 à l'aide d'une substance présentant une affinité pour CD9.

14. Procédé d'assistance au diagnostic de la maladie d'Alzheimer ou d'un trouble cognitif léger selon la revendication 13, dans lequel la substance présentant une affinité pour la phosphatidylsérine est une protéine contenant de l'immunoglobuline-mucine de lymphocyte T.

15. Procédé d'assistance au diagnostic de la maladie d'Alzheimer ou d'un trouble cognitif léger selon la revendication 11, dans lequel le spécimen biologique est un échantillon de sang ou un liquide cérébrospinal.

**16.** Utilisation in vitro d'un kit de réactif assistant au diagnostic de la maladie d'Alzheimer, le kit de réactif comprenant :

une substance présentant une affinité pour la tétraspanine, et
une substance présentant une affinité pour la phosphatidylsérine.

**17.** Utilisation in vitro d'un kit de réactif assistant au diagnostic de la maladie d'Alzheimer ou d'un trouble cognitif léger, le kit de réactif comprenant :

une substance présentant une affinité pour CD9, et
une substance présentant une affinité pour la phosphatidylsérine.

**18.** Utilisation in vitro d'un jeu de biomarqueur assistant au diagnostic de la maladie d'Alzheimer, le biomarqueur comprenant :
une vésicule extracellulaire présentant de la phosphatidylsérine et de la tétraspanine.

**19.** Utilisation in vitro d'un jeu de biomarqueur assistant au diagnostic de la maladie d'Alzheimer ou d'un trouble cognitif léger, le jeu de biomarqueur comprenant :
une vésicule extracellulaire présentant de la phosphatidylsérine et CD9, et

EP 4 067 906 B1

# FIG. 1

Capture: Tim4, Detection: CD9 ANTIBODY

# FIG. 2

Capture: Tim4, Detection: CD9 ANTIBODY/
Capture: CD9 ANTIBODY, Detection: CD9 ANTIBODY

## FIG. 3

Capture: Tim4, Detection: CD9 ANTIBODY

## FIG. 4

Capture: Tim4, Detection: CD9 ANTIBODY/
Capture: CD9 ANTIBODY, Detection: CD9 ANTIBODY

FIG. 5

[Capture: Tim4, Detection: CD9 ANTIBODY/
  Capture: CD9 ANTIBODY, Detection: CD9 ANTIBODY]
  × [Aβ42/Aβ40]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2016550673 A
- JP 2017520760 A
- EP 3228709 A1

- JP 2016161480 A
- WO 2015061634 A2
- WO 2016088689 A

### Non-patent literature cited in the description

- **C.N. WINSTON et al.** Alzheimer's & Dementia: Diagnosis. *Assessment & Disease Monitoring*, 2016, vol. 3, 63-72
- **MUSTAPIC M et al.** *Front. Neurosci.*, 2017, vol. 11, 278

- **W. NAKAI et al.** *SCIENTIFIC REPORTS*, 23 September 2016, vol. 6 (33935), 1-11
- **NAOTO AOKI**. Study of Obesity. *Nature Reviews Immunology 9*, 2007, vol. 13 (2), 581-593
- Immunoassay. Kodansha Ltd., 2014
- Immunoassay. Kodansha Ltd, 2014